(19) Europäisches Patentamt
European Patent Office
Office européen des brevets

(11) EP 2 823 819 B1

(12) # EUROPEAN PATENT SPECIFICATION

(45) Date of publication and mention of the grant of the patent:
**02.06.2021 Bulletin 2021/22**

(21) Application number: **12776128.6**

(22) Date of filing: **30.04.2012**

(51) Int Cl.:
***A61K 31/56*** *(2006.01)* ***A61K 36/48*** *(2006.01)*
***A61P 29/00*** *(2006.01)* ***A61P 11/06*** *(2006.01)*

(86) International application number:
**PCT/KR2012/003383**

(87) International publication number:
**WO 2012/148247 (01.11.2012 Gazette 2012/44)**

(54) **OLEANOLIC ACID ACETATE AS AN ACTIVE INGREDIENT FOR PREVENTING OR TREATING TLR- OR IL-6-MEDIATED DISEASES**

OLEANOLSÄUREACETAT ALS WIRKSTOFF ZUR VORBEUGUNG ODER BEHANDLUNG VON TLR-ODER IL-6-VERMITTELTEN ERKRANKUNGEN

ACÉTATE D'ACIDE OLÉANOLÏQUE EN TANT QUE PRINCIPE ACTIF POUR LA PRÉVENTION OU LE TRAITEMENT DE MALADIE À MÉDIATION PAR TLR OU IL-6

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.04.2011 KR 20110041189**
**28.09.2011 KR 20110098547**

(43) Date of publication of application:
**14.01.2015 Bulletin 2015/03**

(73) Proprietor: **Korea Research Institute of Bioscience and Biotechnology**
**Daejeon 305-806 (KR)**

(72) Inventors:
- **RHO, Mun Chual**
**Daejeon 305-806 (KR)**
- **LEE, Woo Song**
**Daejeon 305-806 (KR)**
- **OH, Hyun Mee**
**Daejeon 305-806 (KR)**
- **KIM, Young Min**
**Daejeon 305-806 (KR)**
- **RYU, Young Bae**
**Daejeon 305-806 (KR)**
- **PARK, Su Jin**
**Daejeon 305-806 (KR)**
- **LEE, Seung Woong**
**Daejeon 305-806 (KR)**
- **CHO, Kyoung Oh**
**Gwangju 500-734 (KR)**

(74) Representative: **Carpintero Lopez, Francisco et al**
**Herrero & Asociados, S.L.**
**Cedaceros 1**
**28014 Madrid (ES)**

(56) References cited:
**WO-A1-02/09698** **WO-A2-2007/121352**
**US-A1- 2007 014 739**

- **JOSÉ M. NARVÁEZ-MASTACHE ET AL: "Antihyperglycemic Activity and Chemical Constituents of Eysenhardtia platycarpa", JOURNAL OF NATURAL PRODUCTS, vol. 69, no. 12, 1 December 2006 (2006-12-01), pages 1687-1691, XP055121351, ISSN: 0163-3864, DOI: 10.1021/np060166z**
- **RODRIGO N GUIMARÃES ET AL: "Free radical scavenging and anti-edematogenic activities of Paullinia elegans Cambess., Sapindaceae, leaves extracts", BRAZILIAN JOURANL OF PHARMACOGNOSY, vol. 20, no. 1, 1 January 2010 (2010-01-01), pages 113-116, XP055151206,**
- **SEISUKE ITO ET AL: "Triterpenic lipids in Azuki bean seeds", NIPPON NOGEI KAGAKUKAISHI - JOURNAL OF THE AGRICULTURAL CHEMICAL SOCIETY OF JAPAN, NIPPON NOGEI KAGAKUKAI, TOKYO, JP, vol. 59, no. 9, 1 January 1985 (1985-01-01), pages 895-900, XP008173461, ISSN: 0002-1407**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- RODRIGO N. GUIMARAES ET AL.: 'Free radical scavenging and anti-edematogenic activities of Paullinia elegans Cambess., Sapindaceae, leaves extracts' BRAZILIAN JOURNAL OF PHARMACOGNOSY vol. 20, no. 1, 2010, pages 113 - 116, XP055151206
- SEISUKE ITO ET AL.: 'Triterpenic lipids in Azuki bean seeds' JOURNAL OF AGRICULTURAL CHEMICAL SOCIETY OF JAPAN vol. 59, no. 9, 1985, pages 895 - 900, XP008173461
- YING WEI ET AL.: 'Anti-HIV-1 protease triterpenoids from Stauntonia obovatifoliola Hayata subsp. intermedia' PHYTOCHEMISTRY vol. 69, no. 9, 2008, pages 1875 - 1879, XP022700583
- K. KINOSHITA ET AL.: 'Inhibitory effect of some triterpenes from cacti on 32Pi- incorporation into phospholipids of HeLa cells promoted by 12-0-tetradecanoylphorbol-13-acetate' PHYTOMEDICINE vol. 6, no. 2, 1999, pages 73 - 77, XP055151210
- JOSE M. NARVAEZ-MASTACHE ET AL.: 'Antihyperglycemic activity and chemical constituents of Eysenhardtia platycarpa' J. NAT. PROD. vol. 69, no. 12, 2006, pages 1687 - 1691, XP055121351
- MUKAI Y ET AL: "Polyphenol-containing azuki bean (Vigna angularis) seed coats attenuate vascular oxidative stress and inflammation in spontaneously hypertensive rats", THE JOURNAL OF NUTRITIONAL BIOCHEMISTRY, ELSEVIER, AMSTERDAM, NL, vol. 22, no. 1, 1 January 2011 (2011-01-01), pages 16-21, XP027555849, ISSN: 0955-2863 [retrieved on 2010-02-25]

## Description

## BACKGROUND OF THE INVENTION

### 1. Field of the Invention

The present invention relates to oleanolic acid acetate as an active ingredient for use in preventing or treating TLR- and IL-6-mediated diseases. More particularly, the present invention relates to a pharmaceutical composition including oleanolic acid acetate or a pharmaceutically acceptable salt thereof as an active ingredient for use in preventing or treating TLR- and IL-6-mediated diseases, a pharmaceutical composition including an adzuki bean extract containing the compound or the salt thereof or a fraction thereof as an active ingredient for use in preventing or treating or TLR- and IL-6-mediated diseases. Further, the present invention relates to a skin composition for external use, a health functional food, and a personal hygiene product which include the active ingredient for use in preventing or improving TLR- and IL-6-mediated diseases.

### 2. Description of the Related Art

Immune responses are a series of reactions that are caused by activated immune cells against exogenous and endogenous substances (antigens). When microorganisms including bacteria, virus, etc. and foreign bodies enter the living body, host cells secrete factors such as cytokines associated with inflammation and induce inflammatory responses in order to overcome infections. When these microorganisms and foreign bodies are recognized by immune cells, immune cells are activated and many factors that are the causes of inflammation are secreted by the activated immune cells to cause inflammatory responses (Chen G.Y., Nat. Rev. Immunol., 10(12): 826-837, 2010).

With the development of molecular biology and genetic engineering, inflammation-associated intracellular molecular targets have been revealed. In particular, as genes recognizing the specific region of a pathogen in the nonspecific innate immune response in the early stage of infection have been identified, the new molecular mechanisms for initial inflammation events have been proposed. Recently, Toll-like receptor (TLR), which is a gene recognizing pathogens in the early stage of inflammation, has become known to recognize the plasma membrane components (lipopolysaccharide (LPS:endotoxin), peptidoglycan) and nucleic acid components (ds RNA, single strand RNA, GpG DNA etc.) of the pathogen, and thus many studies have been actively conducted on TLR (Brown J., et al, J. Dent. Res., 2010, Epub ahead of print: Palusinska-Szysz M., et al., Folia Microbiol.(Praha), 2010, 55(5), 508-14).

TLR is a type I transmembrane signaling molecule and is mainly expressed in cells of the innate immune system. TLR in these cells recognizes PAMPs (pathogen-associated molecular patterns) or MAMPs (microorganism-associated molecular patterns) through an extracellular domain, and consequently, initiates inflammatory cell activation in the innate immune cells. Since the identification of TLRs, many efforts have been made to find PAMPs binding to TLRs. Until now, one or more ligands thereof have been identified, excluding those of TLR10, TLR12, and TLR13 (Kawai T. et al., Ann. N.Y. Acad. Sci., 2008, 1143, 1-20). Although TLRs are different from each other, they share common signaling pathways (Liew F.Y., et al., Nat. Rev. Immunol., 2005, 5(6), 446-458) .

The common signaling pathway of TLRs is NF-κB activation via TIR (Toll/interleukin-1 receptor-like) domain. In the signal transduction by TLR, it interacts with MyD88 having a TIR domain to form a complex. The TIR domain located at the C-terminus of MyD88 forms a complex with TLR, and the Death domain located N-terminus thereof forms a complex with the Ser/Thr kinase, IRAK (IL-1R associated kinase)-1 or IRAK-2. Subsequently, IRAK-1 activates NIK (NΓ-κB-inducing kinase) via interaction with TRAF6 (tumor necrosis factor receptor-associated factor 6), and NIK activation phosphorylates IKK (IκB kinase). In turn, the phosphorylated IKK phosphorylates IκB (inhibitory κB), leading to the activation of NF-κB. Activation of NF-κB results in its translocation to the nucleus, where it acts as a transcription factor to induce expression of cytokine and cell adhesion molecule-related genes (Brown J., et al, J. Dent. Res., 2010, Epub ahead of print: Palusinska-Szysz M., et al., Folia Microbiol. (Praha), 2010, 55(5), 508-14).

In particular, TLR-7 is known to recognize ssRNA (single-stranded RNA) and its analogue polyI (polyinosinic acid). That is, TLR-7 recognizes ssRNA and then reacts with the adaptor protein MyD88 to activate the transcription factors NFkB and AP-1, resulting in production of inflammatory cytokines. In another TRIF-dependent mechanism, TLR transduces signals through TRIF, TBK1 (ANK-binding kinase 1), TRAF3 and the transcription factor IRF3, leading to the production of type I interferons. TLR-3 is known to recognize dsRNA (double-stranded RNA) and its analogue polyI:C(polyinosinic-polycytidylic acid) generated during RNA virus proliferation. That is, the leucine-rich repeat ectodomain of TLR-3 recognizes dsRNA, and then reacts with the adaptor protein TRIF to activate the transcription factors IRF3 and NFκB, leading to the production of inflammatory cytokines such as type I IFN, IL-6, IL-12 or the like (Alexopoulou L., et al, Nature, 2001, 413, 732-738). Excessive production of inflammatory mediators causes excessive immune responses, resulting in a worsening of various human diseases such as colitis, pancreatitis, rheumarthritis, asthma, or the like. Therefore, development of inhibitors of the inflammatory mediators such as TNF-α, IL-1β, IL-6, and IL-10 will

contribute to the treatment of various immune diseases and human diseases.

STAT3 (signal transducers and activators of transcription) is known to act as another important transcription factor in autoimmune diseases including rheumarthritis, in addition to NF-κB. Particularly, STAT3 is known to be activated by an inflammation-mediating cytokine, interleukin-6 (IL-6), and also by epithelial growth factor (EGF) (Darnell Jr. J.E., et al., Science, 1994, 264, 1415-1421).

IL-6 is a cytokine known as B-cell stimulating factor 2 or interferon β2, and was found to be a differentiation factor involved in the activation of B-lymphocyte cells (Hirano T., et al., Nature, 1986, 324, 73-76), and later, it was also revealed that IL-6 is a multifunctional cytokine affecting T cell differentiation and proliferation, nerve cell differentiation, osteoclast formation, and protein synthesis in the liver (Akira S., et al., Adv. In Immunology, 1993, 54, 1-78).

Two functionally different membrane receptor molecules are required for the induction of IL-6 activation. One of them is interleukin-6 receptor (IL-6R) having a molecular weight of approximately 80 KD which specifically binds to IL-6, and the other is gp130 having a molecular weight of approximately 130 KD which is involved in signal transduction. IL-6 and IL-6R form an IL-6/IL-6R complex, which binds to the other membrane protein gp130, leading to the induction of intracellular IL-6 signal transduction (Taga T., et al., J. Exp. Med., 1987, 196, 967). It was revealed that during the induction of intracellular IL-6 signal transduction, phosphorylation of gp130 and JAK (Janus kinase) is essential for STAT3 transcriptional activation (Heinrich P., et al., Biochem. J., 2003, 374, 1-20). Binding of IL-6 to receptors results in activation of intracellular JAK2 (Janus Kinases 2) by phosphorylation, and several tyrosine residues in its cytoplasmic domain are phosphorylated by the phosphorylated JAK2, providing the docking site for cytoplasmic proteins such as STAT3 having SH2 or other phosphotyrosine-binding motif. STAT3 binding to the cytoplasmic domain of the receptor is phosphorylated by JAK2, and then released from the receptor. The activated STAT3 bind to each other in the cytoplasm, and then form homo- or heterodimers that translocate to the nucleus where they bind to the transcriptional activation region of a target molecule to promote transcription (Levy, D.E., et al., Nat. Rev. Mol. Cell Biol., 2002, 3, 651-62: Darnell, J.E., Science, 1997, 277, 1630-1635). It has been revealed that excessive production of IL-6 is involved in various diseases such as rheumarthritis, castleman syndrome, Crohn's disease, osteoporosi, cancer cachexia, arteriosclerosis, diabetes or the like (Akira S., et al., Adv. Immunology, 1993, 54, 1-78: Kallen K., et al., Exp. Opin. Invest. Drugs, 1997, 6, 237-266). Furthermore, constitutive activation and overexpression of STAT3 appear to be involved in several forms of cancer, including myeloma, breast carcinomas, prostate cancer, brain tumors, head and neck carcinomas, melanoma, leukemias and lymphomas, particularly chronic myelogenous leukemia and multiple myeloma (Niu, et al., Cancer Res., 1999, 59, 5059-5063).

Previously, there have been reports that a compound found to have an antioxidant activity, such as inhibition of iNOS activity, could show therapeutic effects on inflammatory diseases. However, it has since been reported that antioxidants have no therapeutic effect on the diseases classified as inflammatory diseases, such as rheumarthritis and lupus (Karen H. Costenbader, et al., American Journal of Epidemiology, 172(2) :205-216, 2010). Thus, there is a problem that antioxidant effects cannot be directly applied to the treatment of inflammatory diseases.

Meanwhile, atopic dermatitis is a representative autoimmune skin disease. Atopic dermatitis is a chronic recurrent skin disease, accompanied by wheal, eczema, itching, and inflammation. Its pathological and genetic mechanisms have not been clarified yet. Atopy is a multifactorial immune response arising from susceptible genetic background, environment, damage of skin barrier, and immunological factors. Atopic dermatitis is defined as a typical Th2-type immune disease (Mamessier, E., et al, European Journal of Dermatology, 2006, 16(2), 103), because Th2 immune response plays an important role in the onset of atopic dermatitis. When allergens functioning as an antigen enter the body through the skin, antigen presenting cells in the skin recognize and react with it. At this time, allergens specifically induce Th2-type immune response, and Th2-phenotype APCs directs differentiation of T cells to Th2 cells. Th2 cells express large amounts of Th2-type cytokines, and Th2-type cytokines activate mast cells to promote secretion of soluble mediators such as histamine and stimulate B cells to promote IgE production. These mediators recruit immune cells into the site of inflammation to induce more enhanced Th2-type immune response, consequently leading to atopic dermatitis. Candidate target genes for the treatment of atopic dermatitis include FceRlb with high IgE affinity, Th2-type cytokines such as IL-4, IL-5, and IL-13, and chemokines such as eotaxin and RANTES, which play a critical role in atopic dermatitis (Hoffjan, S., et al., Journal of Molecular Medicine, 2005, 83(9), 682-692: Cookson, W., Nat. Rev. Immunol., 2004, 4(12), 978-988). Therefore, development of compounds capable of regulating gene expressions of Th2-type cytokines and chemokines greatly will greatly contribute to the treatment of various immune diseases including atopic dermatitis and human diseases.

The present inventors have made many efforts to find a therapeutic substance for TLR- and IL-6-mediated diseases. As a result, they demonstrated that an adzuki bean (Phaseoli angularis Wight or Phaseolus calcaratus Roxburgh) extract from the natural resources, a fraction thereof, and a compound purified therefrom, namely oleanolic acid acetate or a pharmaceutically acceptable salt thereof, have the effects of inhibiting TLR-3 and TLR-7 activation induced by the synthetic analogues of dsRNA and ssRNA, Poly(I:C) and Poly(I) and blocking the NF-κB signaling pathway, the effect of inhibiting transcriptional activation and phosphorylation of an inflammation-related transcription factor STAT3 which is activated by IL-6, and the effect of inhibiting DNCB-induced atopic dermatitis in NC/Nga mouse

and collagen-induced arthritis, thereby completing the present invention for developing a pharmaceutical composition for the prevention and treatment of TLR- and IL-6-mediated diseases.

Narvaez-Mastache J.M. et al [Journal of natural products (2006)Vol.69(12):1687-1691] refers the antihyperglycemic activity of 3-O-acetyloleanolic acid compound but it does not anticipate its ability to treat the metabolic diseases of the present invention.

Guimaraes R.N. et al [Brazilian Journal of Pharmacognosy (2010)Vol.20(1):113-116] discloses an hexane fraction of *Paullinia elegans* extract showing free radical scavenging antiedematogenic activity. This document does not teach nor suggest however that said anti-inflammatory activity is due to 3-O-actetyloleanolic acid.

Finally, WO2007121352 discloses birch bark extracts and compounds isolated therefrom such as a) betulin caffeate; b) betulinic acid; c) oleanolic acid; d) betulin; e) lupeol; f)3-acetoxyoleanolic acid; g) betulin aldehyde, h) betulonic aldehyde; and i) pycarehic acid (betulinic acid-3 caffeate). This document teaches the cell growth inhibitory effect of betulin 3-caffeate but silent about any inhibitory cell growth activity of 3-acetoxyoleanolic acid.

## SUMMARY OF THE INVENTION

An object of the present invention is to provide a pharmaceutical composition including oleanolic acid acetate or a pharmaceutically acceptable salt thereof as an active ingredient for use in preventing or treating TLR- and IL-6-mediated diseases.

Another object of the present invention is to provide a pharmaceutical composition including an adzuki bean extract containing the compound or the pharmaceutically acceptable salt thereof, or a fraction thereof as an active ingredient for use in preventing or treating TLR- and IL-6-mediated diseases.

Still another object of the present invention is to provide a skin composition for external use, a health functional food, and a personal hygiene product including, as an active ingredient, oleanolic acid acetate or the pharmaceutically acceptable salt thereof; or the adzuki bean extract containing the compound or the pharmaceutically acceptable salt thereof, or the fraction thereof, for use in preventing or improving TLR- and IL-6-mediated diseases.

## BRIEF DESRIPTION OF THE DRAWINGS

FIG. 1a is a chromatogram showing the results of HPLC of the methanol extract, the hexane fraction, the ethyl acetate fraction and the water fraction of Phaseoli angularis Wight;

FIG. 1b is a chromatogram showing the results of HPLC of the methanol extract, the hexane fraction, the ethyl acetate fraction and the water fraction of Phaseolus calcaratus Roxburgh;

FIGs. 2a and 2b are a graph and a table showing the inhibitory effects of the methanol extract, the hexane fraction, the ethyl acetate fraction and the water fraction of adzuki bean on SEAP activity increased by poly(I) treatment in THP-1-blue cells;

FIG. 3 is a graph showing the inhibitory effect of the methanol extract of adzuki bean on poly(I:C)-induced SEAP activity in THP-1-blue cells;

FIGs. 4a and 4b are Western blot images showing the inhibitory effect of the adzuki bean methanol extract on NFκB P50 that is activated by poly(I) treatment and translocates to the nucleus (FIG. 4a) and the inhibitory effect on C-Jun activity of AP-1 (FIG. 4b) in THP-1 cells;

FIGs. 4c to 4e are Western blot images showing the inhibitory effect of the adzuki bean methanol extract on poly (I)-induced IKK $\alpha/\beta$ (FIG. 4c), IκB (FIG. 4d), p38 and JNK (FIG. 4e) phosphorylations in THP-1 cells;

FIGs. 5a and 5b are Western blot images showing the inhibitory effect of the adzuki bean methanol extract on NFκB P65/p50 that is activated by poly(I:C) treatment and translocates to the nucleus in THP-1 cells (FIG. 5a) and the inhibitory effect on C-Jun activity of AP-1 (FIG. 5b);

FIGs. 5c to 5e are Western blot images showing the inhibitory effect of the adzuki bean methanol extract on poly (I: C)-induced IKK $\alpha/\beta$ (FIG. 5c), IκB (FIG. 5d), p38 and JNK (FIG. 5e) phosphorylations in THP-1 cells;

FIG. 5f is a Western blot image showing the inhibitory effect of the adzuki bean methanol extract on poly(I:C)-induced IRF3 phosphorylation in THP-1 cells;

FIGs. 6a to 6e are graphs showing the inhibitory effects of the adzuki bean methanol extract on mRNA expressions of a poly(I)-induced inflammatory cytokine TNF$\alpha$ (FIG. 6a), an inflammatory cytokine IL-6 (FIG. 6b), a chemokine RANTES (FIG. 6c), IFN$\beta$ (FIG. 6d), and a chemokine MCP-1 (FIG. 6e) in THP-1 cells;

FIGs. 7a to 7d are a graph showing the inhibitory effect of the adzuki bean methanol extract on mRNA expressions of a poly(I:C)-induced inflammatory factor TNF$\alpha$ (FIG. 7a), an inflammatory factor IL-6 (FIG. 7b), a cell adhesion factor ICAM-1 (FIG. 7c), and a chemokine MCP-1 (FIG. 7d) ;

FIG. 8a is a photograph showing the inhibitory effect of the adzuki bean methanol extract on IL-6-induced STAT3

phosphorylation in Hep3B cells;

FIG. 8b is a photograph showing the inhibitory effect of the compound of Chemical Formula 1 isolated and purified from adzuki bean on IL-6-induced STAT3 phosphorylation in Hep3B cells;

FIG. 9 is a photograph showing the back skin of NC/Nga mouse, in which the adzuki bean methanol extract showed the effect of improving the symptoms of DNCB-induced atopic dermatitis in NC/Nga mouse;

FIG. 10 is a graph showing skin severity, in which the adzuki bean methanol extract showed the effect of improving the symptoms of DNCB-induced atopic dermatitis in NC/Nga mouse.

FIG. 11 is a graph showing the effect of the adzuki bean methanol extract on the blood eosinophil count according to DNCB-induced atopic dermatitis of NC/Nga;

FIG. 12 is a photograph showing the forelegs and hind legs of normal DBA/1 OlaHsd and CIA-induced mouse model;

FIG. 13 is a CIA index showing the improvement effect of the adzuki bean methanol extract on the symptoms of CIA-induced arthritis of DBA/1 OlaHsd mice;

FIG. 14 is a graph showing the inhibitory effect of each concentration of oleanolic acid or oleanolic acid acetate on IL-6-induced STAT3 luciferase activity in Hep3B cells;

FIG. 15 is a photograph showing the inhibitory effects of the adzuki bean methanol extract on differentiation of osteoclasts from the bone marrow macrophages isolated from mouse bone marrow cells; and

FIG. 16 is a photograph showing the inhibitory effects of oleanolic acid acetate on differentiation of osteoclasts from the bone marrow macrophages isolated from mouse bone marrow cells

## DETAILED DESCRIPTION OF THE PREFERRED EMBODIMENTS

In one aspect to achieve the above objects, the present invention provides a a compound represented by the following Chemical Formula 1 or a pharmaceutically acceptable salt thereof as an active ingredient for use in preventing or treating TLR- and IL-6-mediated diseases.

[Chemical Formula 1]

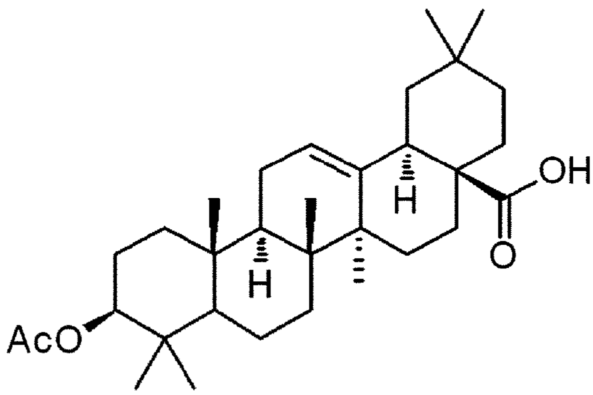

In one embodiment of the present invention, the present inventors obtained each methanol extract from Phaseoli angularis Wight and Phaseolus calcaratus Roxburgh belonging to adzuki bean, and they performed fractionation of the methanol extracts using water, n-hexane, ethyl acetate or the like so as to obtain their fractions. The HPLC results of the extracts and fractions showed that Phaseoli angularis Wight and Phaseolus calcaratus Roxburgh showed similar HPLC histograms, and oleanolic acid acetate (Chemical Formula 1) and stigmasterol (Chemical Formula 2) were purified as active ingredients from the n-hexane fractions.

The compound of the following Chemical Formula 1 or 2 may be commercially available or may be obtained by a known method. Extraction, separation and purification methods of Compound 1 and Compound 2 from the adzuki bean according to the present invention are as follows: The hexane soluble fractions of adzuki bean are separated using a solution mixture of hexane and ethyl acetate by silica gel column chromatography. Among them, Fr.3 and Fr.4 were recrystallized using methanol, and the pure compounds of Chemical Formulae 1 and 2 are separated.

Chemical Formula 1

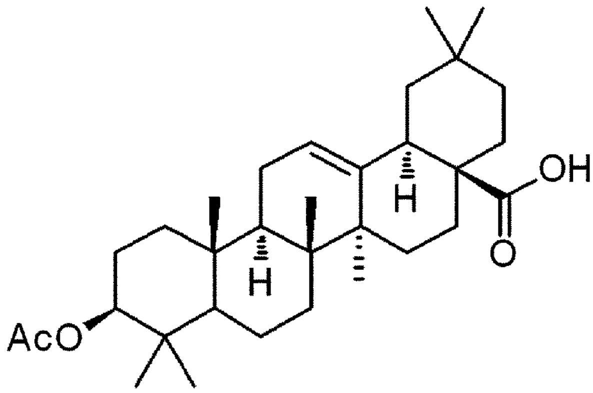

Chemical Formula 2

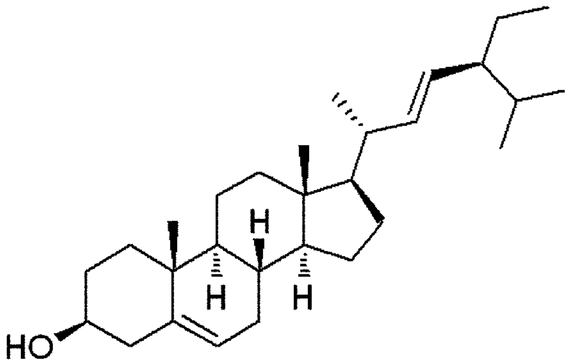

As used herein, the term "TLR- and IL-6-mediated diseases" refer to diseases caused as follows: NF-κB that is activated by activation of TLR-3 and TLR-7 belonging to Toll-like receptor (TLR) translocates into the nucleus, where expressions of various cytokine and cell adhesion molecule-related genes are induced, and IL-6-induced STAT3 activation is inhibited to cause the diseases. The TLR- and IL-6-mediated diseases may include largely, but are not particularly limited to, inflammatory diseases, autoimmune diseases, cancer diseases or metabolic diseases.

In the present invention, the inflammatory disease collectively refers to a disease having inflammation as a major lesion. With respect to the objects of the present invention, the inflammatory disease may be preferably a TLR- and IL-6-mediated inflammatory disease. The inflammatory disease may preferably include, but is not limited to, ssRNA and dsRNA virus infection, sepsis, multiple chondritis, scleroderma, dermatitis, eczema, gout, periodontal disease, Behcet's syndrome, edema, vasculitis, Kawasaki disease, diabetic retinitis, autoimmune pancreatitis, angiitis, glomerulonephritis, acute and chronic bacterial and fungal infections, acute and chronic bronchitis, and influenza infections.

In one embodiment of the present invention, it was confirmed that oleanolic acid acetate of Chemical Formula 1 inhibited STAT3 luciferase activity in a concentration-dependent manner (Table 7), and in particular, it is able to exhibit remarkably excellent prophylactic and therapeutic effects on inflammatory diseases, compared to the known oleanolic acid (FIG. 14), and a pharmaceutical composition including the compound as an active ingredient is effective for the prevention or treatment of inflammatory diseases related to IL-6-induced STAT3 activation.

In the present invention, the autoimmune disease collectively refers to a disease that is directly or indirectly caused by pathological immune responses against a self antigen. With respect to the objects of the present invention, the autoimmune disease may be preferably a TLR- and IL-6-mediated autoimmune disease. The autoimmune disease may include, but is not limited to, preferably atopic dermatitis, arthritis, psoriasis, asthma, graft- versus-host disease, immune deficiency syndrome, systemic erythematous lupus, and multiple sclerosis, and more preferably, atopic dermatitis or arthritis. TLR stimulates immune responses and is associated with the development of autoimmune diseases, and IL-6-activated STAT3 functions as an important transcription factor in various autoimmune diseases. Therefore, oleanolic acid acetate of Chemical Formula 1, which was found to inhibit STAT3 luciferase activity in a concentration-dependent manner in the present invention, is effective for TLR- and IL-6-mediated autoimmune diseases. The pharmaceutical composition including the compound represented by Chemical Formula 1 of the present invention or the pharmaceutically acceptable salt thereof as an active ingredient is able to exhibit the therapeutic effect by an action mechanism that is different from those of conventional antioxidants.

In the present invention, the cancer disease refers to a disease that forms a mass or tumor consisting of

undifferentiated cells with an unlimited capacity of proliferation regardless of the condition in the tissue. With respect to the objects of the present invention, the cancer disease may be preferably a TLR- and IL-6-mediated cancer disease. The cancer disease may preferably include, but is not limited to, myeloma, breast carcinoma, prostate cancer, brain tumors, head and neck carcinoma, melanoma, leukemia, and lymphoma. Constitutive activity and overexpression of IL-6-activated STAT3 are associated with various types of cancer including chronic myelogenous leukemia or multiple myeloma, and the cancer diseases can be prevented or treated by inhibiting STAT3 activation.

In the present invention, the metabolic disease collectively refers to a disease that is caused by metabolic disorders in the body. With respect to the objects of the present invention, the metabolic disease may be preferably a TLR- and IL-6-mediated metabolic disease. The metabolic disease are limited to, preferably osteoporosis, arteriosclerosis, myocardial infarction, and more preferably, osteoporosis. Many studies have revealed that the excessive production of IL-6 is particularly involved in osteoporosis, and therefore, IL-6-mediated diseases can be prevented or treated by the composition of the present invention. In one embodiment of the present invention, when the compound of Chemical Formula 1 was treated, the number and size of osteoclasts were reduced in a concentration-dependent manner, indicating that the compound exhibits the prophylactic and therapeutic effects on osteoporosis by inhibiting osteoclast differentiation (FIG. 16).

In the present invention, the TLR- and IL-6-mediated diseases may include all diseases that are mediated by TLR and IL-6, in addition to the above diseases. Examples thereof may include anaphylaxis or hypersensitivity reactions, Alzheimer's disease, cachexia, obesity, hyperimmunoglobulinemia, Meniere's disease, pain, hypersensitivity lung diseases, endocrine eye disease and the like.

According to one embodiment of the present invention, the present inventors confirmed that oleanolic acid acetate significantly inhibited poly(I) and poly(I:C)-induced SEAP activation, but stigmasterol did not show the inhibitory effect on poly(I) and poly(I:C)-induced SEAP activation (Table 4). Therefore, the compound of Chemical Formula 1 is able to inhibit TLR7 and TLR3 signaling pathways that are induced by ssRNA poly(I) and dsRNA poly(I:C), and a fraction thereof is able to inhibit poly(I)-induced TLR7 signaling pathway. Thus, it was confirmed that the compound is suitable for the prevention or treatment of TLR- and IL-6-mediated diseases.

Further, according to another embodiment of the present invention, the present inventors confirmed that the compounds of Chemical Formulae 1 and 2 inhibited STAT3 luciferase activity in a concentration-dependent manner (Table 7), and the compound of Chemical Formula 1 is able to exhibit remarkably excellent prophylactic and therapeutic effects on inflammatory diseases, compared to the known oleanolic acid (FIG. 14), suggesting that the compound of Chemical Formula 1 of the present invention or the pharmaceutically acceptable salt thereof can be used for the prevention or treatment of diseases associated with IL-6-induced STAT3 activation.

The pharmaceutical composition including the compound of Chemical Formula 1 of the present invention or the pharmaceutically acceptable salt thereof may further include a proper carrier, excipient or diluent that is typically used in the preparation of the pharmaceutical composition. In this regard, the content of oleanolic acid acetate, the salt thereof, or the extract or fraction thereof in the composition is, but not particularly limited to, 0.0001 to 10% by weight, preferably 0.001 to 1% by weight, based on the total weight of the composition.

As used herein, the term "pharmaceutically acceptable salt" refers to a salt that can be pharmaceutically used, among the substances having cations and anions coupled by electrostatic attraction. Typically, it may include metal salts, salts with organic bases, salts with inorganic acids, salts with organic acids, salts with basic or acidic amino acids or the like. Examples of the metal salts may include alkali metal salts (sodium salts, potassium salts, etc.), alkaline earth metal salts (calcium salts, magnesium salts, barium salts, etc.), aluminum salts or the like; examples of the salts with organic bases may include salts with triethylamine, pyridine, picoline, 2,6-lutidine, ethanolamine, diethanolamine, triethanolamine, cyclohexylamine, dicyclohexylamine, N,N'- dibenzylethylenediamine or the like; examples of the salts with inorganic acids may include salts with hydrochloric acid, hydrobromic acid, nitric acid, sulfuric acid, phosphoric acid, or the like; examples of the salts with organic acids may include salts with formic acid, acetic acid, trifluoroacetic acid, phthalic acid, fumaric acid, oxalic acid, tartaric acid, maleic acid, citric acid, succinic acid, methanesulfonic acid, benzenesulfonic acid, p-toluenesulfonic acid or the like; examples of the salts with basic amino acids may include salts with arginine, lysine, ornithine or the like; and examples of the salt with acidic amino acids include salts with aspartic acid, glutamic acid or the like.

The composition including the compound of Chemical Formula 1 of the present invention or the pharmaceutically acceptable salt thereof may be formulated into oral preparations such as powder, granule, tablet, capsule, suspension, emulsion, syrup, aerosol, etc., or formulated into preparations for external use, suppository or sterile solution for injection according to the typical method. In the present invention, the carrier, excipient and diluent that can be included in the composition including the adzuki bean extract and the fraction thereof may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate and mineral oil. For the formulation, a commonly used diluent or excipient such as a filler, an extender, a binder, a wetting agent, a disintegrating agent, a surfactant or the like is used for preparation. Solid

formulations for oral administration include a tablet, a pill, a powder, a granule, a capsule or the like. Those solid preparations are prepared by mixing at least one excipient, e.g. starch, calcium carbonate, sucrose, lactose, gelatin, etc. with the adzuki bean extract and the fraction thereof. Also, a lubricant such as magnesium stearate or talc may be included in addition to the excipient. Liquid preparations for oral administration include a suspension, a liquid for internal use, an emulsion, a syrup or the like. These preparations may include various excipients such as a wetting agent, a sweetener, an aromatic, a preservative or the like, in addition to general simple diluents such as water and liquid paraffin. Preparations for parenteral administration include a sterilized aqueous solution, a non-aqueous formulation, a suspension, an emulsion, a freezedried formulation and a suppository. As the non-aqueous formulation or suspension, propylene glycol, polyethylene glycol, vegetable oil such as olive oil, injectable ester such as ethyl oleate, or the like may be used. Witepsol, Macrogol, Tween 61, cocoa butter, laurin butter, glycerogelatin or the like may be used as a base of the suppository.

The preferred dosage of the pharmaceutical composition of the present invention may vary depending on the conditions and body weight of a patient, severity of disease, type of the drug, administration route and period, but may be properly selected by those skilled in the art. However, for more effective prevention or treatment, the oleanolic acid acetate of the present invention, the salt thereof, the extract or fraction thereof may be administered in an amount of 0.0001 to 100 mg/kg, and preferably 0.001 to 100 mg/kg once or several times a day.

The pharmaceutical composition of the present invention may be administered to mammals such as a rat, a mouse, a domestic animal, a human or the like via various routes. The administration may be carried out through all possible methods, for example, oral administration, rectal administration, intravenous administration, intramuscular administration, subcutaneous administration, intra-endometrial administration, or intracerebroventricular administration.

In still another aspect, the present invention provides a pharmaceutical composition including the adzuki bean extract containing the compound of Chemical Formula 1 or the pharmaceutically acceptable salt thereof, or the fraction thereof as an active ingredient for use in preventing or treating TLR- and IL-6-mediated diseases.

The pharmaceutical composition of the present invention includes the adzuki bean extract containing the compound or the pharmaceutically acceptable salt thereof, or the fraction thereof as an active ingredient, thereby preventing or treating TLR- and IL-6-mediated diseases. The descriptions of the TLR- and IL-6-mediated diseases are the same as mentioned above. When the adzuki bean extract or the fraction thereof are included as an active ingredient, the TLR- and IL-6-mediated diseases may be preferably autoimmune diseases, cancer diseases, or metabolic diseases.

In one embodiment of the present invention, it was confirmed that the adzuki bean extract improved the CIA score in a representative autoimmune disease, arthritis mouse model (FIG. 13), and the adzuki bean extract showed the symptom-improving effect in a representative autoimmune disease, atopic dermatitis animal model (FIGs. 9 and 10), suggesting that the adzuki bean extract is an active ingredient effective for the prevention or treatment of autoimmune diseases. Further, in another embodiment of the present invention, it was confirmed that treatment of the adzuki bean extract reduced the number and size of osteoclasts in a concentration-dependent manner, suggesting that the extract inhibits osteoclast differentiation to show the prophylactic and therapeutic effect on osteoporosis (FIG. 15) .

As used herein, the term "adzuki bean" refers to a seed of Phaseoli angularis Wight belonging to the family Leguminosae. It has morphological traits of slightly flat, oblate shape, and its seed coat is reddish brown, smooth and glossy. In terms of pharmacological actions, it has been used in the folk medicine for the treatment of diuretic, anti-inflammatory, anti-ulcer, antipyretic, anasarca, hepatocirrhosis, jaundice, abscess, purulent disease, dropsy, beriberi, diabetes, dysenteric diarrhea or the like. However, there have been no reports of its use for the treatment of diseases caused by TLR-3 and TLR-7 activation, which was first demonstrated by the present inventors. With respect to the objects of the present invention, the adzuki bean refers to the seed of Phaseoli angularis Wight or Phaseolus calcaratus Roxburgh, but is not limited thereto.

As used herein, the term "adzuki bean extract" refers to an extract obtained by extracting adzuki beans. With respect to the objects of the present invention, the adzuki bean extract may be a resultant that is obtained by extracting adzuki bean powder using water, alcohol having 1 to 6 carbon atoms (methanol, ethanol, butanol, etc.) or a solvent mixture thereof, but is not limited thereto, and the resultant includes all of a liquid extract, a diluted or concentrated liquid extract, a dry product obtained by drying the liquid extract, or a crude purified product or purified product thereof.

According to one embodiment of the present invention, the adzuki bean powder may be extracted by using about 2 to 20-fold, preferably, 3 to 5-fold volume of a polar solvent, for example, water, C1 to C6 alcohol such as methanol, ethanol, butanol, or the mixtures thereof in a ratio of approximately 1:0.1 to 1:10 at a temperature ranging from 20 to 100°C, preferably room temperature, for a period ranging from about 12 hrs to 4 days, preferably 3 days, by hot water extraction, cold-immersion extraction, reflux cold extraction or sonication. Preferably, it may be continuously extracted by cold-immersion extraction once to 5 times, and then filtered under reduced pressure, and the filtrate is concentrated at 20 to 100°C, preferably room temperature under reduced pressure using a rotary vacuum evaporator to obtain an adzuki bean (Phaseoli angularis Wight and Phaseolus calcaratus Roxburgh) crude extract that is solubilized in water, lower alcohol, or a solvent mixture thereof.

As used therein, the term "fraction" refers to a resultant that is obtained by a fractionation method for separating

particular components or particular groups from a mixture containing various components. In the present invention, the fraction may be preferably a resultant that is obtained by fractionating the adzuki bean extract by a solution fractionation method using a solvent such as n-hexane, ethyl acetate or the like, and include all of a polar solvent fraction and a non-polar solvent fraction. Specifically, all of the hexane fraction, the ethyl acetate fraction and the water fraction may be used.

According to one embodiment of the present invention, the adzuki bean crude extract thus obtained was suspended in distilled water, and then a non-polar solvent such as hexane or ethyl acetate is added thereto in a volume of about 1 to 100-fold, preferably about 1 to 5-fold volume of the suspension so as to extract and separate a non-polar solvent soluble layer once to ten times, and preferably twice to five times. A typical fractionation process may be further performed (Harborne J.B. Phytochemical methods: A guide to modern techniques of plant analysis, 3rd Ed. p6-7, 1998). Specifically, the adzuki bean crude extract is suspended in water, and then continuously extracted using an equal volume of n-hexane and ethyl acetate solvent so as to obtain each solvent soluble adzuki bean extract. More specifically, the adzuki bean crude extract is suspended in water, and then mixed with an equal amount of n-hexane, followed by fractionation to obtain an n-hexane soluble fraction and a water soluble fraction. Ethyl acetate was added to this water soluble fraction to obtain an ethyl acetate soluble fraction and a water soluble fraction.

According to one embodiment of the present invention, the present inventors confirmed that the methanol extract, the hexane fraction, the ethyl acetate fraction and the water fraction of adzuki bean significantly inhibited poly(I)-induced SEAP activation (FIG. 2), the methanol extract of adzuki bean significantly inhibited poly(I:C)-induced SEAP activation (FIG. 3), the 95% methanol extract of adzuki bean significantly inhibited poly(I) and poly(I:C)-induced SEAP activation, but the 50% ethanol extract of adzuki bean showed low inhibitory activity (Tables 2 and 3), suggesting that the adzuki bean extracts inhibit poly(I) or poly(I:C)-induced inflammatory factor expression (FIGs. 6 and 7). Therefore, the adzuki bean extract of the present invention inhibits TLR7 and TLR3 signaling pathways induced by ssRNA poly(I) and dsRNA poly(I:C), and the fraction thereof inhibits poly(I)-induced TLR7 signaling pathway, indicating that it is suitable for the prevention or treatment of TLR- and IL-6-mediated diseases.

According to another embodiment of the present invention, the present inventors confirmed that the adzuki bean extracts reduced nuclear translocation of poly(I) and poly(I:C)-activated transcription factors NF-κB (p65, p50) and AP-1 (p-c-Jun) in a concentration dependent manner (FIGs. 4a, 4b, 5a and 5b), also reduced activation of IKK and IκB located upstream of NF-κB in a concentration dependent manner (FIGs. 4c, 4d, 5c and 5d), and reduced phosphorylation of MAP kinase signaling system, JNK and p-38 in a concentration dependent manner (FIGs. 4e and 5e). The adzuki bean extract was also found to show the inhibitory effect on phosphorylation of IRF3 in the poly(I:C)-induced TLR3 signal pathway in a concentration dependent manner FIG. 5f). These results suggest that the adzuki bean extract of the present invention is suitable for the prevention or treatment of TLR- and IL-6-mediated diseases.

According to still another embodiment, the present inventors confirmed that the extract, the hexane fraction, the ethyl acetate fraction and the water fraction of adzuki bean inhibited STAT3 luciferase activity in a concentration dependent manner (Tables 5 and 6), and administration of the adzuki bean extract to an atopic dermatitis animal model improved the symptoms of atopic dermatitis (FIGs. 9 and 10), and also reduced the eosinophil count, compared to the dermatitis-induced and non-treated atopic control group (FIG. 11) . Furthermore, the adzuki bean extracts improved CIA score in an arthritis (collagen-induced arthritis, CIA) mouse model (FIG. 13). Therefore, the adzuki bean extract containing the compound of Chemical Formula 1 of the present invention or the pharmaceutically acceptable salt thereof or the fraction thereof can be used for the prevention or treatment of diseases associated with IL-6-induced STAT3 activation, for example, atopic dermatitis, arthritis or the like.

Also, the pharmaceutical composition including the adzuki bean extract of the present invention or the fraction thereof may further include a proper carrier, excipient or diluent that is typically used in the preparation of the pharmaceutical composition. Descriptions thereof are the same as mentioned above.

In still another aspect, the present invention provides a skin composition for external use including, as an active ingredient, the compound represented by Chemical Formula 1 or the pharmaceutically acceptable salt thereof; or the adzuki bean extract containing the compound represented by Chemical Formula 1 or the pharmaceutically acceptable salt thereof, or the fraction thereof, for use in preventing or improving TLR- and IL-6-mediated diseases.

Descriptions of the compound of Chemical Formula 1, the adzuki bean extract or the fraction thereof are the same as mentioned above, and the active ingredient may be included in the skin composition for external use capable of preventing or improving TLR- and IL-6-mediated diseases. The skin composition for external use may be prepared, but is not particularly limited to, preferably in the form of an ointment, lotion, spray, patch, cream, powder, suspension, gelling agent or gel.

In still another embodiment, the present invention provides a heath functional food including, as an active ingredient, the compound represented by Chemical Formula 1 or the pharmaceutically acceptable salt thereof; or the adzuki bean extract containing the compound represented by Chemical Formula 1 or the pharmaceutically acceptable salt thereof, or the fraction thereof, for use in preventing or improving TLR- and IL-6-mediated diseases.

Descriptions of the compound of Chemical Formula 1, the adzuki bean extract or the fraction thereof are the same as mentioned above, and the active ingredient may be included in the heath functional food capable of preventing

or improving TLR- and IL-6-mediated diseases.

Because the compound of Chemical Formula 1 of the present invention is derived from the extract of adzuki bean that has been conventionally used as medicine, its safety has been approved, and thus it can be used as a food composition. The compound of Chemical Formula 1, the pharmaceutically acceptable salt thereof, the adzuki bean extract including the same or the fraction thereof is included in an amount of 0.01 to 100% by weight, and more preferably 1 to 80% by weight, based on the total weight of the food composition. If the food is a drink, it may be included in an amount of 1~30 g, preferably 3 ~ 20 g, based on 100 ml. In addition, the composition may further include additive ingredient capable of augmenting smell, taste, look, etc. which are commonly used in food compositions. For example, vitamins A, C, D, E, B1, B2, B6, and B12, niacin, biotin, folate, panthotenic acid or the like may be included. Further, minerals such as zinc (Zn), iron (Fe), calcium (Ca), chrome (Cr), magnesium (Mg), manganese (Mn), copper (Cu) or the like may be included. Further, amino acids such as lysine, tryptophan, cysteine, valine or the like may be included. Further, food additives such as preservatives (potassium sorbate, sodium benzoate, salicylic acid, sodium dehydroacetate, etc.), germicides (such as bleaching powder and high-grade bleaching powder, sodium hypochlorite, etc.), antioxidants (butylated hydroxy anisole (BHA), butyl hydroxy toluene (BHT), etc.), the colorant (tar color, etc.), color fixatives (sodium nitrite, sodium nitrite, etc.), bleaching agents (sodium sulfite), seasonings (MSG, sodium glutamate, etc.), sweeteners (dulsin, cyclamate, saccharin, sodium, etc.), flavors (vanillin, lactones, etc.), blowing agents (alum, D-potassium hydrogen tartrate. etc.), enhancers, emulsifiers, thickeners (thickening agents), coating agents, chewing gum bases, anti-foaming agents, solvents, conditioners, may be added. These additives are selected according to the type of food, and used in a proper amount.

The food composition including the compound of Chemical Formula 1, the pharmaceutically acceptable salt thereof, the adzuki bean extract including the same or the fraction thereof is used to prepare various functional foods.

As used herein, the term "functional food" is the same term as food for special health use (FoSHU), and refers to a food having high medicinal and medical effects, which is processed to effectively exert a body-regulating function and to supply nutrients. As used herein, the term "health food" refers to a food that positively maintains or improves health compared to general food, and the term "health supplement food" refers to a food to be used as a health supplement. In some cases, the terms "functional food", "health food" and "health supplement food" are used interchangeably with each other. The food can be prepared in various forms such as tablet, capsule, powder, granule, liquid, pill, etc., so as to provide a useful effect of improving and ameliorating TLR- and IL-6-mediated diseases.

Specific examples of the functional food may include processed foods that are modified to have improved storage while maintaining properties of agricultural products, livestock products or marine products using the composition. The heath functional food including the composition may be, but is not particularly limited to, preferably those prepared in the form of margarines, fat continuous or water continuous or bicontinuous spreads, fat reduced spreads, confectionery products such as chocolate or chocolate coatings or chocolate fillings or bakery fillings, ice creams, ice cream coatings, ice cream inclusions, dressings, mayonnaises, cheeses, cream alternatives, dry soups, drinks, cereal bars, sauces, snack bars, dairy products, clinical nutrition products and infant formulations.

In still another aspect, the present invention provides a personal hygiene product including, as an active ingredient, the compound represented by Chemical Formula 1 or the pharmaceutically acceptable salt thereof; or the adzuki bean extract containing the compound represented by Chemical Formula 1 or the pharmaceutically acceptable salt thereof, or the fraction thereof, for preventing or improving TLR- and IL-6-mediated diseases.

Descriptions of the compound of Chemical Formula 1, the adzuki bean extract or the fraction thereof are the same as mentioned above, and the active ingredient may be included in the personal hygiene product capable of preventing or improving TLR- and IL-6-mediated diseases. In this regard, the personal hygiene product may include, but is not limited to, preferably soap, cosmetic, wet tissue, tissue, shampoo, skin cream, facial cream, toothpaste, lipstick, fragrance, make-up foundation, blusher, mascara, eye shadow, sun screen lotion, hair conditioning products, air freshener gel, washing gel or the like.

Hereinafter, the present invention will be described in more detail with reference to Examples. However, these Examples are for illustrative purposes only, and the invention is not intended to be limited by these Examples.

## Example 1: Preparation of adzuki bean extract and fraction and Isolation and purification of compounds of Chemical Formulae 1 and 2

### Example 1-1: Preparation of adzuki bean extract

Adzuki beans (Phaseoli angularis Wight or Phaseolus calcaratus Roxburgh) were washed with water, dried in the shade, and then pulverized in a waring blender. 20 kg of the pulverized adzuki bean was put in 100 L of methanol, and cold-immersion extraction was performed at room temperature for 3 days. A filter paper (whatman, USA) was used to perform filtration under reduced pressure, and the methanol solvent was removed from the filtered extract using a rotary vacuum evaporator at room temperature, so as to prepare 450 g of adzuki bean extract as an extraction residue.

**Example 1-2: Preparation of fraction**

In order to isolate an active fraction from the prepared adzuki bean extract, adzuki bean extract was suspended in 1 L of water, and an equal volume of n-hexane was added thereto, and mixed, followed by fractionation. This procedure was repeated four times, and 1 L of a water soluble fraction and 4 L of n-hexane soluble fraction were obtained.
Subsequently, the n-hexane soluble fraction was concentrated under reduced pressure to obtain 50 g of n-hexane soluble extract.
Further, an equal volume of ethyl acetate was added to 1 L of the water soluble fraction, and mixed with each other, followed by fractionation. This procedure was repeated three times, and 1 L of a water soluble fraction and 3 L of ethyl acetate soluble fraction were obtained.
The ethyl acetate soluble fraction thus obtained was concentrated under reduced pressure so as to obtain 35 g of an ethyl acetate soluble extract, and the remaining water soluble fraction was concentrated under reduced pressure to obtain 35 g of a water fraction.

**Example 1-3: HPLC analysis of adzuki bean extract and fraction**

Each of the adzuki bean extracts and fractions obtained in Examples 1-1 and 1-2 was subjected to HPLC analysis.
At this time, Agilent Technologies 1200 series was used for HPLC, and YMC J'sphere ODS-H80 (YMC, 4 μm, 4.6 mm I.D. x 150 mm) column was used as an analysis column (FIGs. 1a and 1b). At this time, 5%~90% $CH_3CN$ was applied as an analysis solvent at a flow rate of 1 ml/min, and analysis was performed at 210 nm and 10 μl of the sample was injected (Table 1).

[Table 1]

| Solvent conditions | | |
|---|---|---|
| Time (min) | Solvent (%) | |
| | $CH_3CN$ | $H_2O$ |
| 0 | 5 | 95 |
| 15 | 5 | 95 |
| 25 | 30 | 70 |
| 30 | 30 | 70 |
| 50 | 90 | 10 |
| 70 | 90 | 10 |

FIG. 1a is a chromatogram showing the results of HPLC of the methanol extract, the hexane fraction, the ethyl acetate fraction and the water fraction of Phaseoli angularis Wight, and FIG. 1b is a chromatogram showing the results of HPLC of the methanol extract, the hexane fraction, the ethyl acetate fraction and the water fraction of Phaseolus calcaratus Roxburgh.
As shown in FIGs. 1a and 1b, the peaks of catechin-7-glucopyranoside (catechin-7-glu), rutin, oleanolic acid acetate (OAA) and stigmasterol appeared at 5.5, 24.5, 35.5, 35.5 minutes, respectively and HPLC histograms of Phaseoli angularis Wight and Phaseolus calcaratus Roxburgh showed similar pattern.

**Example 1-4: Purification of active ingredient**

80 g of the n-hexane fraction obtained in Example 1-2 was applied to silica gel column chromatography using a step gradient solvent system consisting of hexane:ethyl acetate (100:1 → 1:1) to obtain 5 active fractions (Fr.1-5). Recrystallization was performed by adding methanol to Fraction 3 and Fraction 4 among the active fractions, thereby purifying 2 types of compounds as a white powder.
The 2 types of compounds thus purified were applied to instrumental analysis (1H-, 13C-NMR, MS) and reference values (Voutquenne L. et al. Phytochemistry 2003, 64, 781?789; Kongduang D. et al. Tetrahedron letters 2008, 49, 4067-4072) to identify oleanolic acid acetate (Chemical Formula 1) and stigmasterol (Chemical Formula 2), respectively.

Chemical Formula 1: oleanolic acid acetate

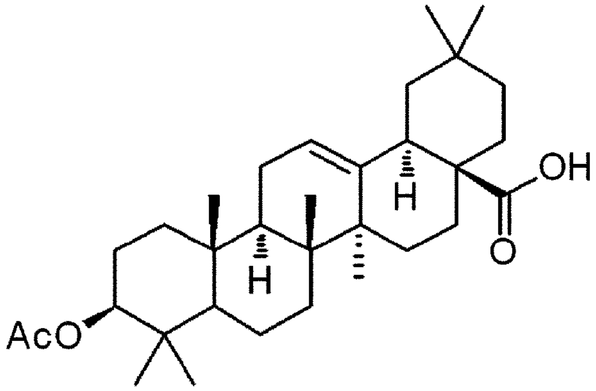

4aS,6aR,6aS,6bR,8aR,10S,12aR,14bS)-10-hydroxy-2,2,6a,6b,9,9,12a-heptamethyl-1,3,4,5,6,6a,7,8,8a,10,11,12,13,14b-tetradecahydropicene-4a-carboxylic acid acetate

Chemical Formula 2: stigmasterol

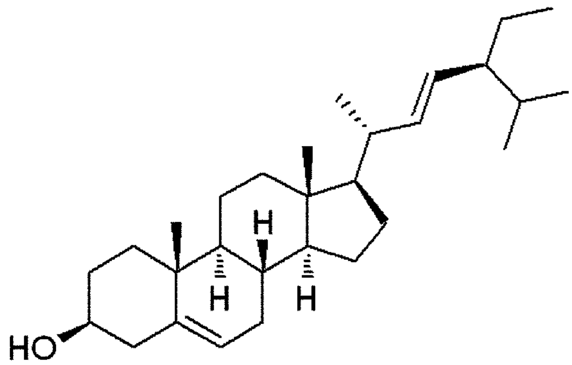

(3S,8S,9S,10R,13R,14S,17R)-17-[(E,2R,5S)-5-ethyl-6-methylhept-3-en-2-yl]-10, 13-dimethyl-2,3,4,7,8,9,11,12,14,15,16,17-dodecahydro-1H-cyclopenta[a]phenanthren-3-ol

## Example 2: Inhibitory effect of adzuki bean extract, fraction and compound on poly(I) or poly(I:C)-treated NFkB/AP-1 dependent SEAP activity

THP1-Blue-CD14 cells were seeded at a density of $2x10^5$ cells/100 μl in a 96-well plate, and cultured at 37°C for 3 hours.

As samples, cell culture media were pretreated with the adzuki bean extracts and fractions obtained in Examples 1-1 to 1-2 for 1 hr, and 50 μg/ml of Poly(I) or 100 μg/ml of poly(I:C) were treated thereto, and then inhibitory effects on NFkB/AP-1-dependent SEAP activity were measured (FIGs. 2 and 3).

FIG. 2 is a graph showing the inhibitory effect of the methanol extract, the hexane fraction, the ethyl acetate fraction and the water fraction of adzuki bean on SEAP activity increased by poly(I) treatment in THP-1-blue cells, and FIG. 3 is a graph showing the inhibitory effect of the adzuki bean methanol extract on poly(I:C)-induced SEAP activity in THP-1-blue cells.

As shown in FIG. 2, the methanol extract, the hexane fraction, the ethyl acetate fraction and the water fraction of adzuki bean significantly inhibited poly(I)-induced SEAP activity. As shown in FIG. 3, the adzuki bean methanol extract significantly inhibited poly(I:C)-induced SEAP activity.

Further, in order to examine the difference according to the sources of the adzuki bean extract, inhibitory effects of the extract derived from the Phaseoli angularis Wight and the 95% and 50% methanol extracts derived from Phaseolus calcaratus Roxburgh on poly(I) and poly(I:C)-induced SEAP activity were measured (Tables 2 and 3).

[Table 2]

| Inhibitory effect on poly(I)-induced SEAP activity | | |
|---|---|---|
| Sample ($\mu$g/ml) | | Inhibition (%) |
| 95% methanol extract of Phaseoli angularis Wight | 100 | 27.09 |
| | 60 | 12.58 |
| | 30 | - |
| 50% methanol extract of Phaseoli angularis Wight | 100 | 3.07 |
| | 60 | 8.79 |
| | 30 | 15.23 |
| 95% methanol extract of Phaseolus calcaratus Roxburgh | 100 | 41. 66 |
| | 60 | 27.21 |
| | 30 | 16.80 |
| 50% methanol extract of Phaseolus calcaratus Roxburgh | 100 | 17.04 |
| | 60 | 24.92 |
| | 30 | 26.07 |
| Luteolin ($\mu$M) | 60 | 96.27 |
| | 30 | 79.23 |

[Table 3]

| Inhibitory effect on poly(I:C)-induced SEAP activity | | |
|---|---|---|
| Sample ($\mu$g/ml) | | Inhibition (%) |
| 95% methanol extract of Phaseoli angularis Wight | 100 | 71.61 |
| | 60 | 64.64 |
| | 30 | 36.96 |
| 50% methanol extract of Phaseoli angularis Wight | 100 | 16.07 |
| | 60 | 30.71 |
| | 30 | 10.71 |
| 95% methanol extract of Phaseolus calcaratus Roxburgh | 100 | 87.50 |
| | 60 | 82.14 |
| | 30 | 68.57 |
| 50% methanol extract of Phaseolus calcaratus Roxburgh | 100 | 27.68 |
| | 60 | 27.68 |
| | 30 | 7.14 |
| Luteolin ($\mu$M) | 60 | 67.32 |
| | 30 | 47.68 |

As shown in Tables 2 and 3, irrespective of the type of adzuki bean (Phaseoli angularis Wight or Phaseolus calcaratus Roxburgh), the 95% methanol extract significantly inhibited poly(I) and poly(I:C)-induced SEAP activity, but the 50% ethanol extract showed the low inhibitory effect.

Meanwhile, the effects of oleanolic acid acetate of Chemical Formula 1 or stigmasterol of Chemical Formula 2 purified from hexane fraction on poly(I) and poly(I:C)-induced SEAP activity were examined (Table 4).

[Table 4]

| Effect of oleanolic acid acetate or stigmasterol on poly(I) and poly(I:C)-induced SEAP activity | | | |
|---|---|---|---|
| Sample (μM) | | Inhibition (%) | |
| | | poly(I:C) | poly(I) |
| Oleanolic acid acetate | 100 | 64.39 | 83.01 |
| | 60 | 47.16 | 74.58 |
| | 30 | 9.28 | 43.90 |
| | 10 | - | 2.02 |
| Stigmasterol | 100 | - | 1.42 |
| | 60 | 4.92 | 2.63 |
| | 30 | 8.71 | - |
| Catechin-7-glucopyranoside | 60 | 11.16 | - |
| | 30 | 13.19 | - |
| Rutin | 60 | - | - |
| | 30 | - | - |

As shown in Table 4, oleanolic acid acetate significantly inhibited poly(I) and poly(I:C)-induced SEAP activity, but stigmasterol did not show the inhibitory activity on poly(I) and poly(I:C)-induced SEAP activity. Moreover, the inhibitory activity of catechin-7-glucopyranoside and rutin known as main compounds of adzuki bean was examined. As a result, they did not affect poly(I) and poly(I:C)-induced SEAP activity.

## Example 3: Effect of adzuki bean methanol extract on poly(I) or poly(I:C)-induced inflammatory factor expression

In order to measure the poly(I) and poly(I:C)-induced inflammatory factor expression of the adzuki bean methanol extract, Human peripheral blood monocytic cell line, THP-1 was used to perform the following experiment.

First, in order to measure the inhibitory effect on the poly(I) or poly(I:C)-induced inflammatory factor, THP-1 cells were seeded at a density of $5x10^5$ per well in a 12-well plate, and cultured in FBS-free RPMI 1640 (100 units of penicillin, 100 μg of streptomycin). 12 hours later, the adzuki bean methanol extract was diluted to a final concentration of 60 μg/ml using DMSO (dimethylsulfoxide) and pretreated thereto for 1 hour, and 50 μg/ml of poly(I) or 100 μg/ml of poly(I:C) was treated to perform cultivation at 37°C for 6 hours. After completing the cultivation, cells were collected in a tube, and centrifuged to discard the medium. The cell pellet was washed with PBS once, and then RNA was extracted using an RNeasy Mini Elute Cleanup kit. The RNA concentration and purity were measured using a 2100 Bioanalyzer system (Agilent Technologies), and a Taqman reverse transcription reagent kit (Applied Biosystems) was used to synthesize cDNA. Expressions of inflammatory factors were measured using a SYBR Green PCR master mix kit (Applied Biosystem) by Real-time PCR (FIGs. 6 and 7).

FIG. 6 is a graph showing the inhibitory effect of adzuki bean methanol extract on poly(I)-induced inflammatory cytokine expression in THP-1 cells, and FIG. 7 is a graph showing the inhibitory effect of adzuki bean methanol extract on poly(I:C)-induced inflammatory cytokine expression in THP-1 cells. As shown in FIGs. 6 and 7, expressions of the inflammatory factors TNFα, IL-6, MCP-1, RANTES, and IFNβ were significantly increased in 50 μg/ml of poly(I)-treated group, compared to a control group. Expressions of the inflammatory factors TNF-α, IL-6, ICAM-1, and MCP-1 were significantly increased in 100 μg/ml of poly(I:C)-treated group, compared to a control group.

In contrast, the levels of all the inflammatory factors were significantly inhibited in the group pretreated with the adzuki bean methanol extract (60 μg/ml) for 1 hour, suggesting that the adzuki bean methanol extract inhibits poly(I) or poly(I:C)-induced inflammatory factor expression.

## Example 4: TLR-7 and TLR-3 signaling pathways regulated by adzuki bean methanol extract

When each ligand binds to TLRs (Toll-like receptors), MyD88 or TRIF adaptor moves to TLRs. When ssRNA and dsRNA as ligands bind to TLR7 and TLR3, respectively, intracellular signal transduction is initiated to activate NFkB and MAPK/AP-1 at the same time, and TLR3 further activates IRF-3 pathway to produce inflammatory cytokines. Therefore, the effects of the adzuki bean extract of the present invention on TLR-7 and TLR-3 signaling pathways were examined by the following experiment.

First, THP-1 cells were seeded at a density of $1.5 \times 10^6$ per well, and cultured in FBS-free RPMI 1640 medium for 12 hours. Thereafter, the cells were pretreated with 30, 60 or 100 μg/ml of the extract for 1 hour, and 50 μg/ml of poly(I) or 100 μg/ml of poly(I:C) were treated thereto for 3 hours. 3 hours later, cells were transferred to a tube, and the medium was removed by centrifugation. The cell pellet was washed with PBS once, and nuclear extract and total protein were extracted using NE-PER Nuclear extraction reagents (Thermo scientific) and cell lysis buffer to perform Western blot analysis. NFkB p65/p50 and p-c-Jun were examined in the nuclear extract, and MAPKs, IKB, IKK isoforms, and IRF3 were measured in the total protein (FIGs. 4 and 5).

FIG. 4a is a Western blot image showing the inhibitory effect of the adzuki bean methanol extract on NFκB P50 that is activated by poly(I) treatment and translocates to the nucleus in THP-1 cells, FIG. 4b is a Western blot image showing the inhibitory effect on C-Jun activity of AP-1, FIG. 4c is a Western blot image showing the inhibitory effect on IKK α/β phosphorylation, FIG. 4d is a Western blot image showing the inhibitory effect on IκB phosphorylation, and FIG. 4e is a Western blot image showing the inhibitory effect on p38 and JNK phosphorylations.

Further, FIG. 5a is a Western blot image showing the inhibitory effect of the adzuki bean methanol extract on NFκB P65/p50 that is activated by poly(I:C) treatment and translocates to the nucleus in THP-1 cells, FIG. 5b is a Western blot image showing the inhibitory effect on C-Jun activity of AP-1, FIG. 5c is a Western blot image showing the inhibitory effect on IKK α/β phosphorylation, FIG. 5d is a Western blot image showing the inhibitory effect on IκB phosphorylation, FIG. 5e is a Western blot image showing the inhibitory effect of the adzuki bean methanol extract on poly(I)-induced p38 and JNK phosphorylation in THP-1 cells, and FIG. 5f is a Western blot image showing the inhibitory effect on IRF3 phosphorylation.

As shown in FIGs. 4 and 5, the adzuki bean extract reduced poly(I) and poly(I:C)-induced NFkB and p-c-Jun nuclear translocation in a concentration dependent manner (FIGs. 4a, 4b, 5a and 5b), and also reduced activation of IKK and IkB in the upstream of NFkB in a concentration dependent manner (FIGs. 4c, 4d, 5c and 5d). The extract also reduced activation of MAPKs, namely, JNK and p-38 in the upstream of AP-1 activation in a concentration dependent manner (FIGs. 4e and 5e), and the adzuki bean extract also showed inhibitory activity on P-IRF3 activation in TLR3 signaling pathway in a concentration dependent manner (FIG. 5f) .

Taken together, the adzuki bean extracts inhibit NFkB and MAPKs/ AP-1 activations in the TLR-7 and TLR-3 signaling pathways, thereby inhibiting expressions of inflammatory factors.

**Example 5: Inhibitory effect of adzuki bean extract, fraction and compound on IL-6-induced STAT3 transcriptional activation**

Inhibitory effects of the adzuki bean extracts, fractions and compounds purified therefrom on IL-6-induced STAT3 activation were examined.

**Example 5-1: Preparation of transformant**

pStat3-Luc and pcDNA3.1 (+) (Clontech laboratories, Palo Alto, CA) were transfected into Hep3B cells (ATCC HB-8064) using lipofectamine plus (Invitrogen, Carlsbad, CA, USA) at the same time. 2 days later, the cells were treated with hygromycin (100 μg/ml) to obtain clones stably expressing luciferase. Luciferase analysis was performed to examine whether luciferase is stably expressed in the clones.

**Example 5-2: Examination of IL-6 reactive STAT3 reporter gene**

The transfected cells in the clones obtained in Example 5-1 were cultured in DMEM (GIBCO 119950965) under serum starvation, and the samples were treated for 1 hour as follows. 10 ng/ml IL-6 (R&D system, USA) was added thereto, followed by cultivation for 12 hours.

1: negative control (non-treatment group);
2: positive control (IL-6 10 ng/ml); and
3: fraction (10, 30, 60 μg/ml) and compound.

The cultured cells were washed with PBS and 50 μl of lysis buffer (luciferase assay system, promega, USA) was added thereto, followed by stirring for 20 minutes. 30-100 μl of luciferase substrate (luciferase assay system, promega, USA) was added to measure color development using a luminometer (EG&G BERTHOLD, USA) within 5 minutes (Tables 5 and 6) .

[Table 5]

| Inhibitory effect of methanol extract, and hexane, ethyl acetate, butanol and water fractions of adzuki bean on STAT3 luciferase activity | | | |
|---|---|---|---|
| Sample (μg/ml) | | Inhibition (%) | Cell viability (%) |
| Methanol | 60 | 34.3 | 98.5 |
| | 30 | 31.1 | 97.0 |
| | 10 | 13.9 | 94.8 |
| | 6 | 3.3 | 93.5 |
| Hexane | 60 | 102.2 | 94.1 |
| | 30 | 96.2 | 99.4 |
| | 10 | 53.2 | 101.5 |
| | 6 | 18.2 | 103.0 |
| Ethanol | 60 | 100.9 | 93.7 |
| | 30 | 98.6 | 98.9 |
| | 10 | 74.4 | 98.5 |
| | 6 | 52.1 | 96.7 |
| Butanol | 60 | 25.1 | 105.2 |
| | 30 | 25.8 | 101.1 |
| | 10 | 5.8 | 97.3 |
| | 6 | - | 94.3 |
| Distilled water | 60 | 27.2 | 89.0 |
| | 30 | 30.2 | 91.2 |
| | 10 | 17.3 | 95.2 |
| | 6 | 9.4 | 93.1 |
| Genistein (μM) | 100 | 95.2 | - |

As shown in Table 5, the methanol extract, the hexane fraction, the ethyl acetate fraction and the water fraction of adzuki bean inhibited STAT3 luciferase activity in a concentration dependent manner.

[Table 6]

| Inhibitory effect of 95% and 50% methanol extracts of Phaseoli angularis Wight and Phaseolus calcaratus Roxburgh on STAT3 luciferase activity | | | |
|---|---|---|---|
| Sample (μg/ml) | | Inhibition (%) | Cell viability (%) |
| 95% methanol extract of Phaseoli angularis Wight | 60 | 37.7 | 94.1 |
| | 30 | 5.9 | 91.4 |
| 50% methanol extract of Phaseoli angularis Wight | 60 | 24.9 | 96.3 |
| | 30 | - | 99.9 |
| 95% methanol extract of Phaseolus calcaratus Roxburgh | 60 | 68.3 | 87.3 |
| | 30 | 34.8 | 89.5 |
| 50% methanol extract of Phaseolus calcaratus Roxburgh | 60 | 22.2 | 101.2 |
| | 30 | 8.7 | 104.3 |

(continued)

| Inhibitory effect of 95% and 50% methanol extracts of Phaseoli angularis Wight and Phaseolus calcaratus Roxburgh on STAT3 luciferase activity | | | |
|---|---|---|---|
| Sample (μg/ml) | | Inhibition (%) | Cell viability (%) |
| Genistein (μM) | 100 | 95.1 | - |

As shown in Table 6, 95% and 50% methanol extracts of Phaseoli angularis Wight and Phaseolus calcaratus Roxburgh inhibited STAT3 luciferase activity in a concentration dependent manner.
Meanwhile, inhibitory effects of the oleanolic acid acetate and stigmasterol purified from hexane fraction on STAT3 luciferase activity were measured (Table 7).

[Table 7]

| Inhibitory effect of oleanolic acid acetate and stigmasterol on STAT3 luciferase activity | | |
|---|---|---|
| Sample (μM) | Inhibition (%) | |
| Oleanolic acid acetate | 30 | 100 |
| | 10 | 97.2 |
| | 6 | 95.1 |
| | 3 | 84.3 |
| | 1 | 51.2 |
| Stigmasterol | 100 | 58.4 |
| | 60 | 29.4 |
| | 30 | - |
| | 10 | - |
| Catechin-7-glucopyranoside | 100 | 0.4 |
| | 60 | 15.9 |
| | 30 | 8.3 |
| | 10 | - |
| Rutin | 100 | - |
| | 60 | - |
| | 30 | - |
| | 10 | - |
| Genistein | 100 | 95.4 |

As shown in Table 7, oleanolic acid acetate and stigmasterol inhibited STAT3 luciferase activity in a concentration dependent manner, and $IC_{50}$ values were measured as <1 μM and 90 μM, respectively. However, catechin-7-glucopyranoside and rutin known as main compounds of adzuki bean did not show the inhibitory effect on STAT3 luciferase activity.

**Example 5-3: Examination of inhibitory activity on IL-6-induced STAT3 phosphorylation**

Hep3B cells were seeded in a 6-well plate at a density of $5\times10^5$ per well, and cultured to a confluency of 80% in a culture plate. Then, the medium was replaced with serum-free medium and further cultured for 12 hours, and the samples were treated for 60 minutes as follows.

1: negative control (non-treatment group);
2: positive control (IL-16 10 ng/ml);

3: fraction (10, 30 or 60 μg/ml) and compound 1 (3, 6, 10 or 30 μM)
4: genistein-treated group (100 μM).

Next, 20 ng/ml of IL-6 was treated, and reacted for 20 minutes, and 40 μl of lysis buffer (pH 8, 20 mM Tris-HCl, 137 mM NaCl, 10% glycerol, 1% Triton X-100, 1 mM $Na_3VO_4$, 2 mM EDTA, 1 mM APMSF, 20 μM leupeptin, 20 μg/ml aprotonin; Sigma, USA) was used for cell lysis. Centrifugation (13000 g, 15 minutes) was performed to obtain supernatants containing proteins. Protein concentrations were quantified using a DC protein assay kit (Bio-Rad, USA), and proteins were loaded in a 4~12% SDS polyacrylamide gel (SDS-PAGE) and electrophoresis was performed at 175 mA for 2 hours. After electrophoresis, the proteins of the gel were transferred onto a PVDF membrane (Westran S, pore size 0.2 mm; Whatman, USA) at 35 V for 90 minutes. The transferred membrane was blocked with Tris-buffer (T-TBS; 50 mM Tri-HCl, pH 7.6, 150 mM NaCl, 0.2% Tween-20, 5% skim milk; Sigma, USA) at room temperature for 1 hour, and washed with T-TBS five times. The membrane was treated with polyclonal antibody of phospho-STAT3 (1:1000 dilution) as a primary antibody at 4°C 12 hours. The membrane was washed with T-TBS five times, and reacted with HRP-conjugated anti-mouse antibody (1:5000 dilution) as a secondary antibody for 1 hour. The membrane was washed with T-TBS, and then a film was developed in a dark room using an ECL kit (Amersham, USA) (FIGs. 8a and 8b).

FIG. 8a is a photograph showing the inhibitory effect of the adzuki bean methanol extract on IL-6-induced STAT3 phosphorylation in Hep3B cells, and FIG. 8b is a photograph showing the inhibitory effect of Compound 1 isolated and purified from adzuki bean on IL-6-induced STAT3 phosphorylation in Hep3B cells.

As shown in FIGs. 8a and 8b, the adzuki bean extract and the compound of Chemical Formula 1 showed the inhibitory effects on IL-6-indcued STAT3 phosphorylation.

**Example 6: Symptom-improving effect of adzuki bean methanol extract on atopic dermatitis**

Specific Pathogen Free (SPF) NC/Nga mouse used as an experimental animal model was purchased from SLC, Inc. (Japan) and these mice were raised at a constant temperature (23±3°C), humidity (55±15%) and light-dark cycle (from 7:00 to 19:00) for experiment. After purchase, 8-week old female NC/Nga mice were stabilized in an SPF animal facility for 1 week, and used for experiment.

NC/Nga mice were largely divided into three experimental groups. Specifically, ①non-atopic dermatitis-induced negative control group, ② atopic dermatitis-induced and non-drug treated control group, and ③ atopic dermatitis-induced and drug-treated control group were prepared.

As the sample for activity evaluation, adzuki bean methanol extract was dissolved in 100% ethanol, and the added to feed, and the subjects were fed at a variety of doses for 2 weeks. 2 weeks later, in order to induce atopic dermatitis, 1% concentration of DNCB (2,4-Dinitrochlorobenzene) was diluted to 0.2%, and 100 μl thereof was applied to the back skin of NC/Nga mouse every 4 days during the entire experimental period for sensitization. After applying DNCB, NC/Nga mice in non-drug treated and drug-treated atopic dermatitis-induced groups were compared to examine skin severity.

With respect to skin severity of atopic dermatitis, erythema, edema, dryness, and excoriation were used as clinical indices, and each symptom was scored. Then, these values were expressed as a mean value of the experimental group. The scores for each symptom were recorded as mild (1 point), moderate (2 points), severe (3 points), and skin severity was calculated by the following Equation (FIGs. 9 and 10).

$$\text{Skin severity} = \sum(\text{erythema} + \text{edema} + \text{dryness} + \text{excoriation})\text{ of subject/number of subject}$$

FIG. 9 is a photograph showing the back skin of NC/Nga mouse, in which the adzuki bean methanol extract showed the effect of improving the symptoms of DNCB-induced atopic dermatitis in NC/Nga mouse, and FIG. 10 is a graph showing skin severity, in which the adzuki bean methanol extract showed the effect of improving the symptoms of DNCB-induced atopic dermatitis in NC/Nga mouse.

As shown in FIGs. 9 and 10, no clinical symptoms including atopic dermatitis were observed in the non-atopic dermatitis induced negative control group, but the symptoms of atopic dermatitis including erythema and edema were observed in the atopic dermatitis induced and non-drug-treated group 7 days after DNCB application.

In contrast, when atopic dermatitis-induced and drug-treated group were ingested with the adzuki bean methanol extract at a concentration of 50 mg/kg, 250 mg/kg, 500 mg/kg for a day, symptoms of atopic dermatitis including erythema and edema were improved in all treatment groups, compared to the atopic dermatitis-induced and non-drug treated control group.

## Example 7: Reduction of blood eosinophil count by adzuki bean methanol extract

Changes in the eosinophil count were examined in the non-atopic dermatitis-induced negative control group, atopic dermatitis-induced and non-drug-treated control group, and atopic dermatitis-induced and drug-treated group of Example 6.

In detail, NC/Nga mice of each experimental group were sacrificed at 22 days after initial DNCB application, and the blood eosinophil count was measured using an automated hematology analyzer (FIG. 11).

FIG. 11 is a graph showing the effect of the adzuki bean methanol extract on changes in the blood eosinophil count according to DNCB-induced atopic dermatitis of NC/Nga. As shown in FIG. 11, the eosinophil count was increased in atopic dermatitis-induced and non-drug-treated control group, compared to the non-atopic dermatitis induced negative control group. When atopic dermatitis-induced and drug-treated group was ingested with the adzuki bean methanol extract at a concentration of 50 mg/kg, 250 mg/kg, 500 mg/kg for a day, the eosinophil count was reduced in all treatment groups, compared to the atopic dermatitis-induced and non-drug treated group.

## Example 8: Symptom improvement of collagen-induced CIA arthritis mouse by adzuki bean methanol extract

First, female 6-week-old DBA/1 OlaHsd mice (15~20 g) were supplied by Harlan (San Jese, CA, USA), and animals were sufficiently fed with solid feed (antibiotic-free, Samyang feed Co.) and water until the start point of the experiment, and adapted at a temperature of 22±2°C, humidity of 55±15%, and light-dark cycle of 12 hr for 1 week, and then used for experiment.

Next, the adapted DBA/1 OlaHsd mice were used to prepare rheumarthritis mouse (CIA) model.

In detail, 6 DBA/1 OlaHsd mice were used per 1 group, and the groups were divided into a non-arthritis induced normal group, a CIA-induced and non-drug treated control group, a methotrexate (2 mg/kg)-treated group, and an adzuki bean methanol extract-treated group (100 mg/kg). CIA induction was performed by subcutaneous injection of a mixture of 100 μg of bovine type 2 collagen and 0.1 ml of adjuvant (Complete Freund's Adjuvants) into the base of tail of 8-week-old DBA/1 OlaHsd mice and boosting with an equal amount thereof after 21 days.

Finally, the adzuki bean methanol extract was administered to the CIA model thus prepared, and analysis of arthritis index (AI) and incidence was performed.

In detail, the normal group and the control group were orally administered with 0.1 ml of a saline solution once a day, and MTX was treated at a concentration of 2 mg/kg. The adzuki bean methanol extract (100 mg/kg)-treated group was orally administered at AM 11 everyday. Subsequently, boosting was performed using type 2 collagen, and incidence (%) was determined based on arthritis signs in 4 paws of DBA/1 OlaHsd mice by the following criteria, and the symptoms of the mice were recorded according to the CIA index shown in the following Table 8 once a week (FIGs. 12 and 13) .

[Table 8]

| CIA index | |
|---|---|
| Arthritis index | Criteria |
| 0 | No edema or erythema in small joints (phalanges, metacarpophalangeal joints (carpometacarpal joints, phalangeal joints, metatarsophalangeal joints) or large joints (wrist/carpal bones, ankle/tarsal bone) |
| 1 | Slight edema and/or erythema in small or large joints |
| 2 | Moderate edema and/or erythema in one or more small or large joints |
| 3 | Severe edema and erythema in large joints and moderate edema and/or erythema in small joints |
| 4 | Very severe edema and erythema in large joints and severe edema and/or erythema in small joints |

FIG. 12 is a photograph showing the forelegs and hind legs of normal DBA/1 OlaHsd and CIA-induced mouse model and FIG. 13 is a CIA index showing the improvement effect of the adzuki bean methanol extract on CIA-induced arthritis of DBA/1 OlaHsd mice.

As shown in FIG. 13, the adzuki bean methanol extract showed the effect of improving CIA induction in CIA-induced arthritis DBA/1 OlaHsd mice model, compared to the control group.

**Example 9: Comparison of oleanolic acid and oleanolic acid acetate activities by examining inhibitory activity on IL-6-induced STAT3 phosphorylation**

In the above Examples, it was confirmed that adzuki bean-derived oleanolic acid acetate of the present invention shows the prophylactic or therapeutic effect on TLR- and IL-6-mediated diseases. In order to examine that oleanolic acid acetate of the present invention is a specific derivative having higher effect than the known oleanolic acid, a difference in the activity between oleanolic acid acetate of the present invention and the known oleanolic acid acetate was examined by the method of Example 5-2.

In detail, the inhibitory effects of oleanolic acid or oleanolic acid acetate on STAT3 luciferase activity was measured in the same manner as in Example 5-2, except that oleanolic acid or oleanolic acid acetate was treated as a sample in an amount of 0.1, 1, 3, 6 or 10 μM, respectively and compared with each other (FGI. 14).

FIG. 14 is a graph showing the inhibitory effect of each concentration of oleanolic acid or oleanolic acid acetate on IL-6-induced STAT3 luciferase activity in Hep3B cells. As shown in FIG. 14, oleanolic acid acetate of the present invention showed the inhibitory activity of $IC_{50}$=0.9 μM, whereas the known oleanolic acid acetate showed only approximately 14% inhibitory activity even at 10 μM.

Therefore, the oleanolic acid acetate of the present invention is able to show remarkably excellent prophylactic and therapeutic effects on inflammatory diseases, compared to the known oleanolic acid.

**Example 10: Effect of adzuki bean extract and oleanolic acid acetate on RNAKL-induced osteoclast differentiation**

Femurs and tibia were removed from 5-week-old mice, and the bone cavity was washed using a 1 cc syringe to obtain bone marrow cells. The isolated bone marrow cells were cultured in α-MEM (α-minimum essential medium) containing 10% FBS, antibiotic, M-CSF (30 ng/ml) for 3 days. 3 days later, the adherent cells were used as bone marrow macrophage (BMM). The bone marrow macrophages were cultured by addition of M-CSF (30 ng/ml) and an osteoclast differentiation factor RNAKL (Receptor activator of nuclear factor kappa-B ligand) (50 ng/ml) and treated with the adzuki bean extract and oleanolic acid acetate at each concentration. 4 days later, the cultured cells were stained with a TRAP solution (Sigma Aldrich, USA), and red stained cells were determined as differentiated osteoclasts.

As a result, osteoclast differentiation was inhibited in the adzuki bean extract- and oleanolic acid acetate-treated experimental groups. FIGs. 15 and 16 are photographs showing the inhibitory effects of adzuki bean methanol extract and oleanolic acid acetate on differentiation of osteoclasts from the bone marrow macrophages isolated from mouse bone marrow cells. As shown in FIG. 15, multinucleated TRAP-positive osteoclasts was observed in the non-adzuki bean extract-treated control group, but the number and size of osteoclast were reduced in a concentration-dependent manner in the adzuki bean extract-treated experimental group. In particular, osteoclast differentiation was remarkably reduced at a concentration of 50 μg/ml. As shown in FIG. 16, osteoclast differentiation was also remarkably inhibited in the oleanolic acid acetate-treated group. In particular, a remarkable effect was observed even at a low concentration of 10 μM.

Therefore, the adzuki bean extract and oleanolic acid acetate of the present invention inhibit osteoclast differentiation, thereby showing remarkable prophylactic and therapeutic effects on osteoporosis.

**Preparation Example 1: Preparation of powder**

0.1 g of oleanolic acid acetate, the adzuki bean extract including the same, or the fraction thereof, 1.5 g of lactose and 0.5 g of talc were mixed and filled in the air-tight bag to prepare a powder.

**Preparation Example 2: Preparation of tablet**

0.1 g of oleanolic acid acetate, the adzuki bean extract including the same, or the fraction thereof, 7.9 g of lactose, 1.5 g of crystalline cellulose, and 0.5g of magnesium stearate were mixed, and a 500 mg-tablet containing 50 mg of the active ingredient was prepared by a direct tableting method).

**Preparation Example 3: Preparation of powder**

0.1 g of oleanolic acid acetate, the adzuki bean extract including the same, or the fraction thereof, 5 g of corn starch, and 4.9 g of carboxy cellulose were mixed well to prepare a powder, and 500 mg of the powder was put in a soft capsule to prepare a capsule.

**Preparation Example** 4: **Preparation** of **injectable formulation**

According to the conventional preparation method of injectable formulations, 2 ml-volume ampule for injection containing 0.1 g of oleanolic acid acetate, the adzuki bean extract including the same, or the fraction thereof, a proper amount of sterile distilled water for injection and a pH adjuster was prepared.

**Preparation Example** 5: **Preparation** of **liquid formulation**

According to the conventional preparation method of liquid formulations, 0.1 g of oleanolic acid acetate, the adzuki bean extract including the same, or the fraction thereof, 10 g of high fructose corn syrup and 5 g of mannitol were added to and dissolved in purified water, and a proper amount of lemon flavor was added thereto. The ingredients were mixed, and then the total volume was adjusted to 100 ml by adding purified water. The mixture was filled in a brown bottle, and sterilized to prepare a liquid formulation.

**Effect of the Invention**

The adzuki bean extract or the compound of the present invention is derived from a natural resource that has been used as a natural medicine for a long time, and it inhibits TLR-3 and TLR-7 activations induced by Poly(I:C) and Poly(I) which are synthetic analogues of dsRNA and ssRNA, blocks NF-κB-including signal transduction pathways, and inhibits transcriptional activation and phosphorylation of IL-6-activated inflammatory transcription factor STAT3 without causing side effects, thereby being widely used for the development of prophylactic or therapeutic agents for TLR- and IL-6-mediated diseases, for example, atopic dermatitis or arthritis.

**Claims**

1. A compound represented by formula 1 or a pharmaceutically acceptable salt thereof for use in the prevention, improvement or treatment of TLR- and IL-6-mediated diseases selected from the group consisting of autoimmune diseases, cancer diseases and metabolic diseases, wherein the metabolic diseases are selected from the group consisting of osteoporosis, arteriosclerosis and myocardial infarction

[Chemical Formula 1]

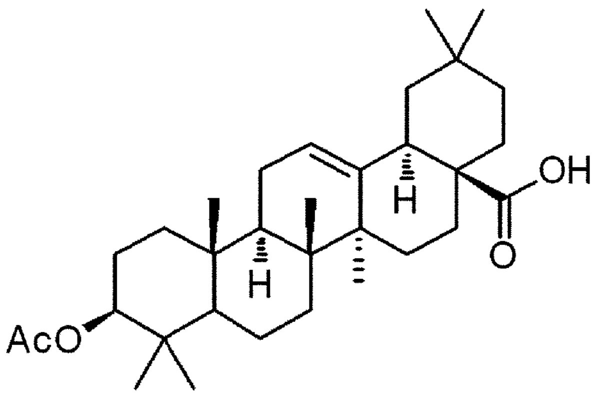

2. The compound for use according to claim 1, wherein the autoimmune diseases are selected from the group consisting of atopic dermatitis, arthritis, psoriasis, asthma, graft-versus-host disease, immune deficiency syndrome, systemic erythematous lupus, and multiple sclerosis.

3. The compound for use according to claim 1, wherein the cancer diseases are selected from the group consisting of myeloma, breast carcinoma, prostate cancer, brain tumors, head and neck carcinoma, melanoma, leukemia, and lymphoma.

4. A formulation for use in the prevention or treatment of TLR- and IL-6-mediated diseases selected from the group consisting of autoimmune diseases, cancer diseases, and metabolic diseases, comprising (i) the compound or a pharmaceutically acceptable salt thereof according to claim 1 as an active ingredient and (ii) a pharmaceutically

acceptable carrier, excipient, or diluent,
wherein the metabolic diseases are selected from the group consisting of osteoporosis, arteriosclerosis, and myocardial infarction.

**5.** A composition comprising an adzuki bean extract containing a compound represented by the following Chemical Formula 1 or a pharmaceutically acceptable salt thereof as an active ingredient, or a fraction thereof for use in the prevention, improvement or treatment of TLR- and IL-6-mediated diseases, selected from the group consisting of autoimmune diseases, cancer diseases and metabolic diseases,wherein the metabolic diseases are selected from the group consisting of osteoporosis, arteriosclerosis and myocardial infarction

[Chemical Formula 1]

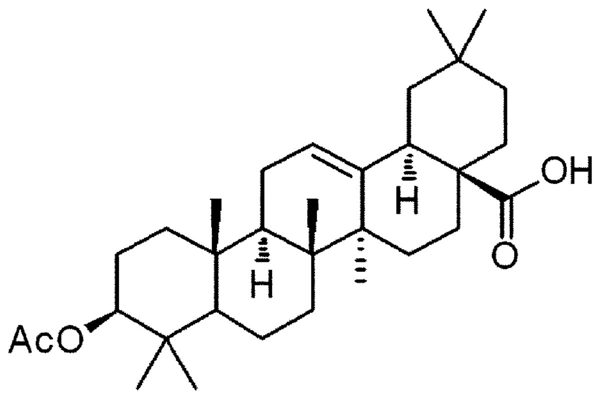

**6.** The composition comprising adzuki bean extract or a fraction thereof according to the use of claim 5, wherein the adzuki bean extract is obtained by extracting adzuki beans using a solvent selected from the group consisting of water, alcohol having 1 to 6 carbon atoms and a solvent mixture thereof.

**7.** The composition comprising adzuki bean extract or a fraction thereof according to the use of claim 5, wherein the fraction is obtained by fractionating the adzuki bean extract using a solvent selected from the group consisting of hexane, ethyl acetate, water and a solvent mixture thereof.

**8.** The composition comprising adzuki bean extract or a fraction thereof according to the use of claim 5, wherein the autoimmune diseases are selected from the group consisting of atopic dermatitis, arthritis, psoriasis, asthma, graft-versus-host disease, immune deficiency syndrome, systemic erythematous lupus, and multiple sclerosis.

**9.** The composition comprising adzuki bean extract or a fraction thereof according to the use of claim 5, wherein the cancer diseases are selected from the group consisting of myeloma, breast carcinoma, prostate cancer, brain tumors, head and neck carcinoma, melanoma, leukemia, and lymphoma.

**10.** A skin composition for external use for use in the prevention or improvement of TLR- and IL-6-mediated diseases selected from the group consisting of autoimmune diseases, cancer diseases, and metabolic diseases, comprising the composition comprising adzuki bean extract or a fraction thereof according to claim 5 as an active ingredient,
wherein the metabolic diseases are selected from the group consisting of osteoporosis, arteriosclerosis, and myocardial infarction.

**11.** A health functional food for use in the prevention or improvement of TLR- and IL-6-mediated diseases selected from the group consisting of autoimmune diseases, cancer diseases, and metabolic diseases, comprising the composition comprising adzuki bean extract or a fraction thereof according to claim 5 as an active ingredient,
wherein the metabolic diseases are selected from the group consisting of osteoporosis, arteriosclerosis, and myocardial infarction.

**12.** A personal hygiene product for use in the prevention or improvement of TLR- and IL-6-mediated diseases selected from the group consisting of autoimmune diseases, cancer diseases, and metabolic diseases, comprising the composition comprising adzuki bean extract, or a fraction thereof according to claim 5 as an active ingredient,
wherein the metabolic diseases are selected from the group consisting of osteoporosis, arteriosclerosis, and myocardial infarction.

**Patentansprüche**

1. Verbindung dargestellt durch Formel 1 oder ein pharmazeutisch annehmbares Salz davon zur Verwendung bei der Vorbeugung, Verbesserung oder Behandlung von TLR- und IL-6-vermittelten Erkrankungen, ausgewählt aus der Gruppe bestehend aus Autoimmunerkrankungen, Krebserkrankungen und Stoffwechselerkrankungen, wobei die Stoffwechselerkrankungen ausgewählt sind aus der Gruppe bestehend aus Osteoporose, Arteriosklerose und Myokardinfarkt

[Chemische Formel 1]

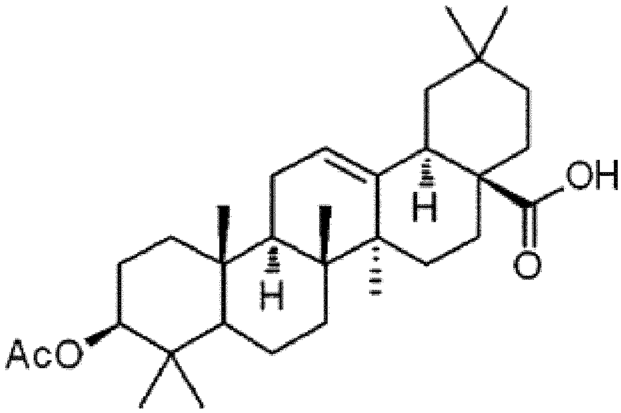

2. Verbindung zur Verwendung nach Anspruch 1, wobei die Autoimmunerkrankungen ausgewählt sind aus der Gruppe bestehend aus atopischer Dermatitis, Arthritis, Psoriasis, Asthma, Graft-versus-Host-Erkrankung, Immundefizienz-Syndrom, systemischem erythematösem Lupus und multipler Sklerose.

3. Verbindung zur Verwendung nach Anspruch 1, wobei die Krebserkrankungen ausgewählt sind aus der Gruppe bestehend aus Myelom, Brustkarzinom, Prostatakrebs, Gehirntumoren, Kopf- und Halskarzinom, Melanom, Leukämie und Lymphom.

4. Formulierung zur Verwendung bei der Vorbeugung oder Behandlung von TLR- und IL-6-vermittelten Erkrankungen, ausgewählt aus der Gruppe bestehend aus Autoimmunerkrankungen, Krebserkrankungen und Stoffwechselerkrankungen, umfassend (i) die Verbindung oder ein pharmazeutisch annehmbares Salz davon gemäß Anspruch 1 als einen Wirkstoff und (ii) einen pharmazeutisch annehmbaren Träger, Hilfsmittel oder Verdünnungsmittel, wobei die Stoffwechselerkrankungen ausgewählt sind aus der Gruppe bestehend aus Osteoporose, Arteriosklerose und Myokardinfarkt.

5. Zusammensetzung, umfassend einen Adzukibohnenextrakt, der eine durch die folgende chemische Formel 1 dargestellte Verbindung oder ein pharmazeutisch annehmbares Salz davon als einen Wirkstoff enthält, oder einen Teil davon zur Verwendung bei der Vorbeugung, Verbesserung oder Behandlung von TLR- und IL-6-vermittelten Erkrankungen, ausgewählt aus der Gruppe bestehend aus Autoimmunerkrankungen, Krebserkrankungen und Stoffwechselerkrankungen, wobei die Stoffwechselerkrankungen ausgewählt sind aus der Gruppe bestehend aus Osteoporose, Arteriosklerose und Myokardinfarkt

[Chemische Formel 1]

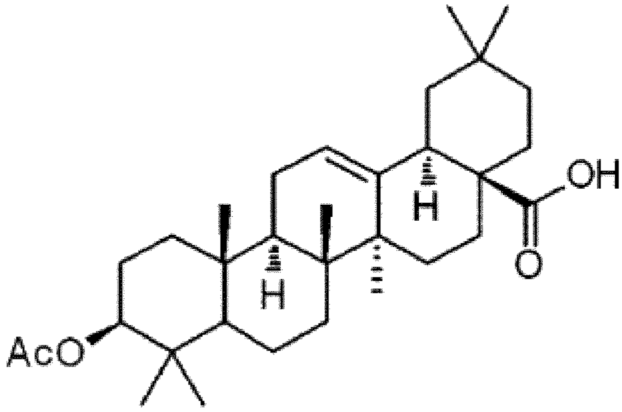

**6.** Zusammensetzung, umfassend Adzukibohnenextrakt oder einen Teil davon gemäß der Verwendung von Anspruch 5, wobei der Adzukibohnenextrakt durch Extraktion von Adzukibohnen unter Verwendung eines Lösungsmittels, ausgewählt aus der Gruppe bestehend aus Wasser, Alkohol mit 1 bis 6 Kohlenstoffatomen und einem Lösungsmittelgemisch davon, erhalten wird.

**7.** Zusammensetzung, umfassend Adzukibohnenextrakt oder einen Teil davon gemäß der Verwendung von Anspruch 5, wobei der Teil durch Fraktionieren des Adzukibohnenextrakts unter Verwendung eines Lösungsmittels, das ausgewählt ist aus der Gruppe bestehend aus Hexan, Ethylacetat, Wasser und einem Lösungsmittelgemisch davon, erhalten wird.

**8.** Zusammensetzung, umfassend Adzukibohnenextrakt oder einen Teil davon gemäß der Verwendung von Anspruch 5, wobei die Autoimmunerkrankungen ausgewählt sind aus der Gruppe bestehend aus atopischer Dermatitis, Arthritis, Psoriasis, Asthma, Graft-versus-Host-Erkrankung, Immundefizienz-Syndrom, systemischem erythematösem Lupus und multipler Sklerose.

**9.** Zusammensetzung, umfassend Adzukibohnenextrakt oder einen Teil davon gemäß der Verwendung von Anspruch 5, wobei die Krebserkrankungen ausgewählt sind aus der Gruppe bestehend aus Myelom, Brustkarzinom, Prostatakrebs, Gehirntumoren, Kopf-Hals-Karzinom, Melanom, Leukämie und Lymphom.

**10.** Zusammensetzung zur äußerlichen Anwendung auf der Haut zur Verwendung bei der Vorbeugung oder Verbesserung von TLR- und IL-6-vermittelten Erkrankungen, ausgewählt aus der Gruppe bestehend aus Autoimmunerkrankungen, Krebserkrankungen und Stoffwechselerkrankungen, umfassend die Zusammensetzung, die Adzukibohnenextrakt oder einen Teil davon gemäß Anspruch 5 als einen Wirkstoff umfasst, wobei die Stoffwechselerkrankungen ausgewählt sind aus der Gruppe bestehend aus Osteoporose, Arteriosklerose und Myokardinfarkt.

**11.** Funktionelles Gesundheitsnahrungsprodukt zur Verwendung bei der Vorbeugung oder Verbesserung von TLR- und IL-6-vermittelten Erkrankungen, ausgewählt aus der Gruppe bestehend aus Autoimmunerkrankungen, Krebserkrankungen und Stoffwechselerkrankungen, umfassend die Zusammensetzung, die Adzukibohnenextrakt oder einen Teil davon gemäß Anspruch 5 als einen Wirkstoff umfasst, wobei die Stoffwechselerkrankungen ausgewählt sind aus der Gruppe bestehend aus Osteoporose, Arteriosklerose und Myokardinfarkt.

**12.** Körperpflegeprodukt zur Verwendung bei der Vorbeugung oder Verbesserung von TLR- und IL-6-vermittelten Erkrankungen, ausgewählt aus der Gruppe bestehend aus Autoimmunerkrankungen, Krebserkrankungen und Stoffwechselerkrankungen, umfassend die Zusammensetzung, die Adzukibohnenextrakt oder einen Teil davon gemäß Anspruch 5 als einen Wirkstoff umfasst, wobei die Stoffwechselerkrankungen ausgewählt sind aus der Gruppe bestehend aus Osteoporose, Arteriosklerose und Myokardinfarkt.

## Revendications

**1.** Composé représenté par la formule 1 ou sel pharmaceutiquement acceptable de celui-ci pour une utilisation dans la prévention, l'amélioration ou le traitement de maladies médiées par TLR et IL-6 choisies dans le groupe consistant

en des maladies auto-immunes, des maladies cancéreuses et des maladies métaboliques, dans lequel les maladies métaboliques sont choisies dans le groupe consistant en l'ostéoporose, l'artériosclérose et l'infarctus du myocarde

[Formule chimique 1]

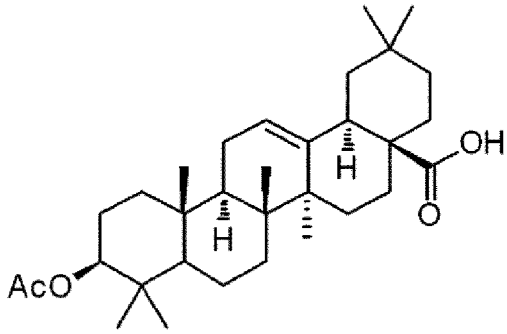

2. Composé pour une utilisation selon la revendication 1, dans lequel les maladies auto-immunes sont choisies dans le groupe consistant en la dermatite atopique, l'arthrite, le psoriasis, l'asthme, la maladie du greffon contre l'hôte, un syndrome d'immunodéficience, le lupus érythémateux disséminé, et la sclérose en plaques.

3. Composé pour une utilisation selon la revendication 1, dans lequel les maladies cancéreuses sont choisies dans le groupe consistant en le myélome, le carcinome du sein, le cancer de la prostate, les tumeurs cérébrales, le carcinome de la tête et du cou, le mélanome, la leucémie, et le lymphome.

4. Formulation pour une utilisation dans la prévention ou le traitement de maladies médiées par TLR et IL-6 choisies dans le groupe consistant en des maladies auto-immunes, des maladies cancéreuses, et des maladies métaboliques, comprenant (i) le composé ou sel pharmaceutiquement acceptable de celui-ci selon la revendication 1 en tant que principe actif et (ii) un support, excipient, ou diluant pharmaceutiquement acceptable,
dans laquelle les maladies métaboliques sont choisies dans le groupe consistant en l'ostéoporose, l'artériosclérose, et l'infarctus du myocarde.

5. Composition comprenant un extrait de haricot azuki contenant un composé représenté par la formule chimique 1 suivante ou un sel pharmaceutiquement acceptable de celui-ci en tant que principe actif, ou une fraction de celui-ci pour une utilisation dans la prévention, l'amélioration ou le traitement de maladies médiées par TLR et IL-6 choisies dans le groupe consistant en des maladies auto-immunes, des maladies cancéreuses et des maladies métaboliques, dans laquelle les maladies métaboliques sont choisies dans le groupe consistant en l'ostéoporose, l'artériosclérose et l'infarctus du myocarde

[Formule chimique 1]

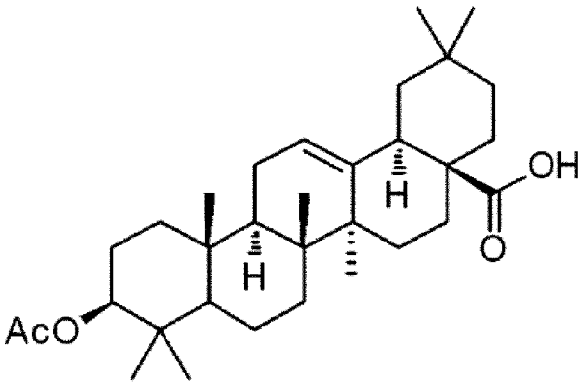

6. Composition comprenant de l'extrait de haricot azuki ou une fraction de celui-ci selon l'utilisation de la revendication 5, dans laquelle l'extrait de haricot azuki est obtenu par extraction de haricots azuki à l'aide d'un solvant choisi dans le groupe consistant en l'eau, un alcool comportant 1 à 6 atomes de carbone et un mélange de solvants de ceux-ci.

**7.** Composition comprenant de l'extrait de haricot azuki ou une fraction de celui-ci selon l'utilisation de la revendication 5, dans laquelle la fraction est obtenue par fractionnement de l'extrait de haricot azuki à l'aide d'un solvant choisi dans le groupe consistant en l'hexane, l'acétate d'éthyle, l'eau et un mélange de solvants de ceux-ci.

**8.** Composition comprenant de l'extrait de haricot azuki ou une fraction de celui-ci selon l'utilisation de la revendication 5, dans laquelle les maladies auto-immunes sont choisies dans le groupe consistant en le dermatite atopique, l'arthrite, le psoriasis, l'asthme, la maladie du greffon contre l'hôte, un syndrome d'immunodéficience, le lupus érythémateux disséminé, et la sclérose en plaques.

**9.** Composition comprenant de l'extrait de haricot azuki ou une fraction de celui-ci selon l'utilisation de la revendication 5, dans laquelle les maladies cancéreuses sont choisies dans le groupe consistant en le myélome, le carcinome du sein, le cancer de la prostate, les tumeurs cérébrales, le carcinome de la tête et du cou, le mélanome, la leucémie, et le lymphome.

**10.** Composition pour la peau destinée à une utilisation externe pour une utilisation dans la prévention ou l'amélioration de maladies médiées par TLR et IL-6 choisies dans le groupe consistant en des maladies auto-immunes, des maladies cancéreuses, et des maladies métaboliques, comprenant la composition comprenant de l'extrait de haricot azuki ou une fraction de celui-ci selon la revendication 5 en tant que principe actif,
dans laquelle les maladies métaboliques sont choisies dans le groupe consistant en l'ostéoporose, l'artériosclérose, et l'infarctus du myocarde.

**11.** Aliment fonctionnel pour la santé destiné à une utilisation dans la prévention ou l'amélioration de maladies médiées par TLR et IL-6 choisies dans le groupe consistant en des maladies auto-immunes, des maladies cancéreuses, et des maladies métaboliques, comprenant la composition comprenant de l'extrait de haricot azuki ou une fraction de celui-ci selon la revendication 5 en tant que principe actif,
dans lequel les maladies métaboliques sont choisies dans le groupe consistant en l'ostéoporose, l'artériosclérose, et l'infarctus du myocarde.

**12.** Produit d'hygiène personnelle destiné à une utilisation dans la prévention ou l'amélioration de maladies médiées par TLR et IL-6 choisies dans le groupe consistant en des maladies auto-immunes, des maladies cancéreuses, et des maladies métaboliques, comprenant la composition comprenant de l'extrait de haricot azuki ou une fraction de celui-ci selon la revendication 5 en tant que principe actif,
dans lequel les maladies métaboliques sont choisies dans le groupe consistant en l'ostéoporose, l'artériosclérose, et l'infarctus du myocarde.

[FIG. 1a]

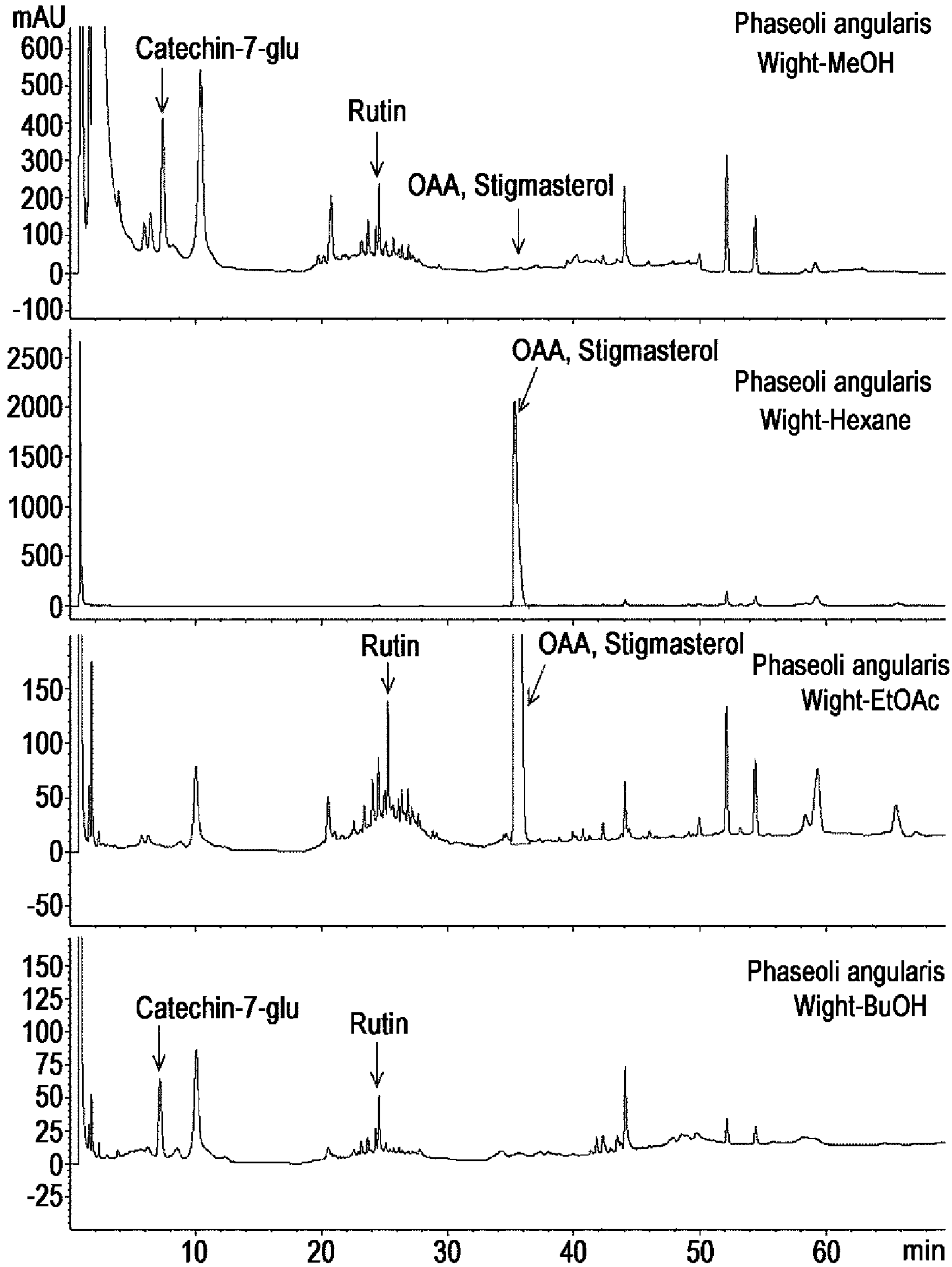
mAU
Catechin-7-glu
Rutin
OAA, Stigmasterol
Phaseoli angularis Wight-MeOH
Phaseoli angularis Wight-Hexane
Phaseoli angularis Wight-EtOAc
Phaseoli angularis Wight-BuOH
min

[FIG. 1b]

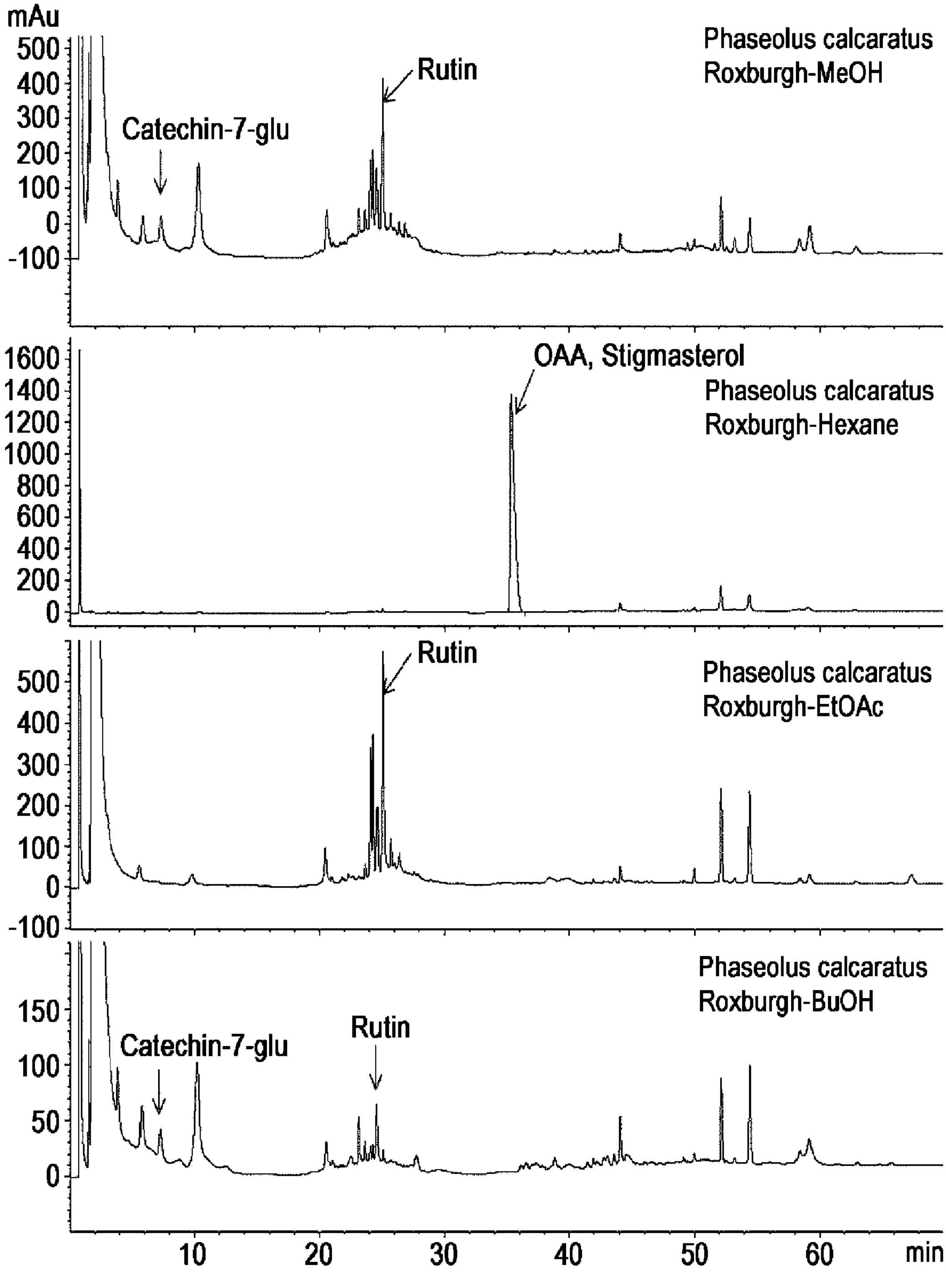
mAu
Phaseolus calcaratus Roxburgh-MeOH
Rutin
Catechin-7-glu
500
400
300
200
100
0
-100
OAA, Stigmasterol
Phaseolus calcaratus Roxburgh-Hexane
1600
1400
1200
1000
800
600
400
200
0
Rutin
Phaseolus calcaratus Roxburgh-EtOAc
500
400
300
200
100
0
-100
Phaseolus calcaratus Roxburgh-BuOH
Catechin-7-glu
Rutin
150
100
50
0
10
20
30
40
50
60
min

[FIG. 2a]

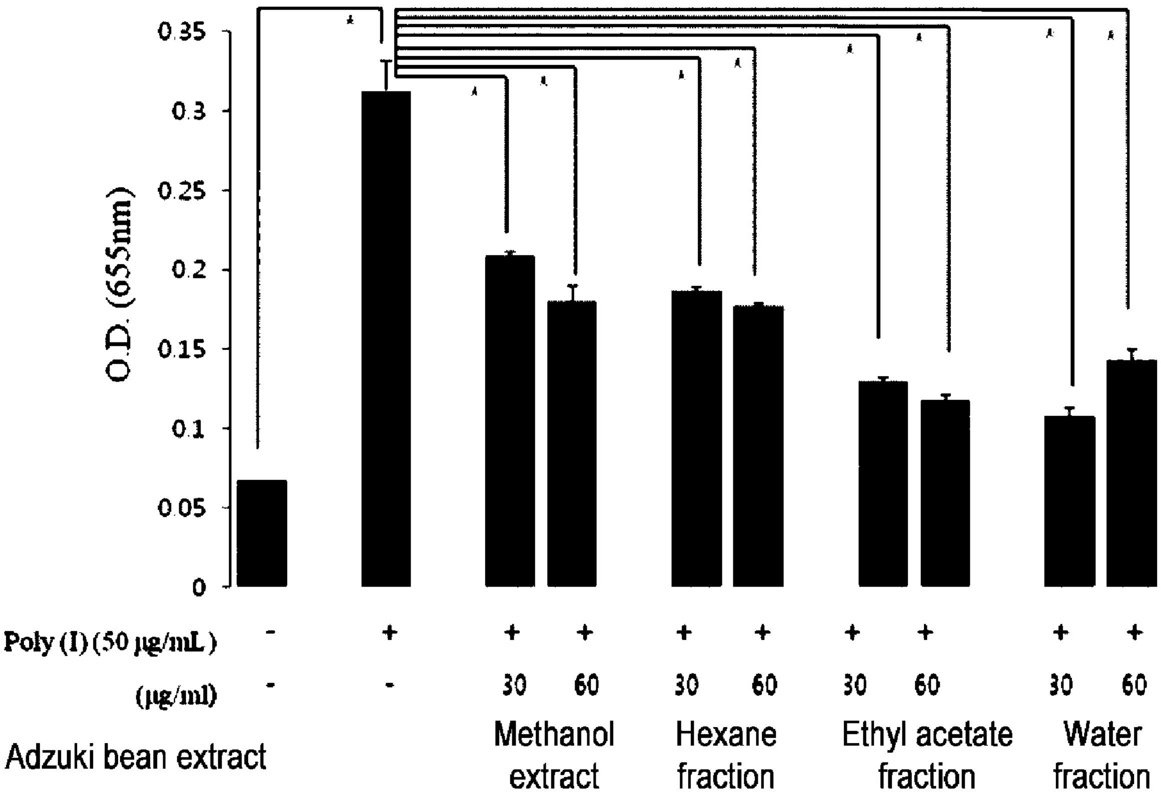


[FIG. 2b]

| Samples | Dose | Inhibition(%) | Viability(%) |
|---|---|---|---|
| Adzuki bean MeOH ext. | 30 µg/ml | 42.2 | 87.8 |
| | 60 µg/ml | 54.0 | 93.3 |
| Adzuki bean Hexane fr. | 30 µg/ml | 51.3 | 108.7 |
| | 60 µg/ml | 55.2 | 97.5 |
| Adzuki bean Ethyl acetate fr. | 30 µg/ml | 74.3 | 98.8 |
| | 60 µg/ml | 79.0 | 101.5 |
| Adzuki bean $H_2O$ fr. | 30 µg/ml | 83.5 | 98.9 |
| | 60 µg/ml | 69.0 | 103.9 |

[FIG. 3]

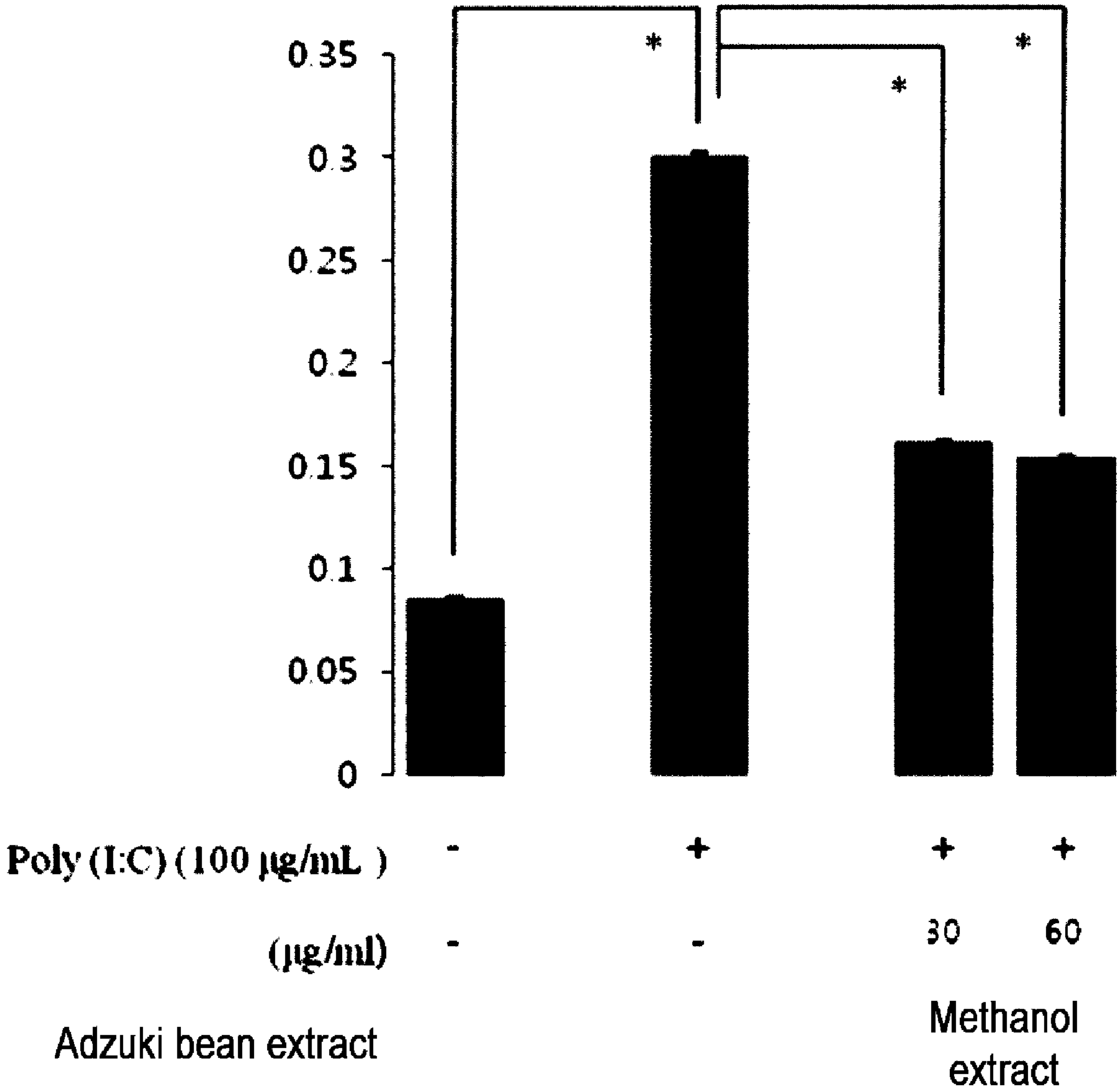


| Samples | | Inhibition(%) | Viability(%) |
|---|---|---|---|
| Adzuki bean extract | 30 µg/ml | 64.4 | 87.8 |
| | 60 µg/ml | 68.1 | 93.3 |

[FIG. 4a]

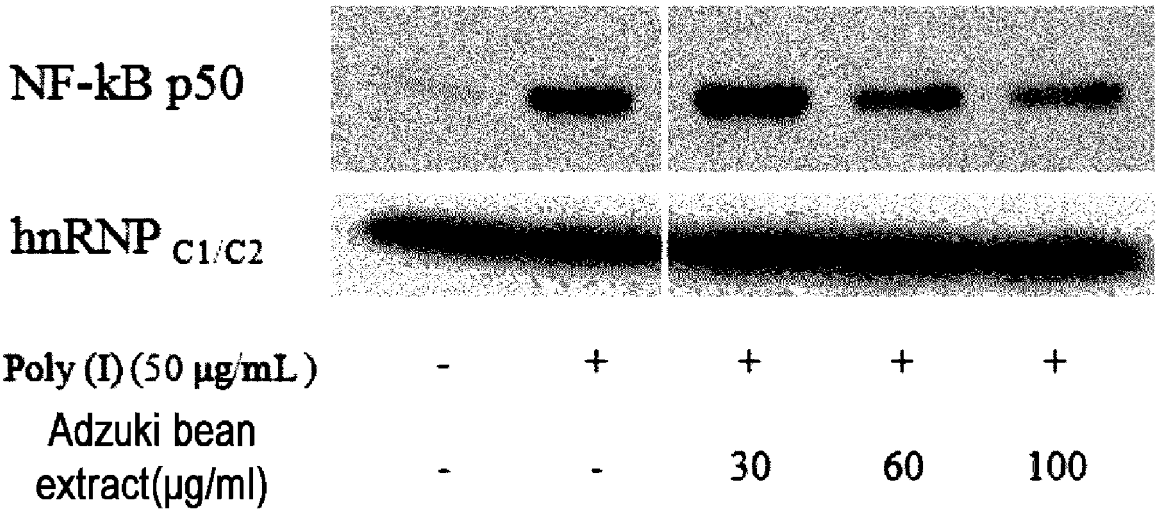
NF-kB p50
hnRNP C1/C2
Poly (I) (50 μg/mL)
- + + + +
Adzuki bean extract(μg/ml)
- - 30 60 100

[FIG. 4b]

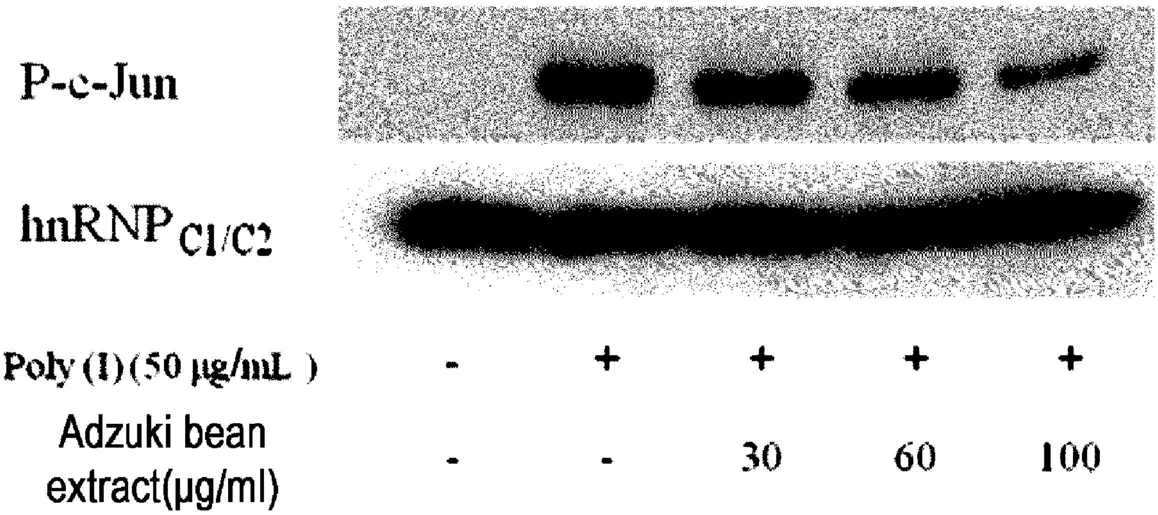
P-c-Jun
hnRNP C1/C2
Poly (I) (50 μg/mL)
- + + + +
Adzuki bean extract(μg/ml)
- - 30 60 100

[FIG. 4c]

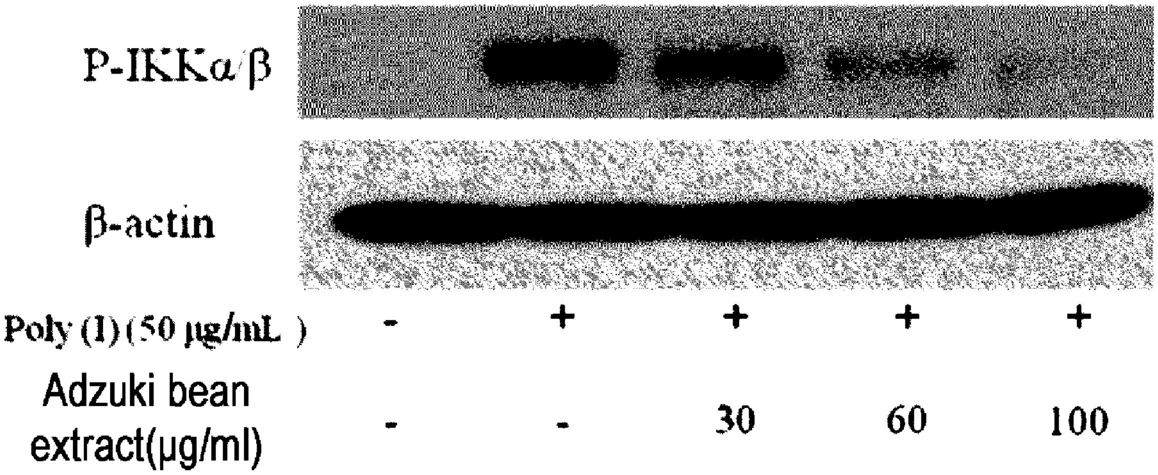
P-IKKα/β
β-actin
Poly (I) (50 μg/mL )
-
+
+
+
+
Adzuki bean extract(μg/ml)
-
-
30
60
100

[FIG. 4d]

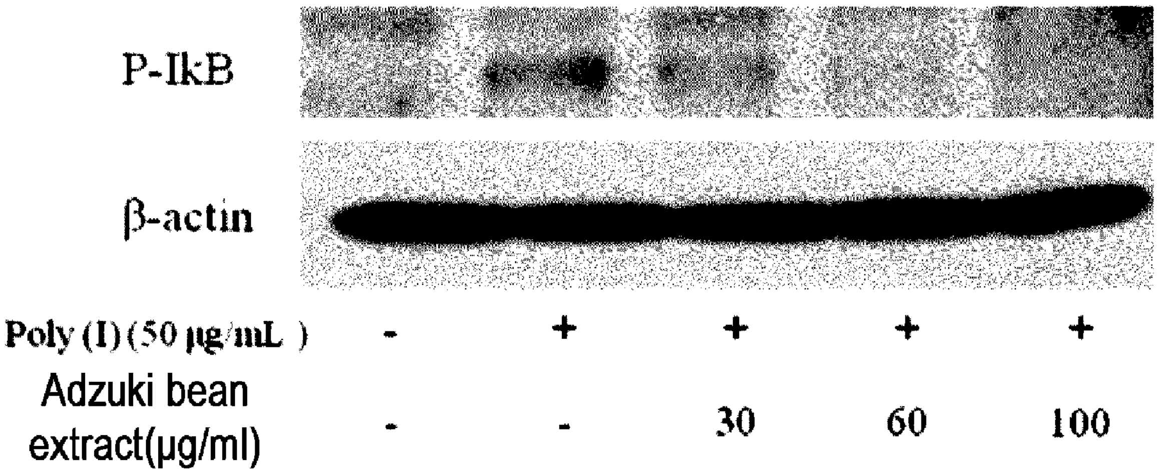
P-IkB
β-actin
Poly (I) (50 μg/mL )
-
+
+
+
+
Adzuki bean extract(μg/ml)
-
-
30
60
100

[FIG. 4e]

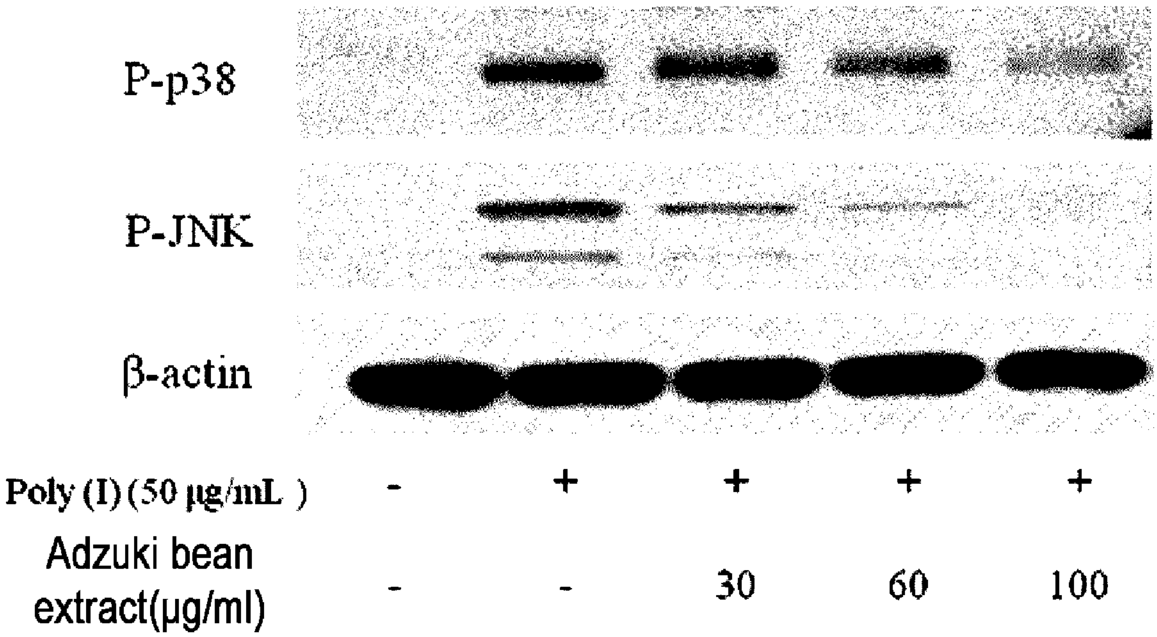
P-p38
P-JNK
β-actin
Poly (I) (50 μg/mL )
- + + + +
Adzuki bean extract(μg/ml)
- - 30 60 100

[FIG. 5a]

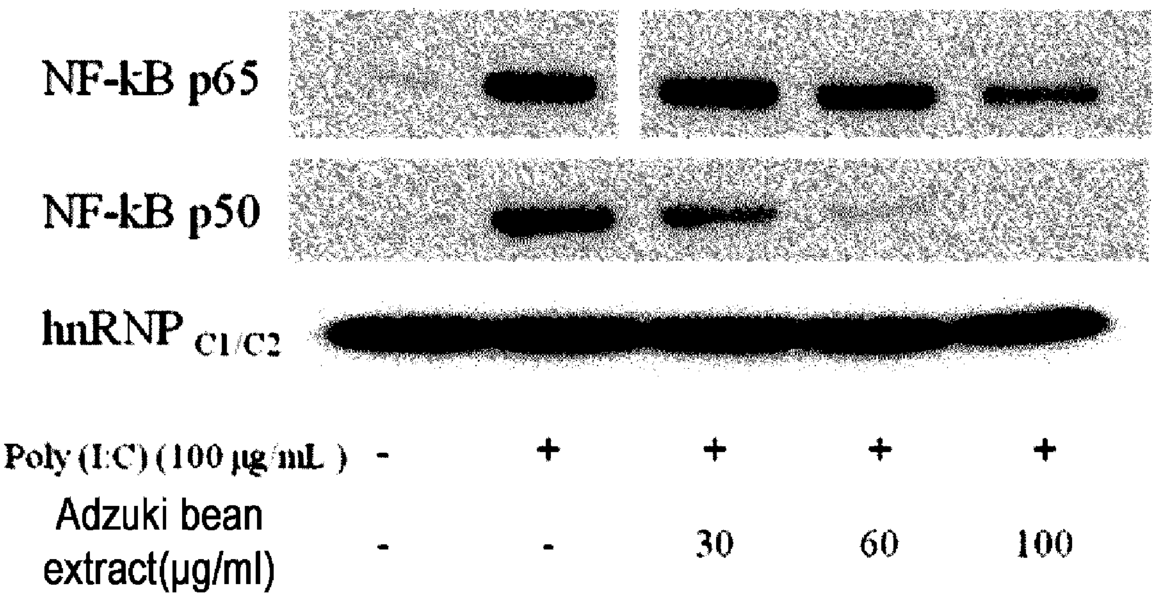
NF-kB p65
NF-kB p50
hnRNP C1/C2
Poly (I:C) (100 μg/mL )
- + + + +
Adzuki bean extract(μg/ml)
- - 30 60 100

[FIG. 5b]

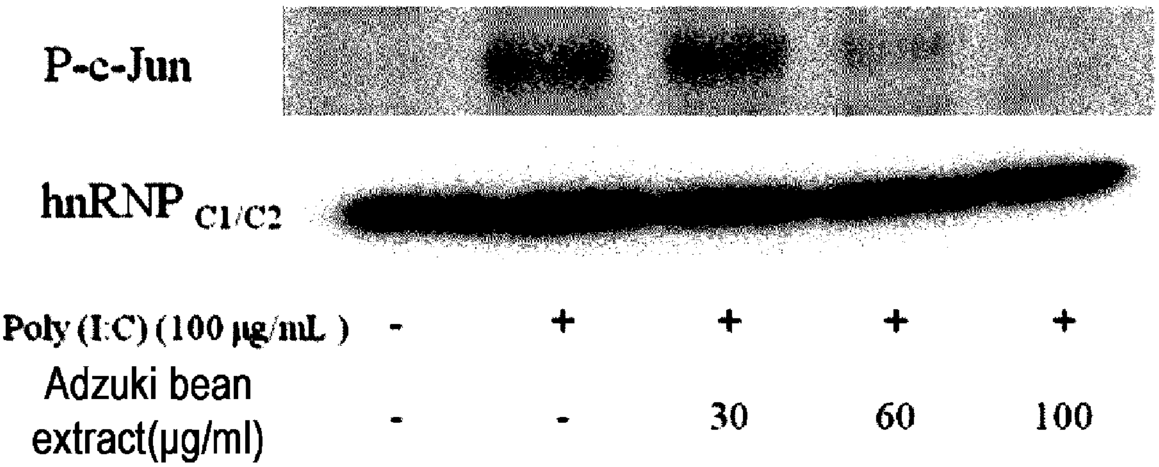
P-c-Jun
hnRNP C1/C2
Poly (I:C) (100 μg/mL ) - + + + +
Adzuki bean extract(μg/ml) - - 30 60 100

[FIG. 5c]

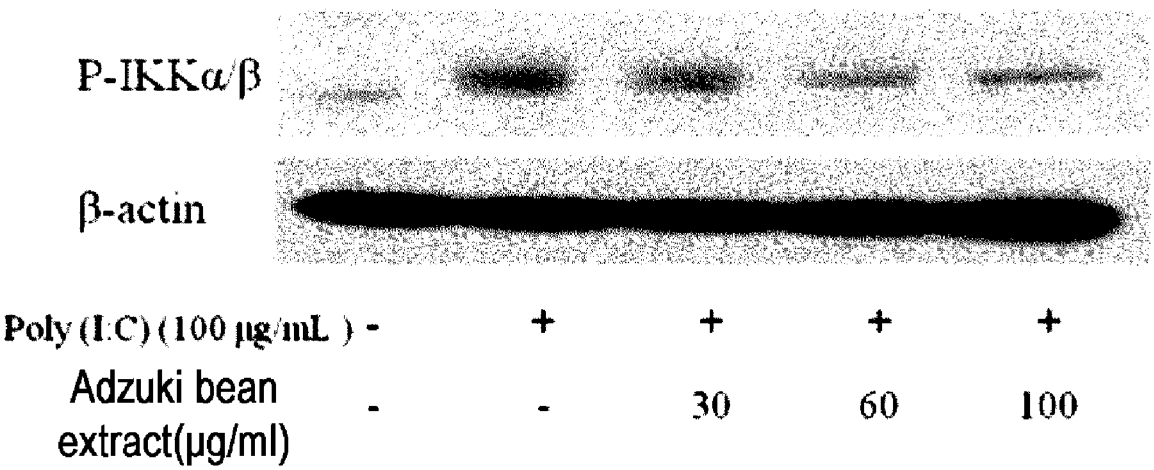
P-IKKα/β
β-actin
Poly (I:C) (100 μg/mL ) - + + + +
Adzuki bean extract(μg/ml) - - 30 60 100

[FIG. 5d]

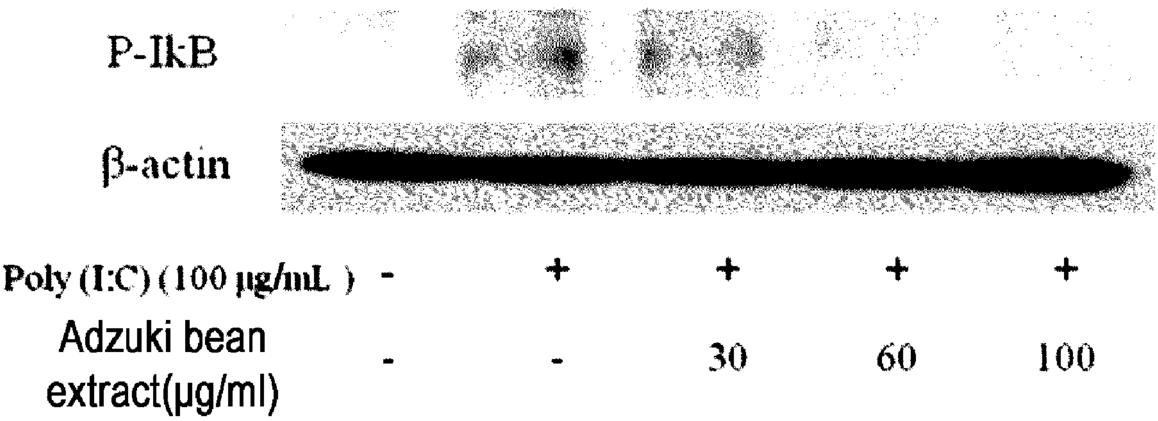
P-IkB
β-actin
Poly (I:C) (100 μg/mL )
-
+
+
+
+
Adzuki bean extract(μg/ml)
-
-
30
60
100

[FIG. 5e]

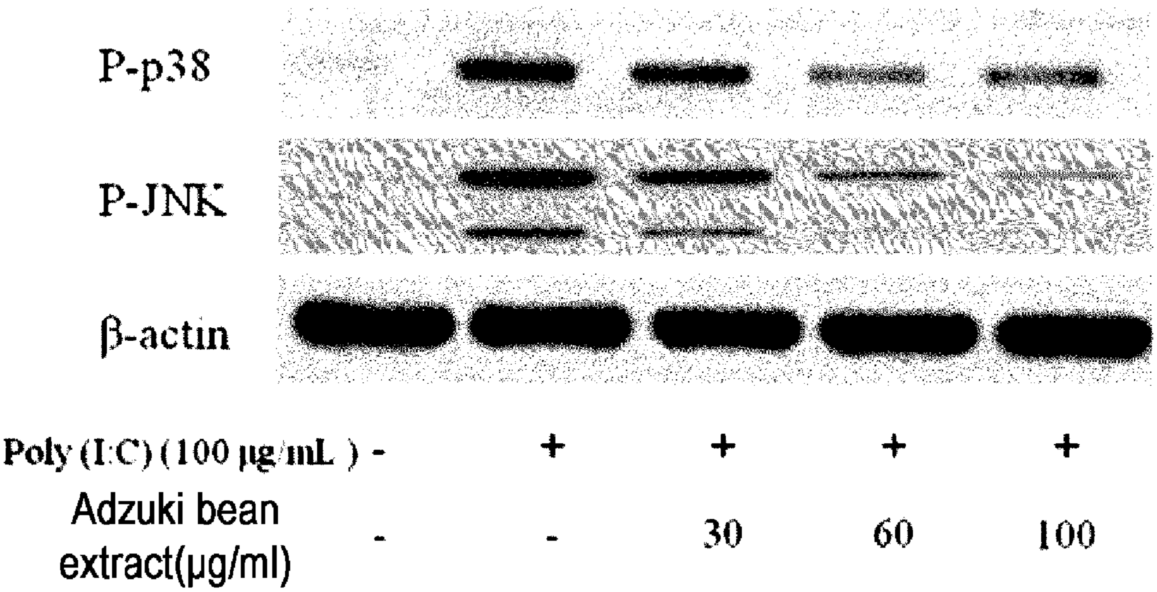
P-p38
P-JNK
β-actin
Poly (I:C) (100 μg/mL )
-
+
+
+
+
Adzuki bean extract(μg/ml)
-
-
30
60
100

[FIG. 5f]

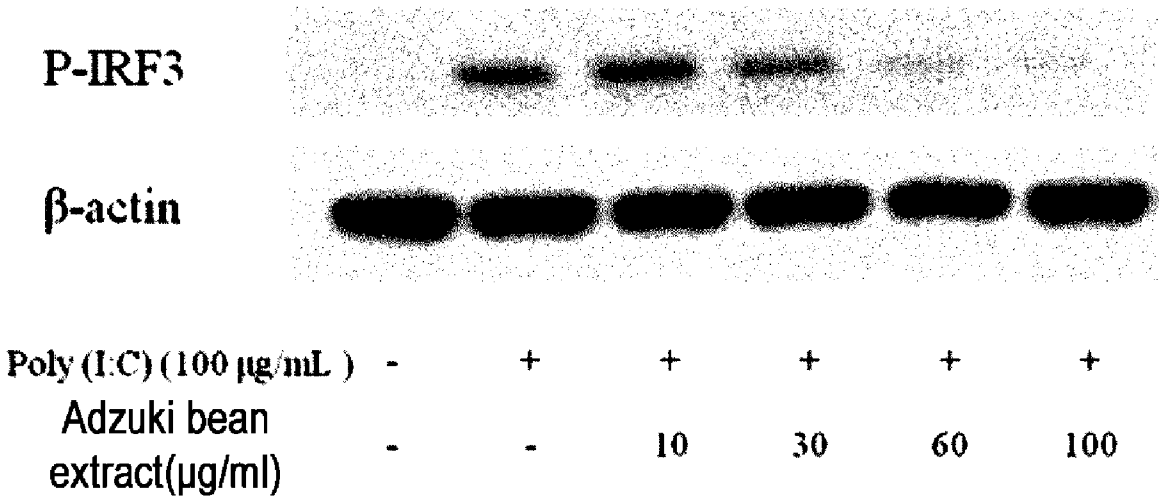
P-IRF3
β-actin
Poly (I:C) (100 μg/mL) - + + + + +
Adzuki bean extract(μg/ml) - - 10 30 60 100

[FIG. 6a]

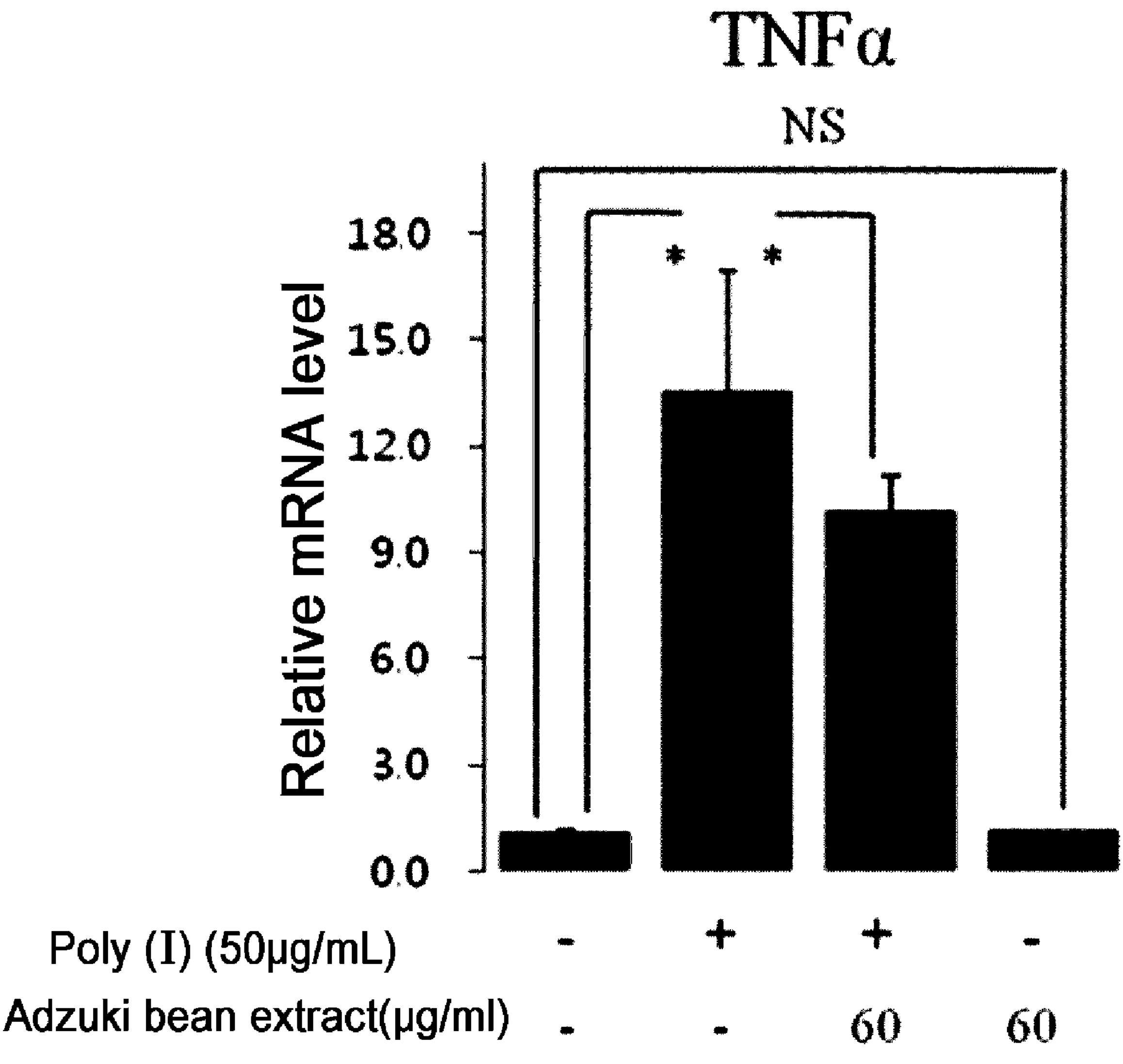
TNFα
NS
Relative mRNA level
18.0
15.0
12.0
9.0
6.0
3.0
0.0
*
*
Poly (I) (50μg/mL) - + + -
Adzuki bean extract(μg/ml) - - 60 60

[FIG. 6b]

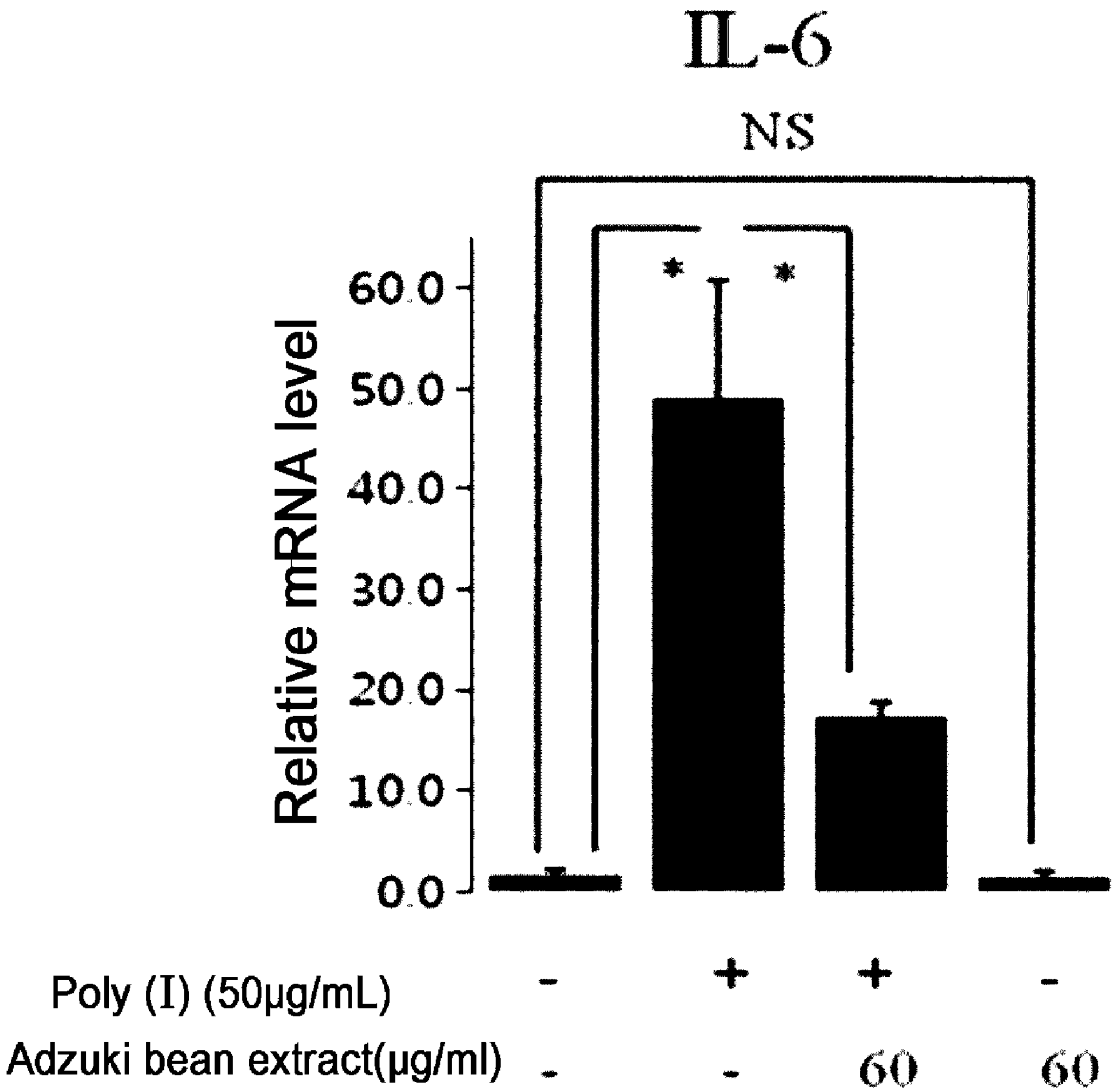
IL-6
NS
*
*
Relative mRNA level
60.0
50.0
40.0
30.0
20.0
10.0
0.0
Poly (I) (50μg/mL)
-
+
+
-
Adzuki bean extract(μg/ml)
-
-
60
60

[FIG. 6c]

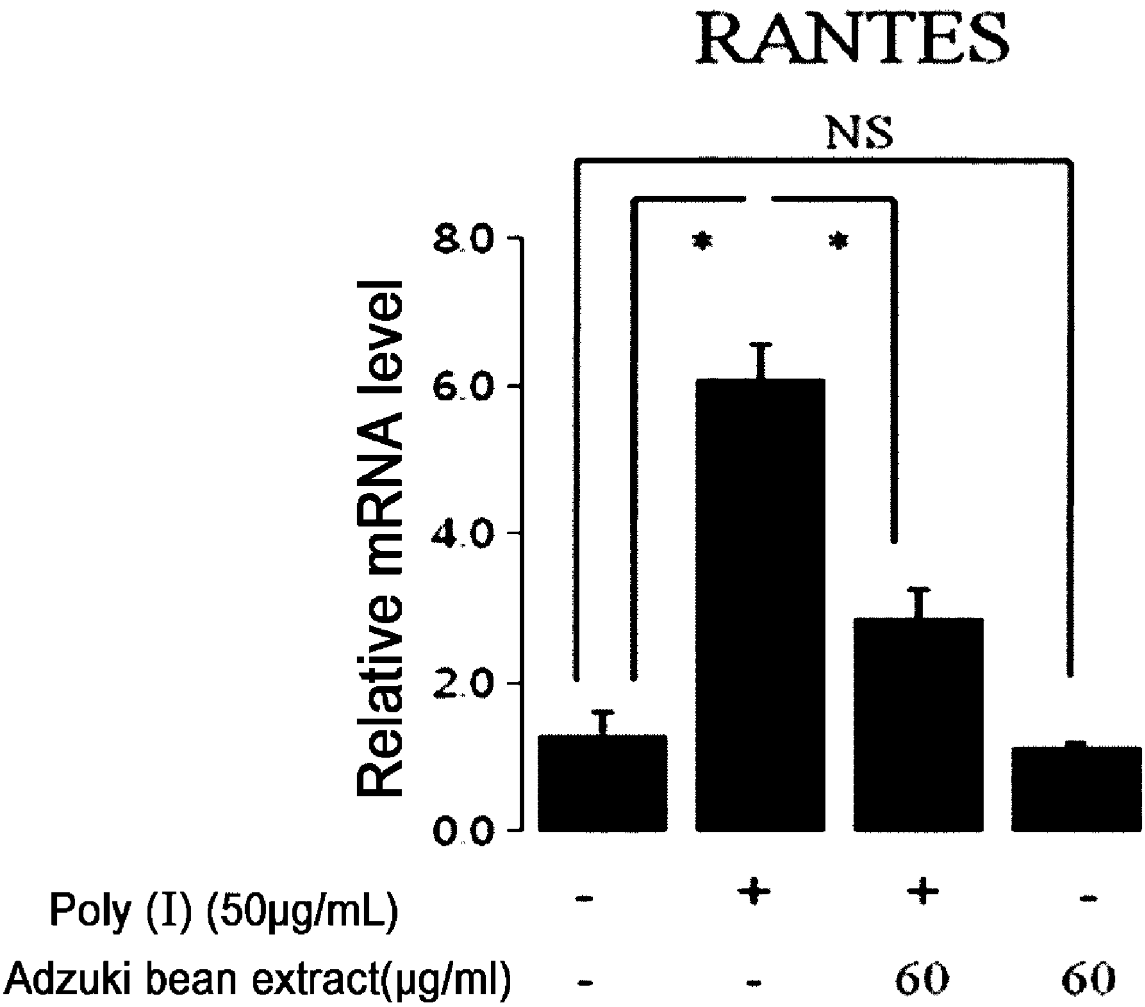
RANTES
NS
*
*
Relative mRNA level
8.0
6.0
4.0
2.0
0.0
Poly (I) (50μg/mL)
-
+
+
-
Adzuki bean extract(μg/ml)
-
-
60
60

[FIG. 6d]

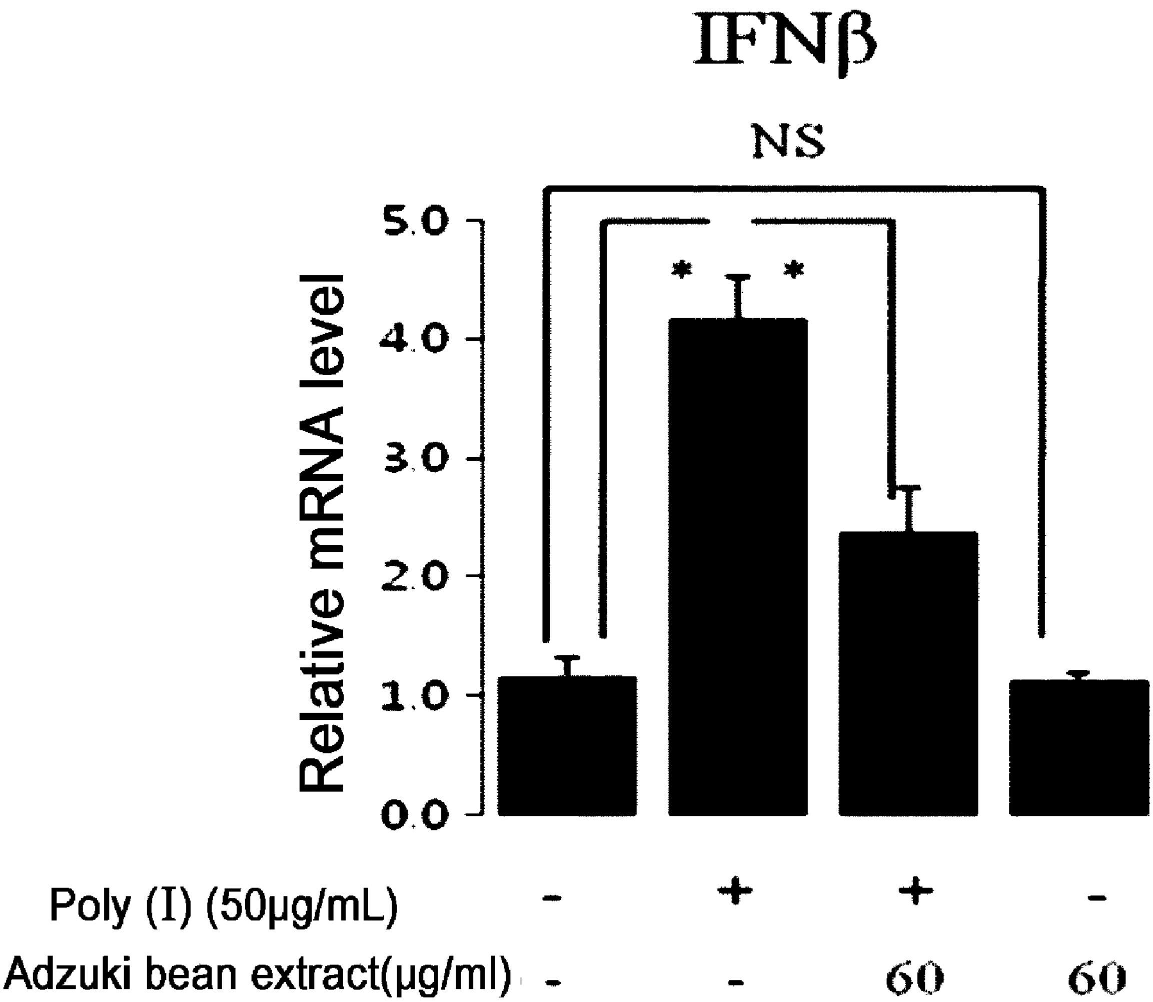
IFNβ
NS
Relative mRNA level
5.0
4.0
3.0
2.0
1.0
0.0
*
*
Poly (I) (50μg/mL) - + + -
Adzuki bean extract(μg/ml) - - 60 60

[FIG. 6e]

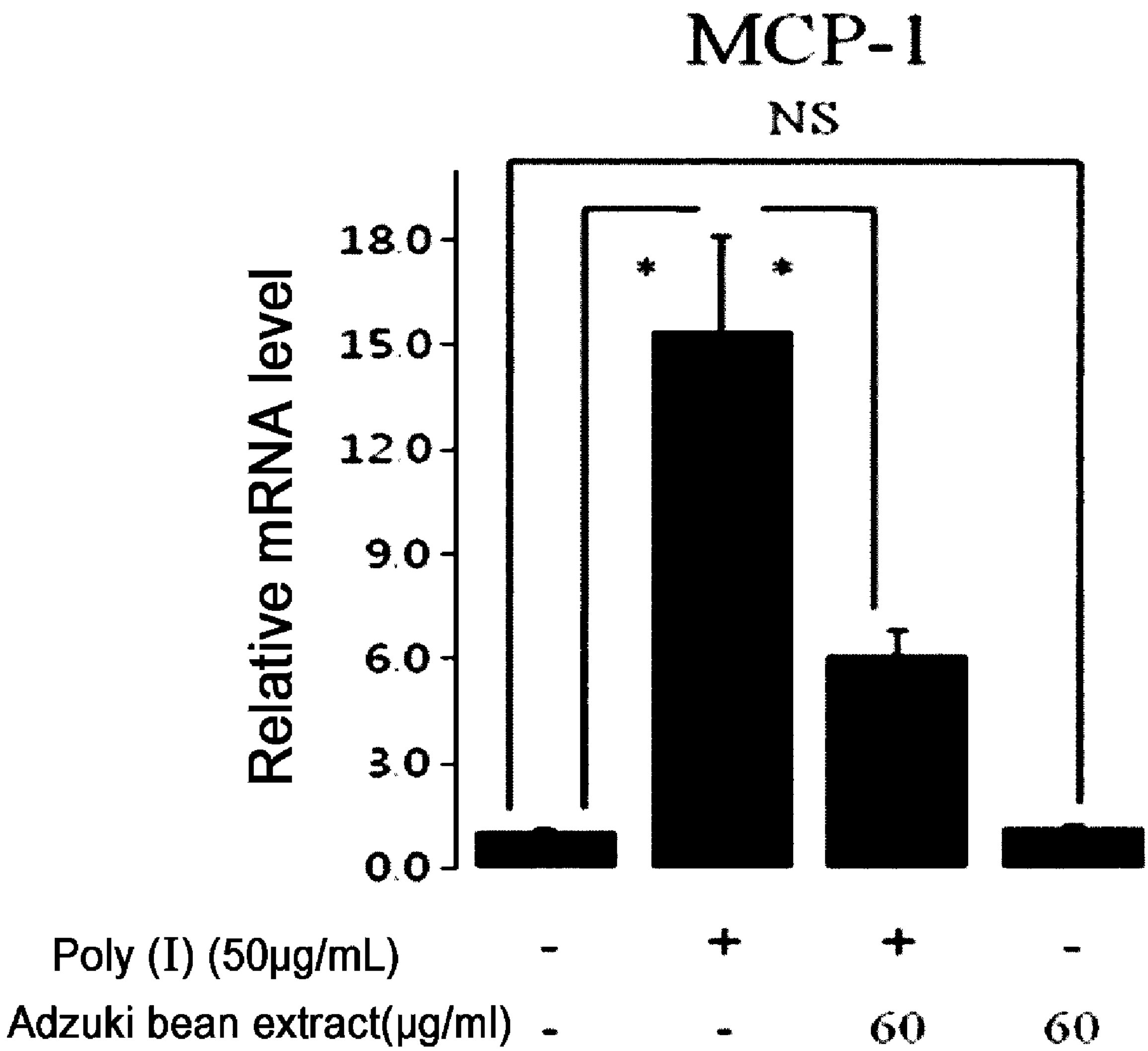
MCP-1
NS
*
*
Relative mRNA level
18.0
15.0
12.0
9.0
6.0
3.0
0.0
Poly (I) (50μg/mL)
-
+
+
-
Adzuki bean extract(μg/ml)
-
-
60
60

[FIG. 7a]

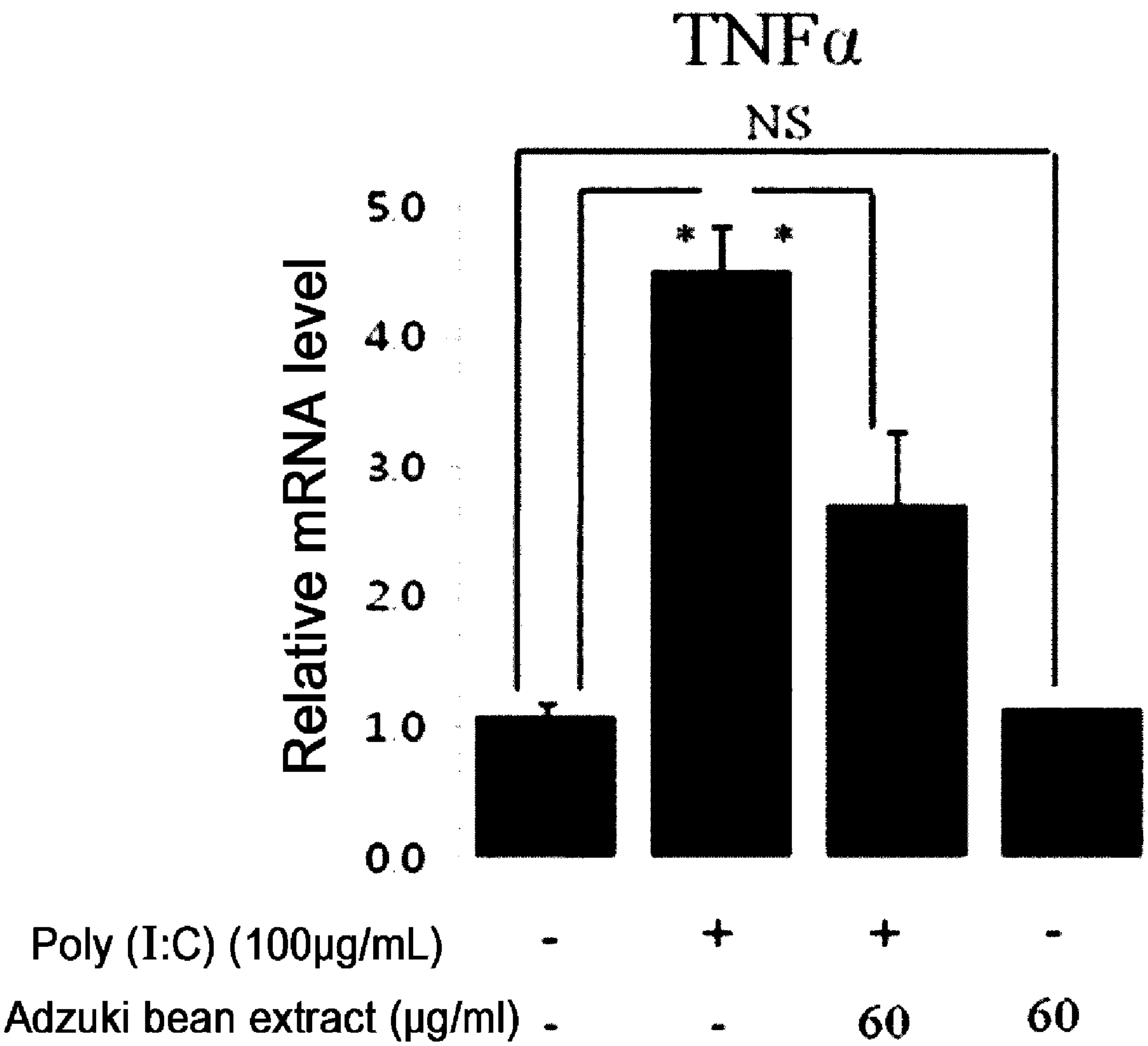
TNFα
NS
*
*
Relative mRNA level
5.0
4.0
3.0
2.0
1.0
0.0
Poly (I:C) (100μg/mL) - + + -
Adzuki bean extract (μg/ml) - - 60 60

[FIG. 7b]

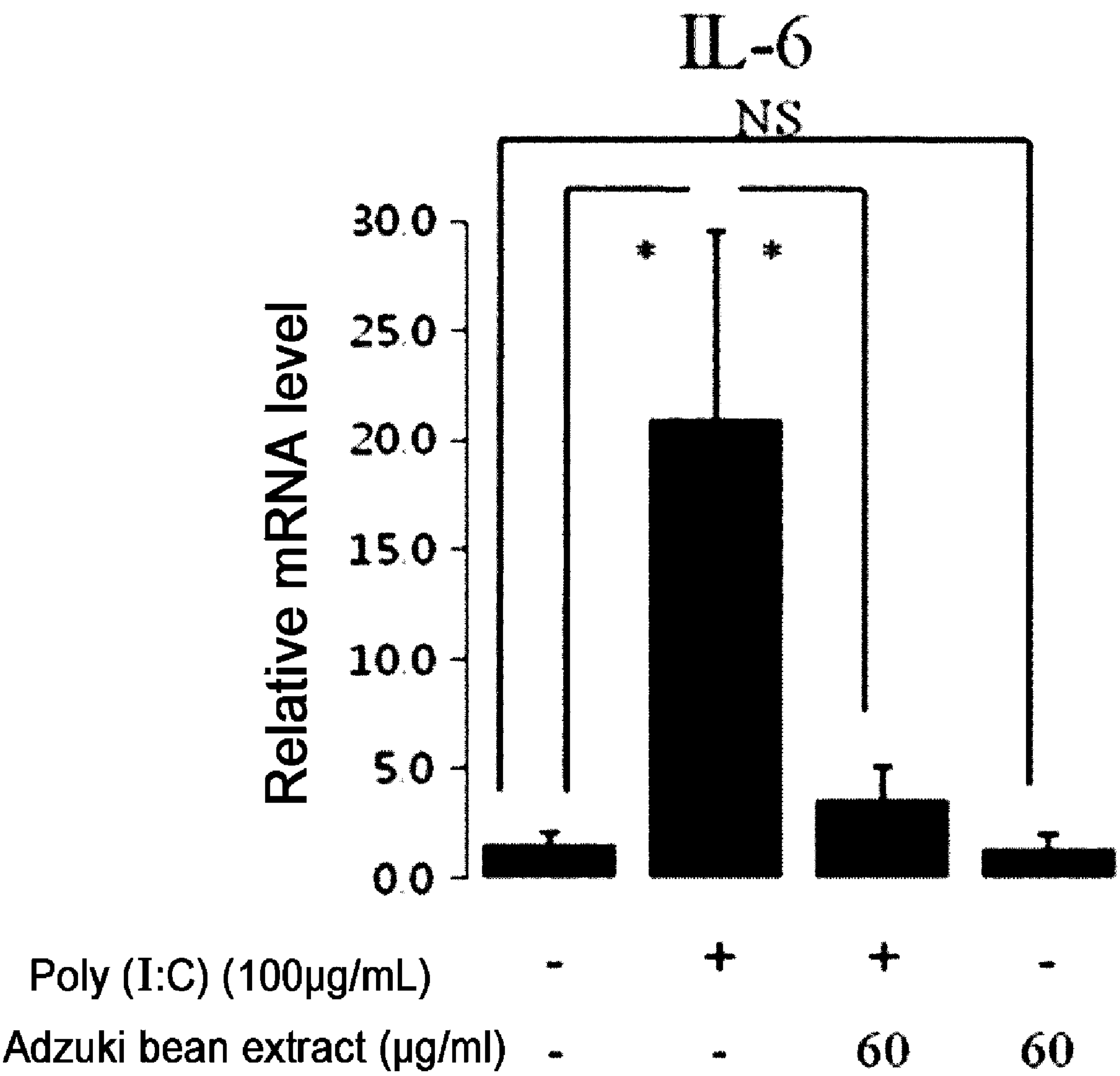
IL-6
NS
*
*
Relative mRNA level
30.0
25.0
20.0
15.0
10.0
5.0
0.0
Poly (I:C) (100μg/mL)
-
+
+
-
Adzuki bean extract (μg/ml)
-
-
60
60

[FIG. 7c]

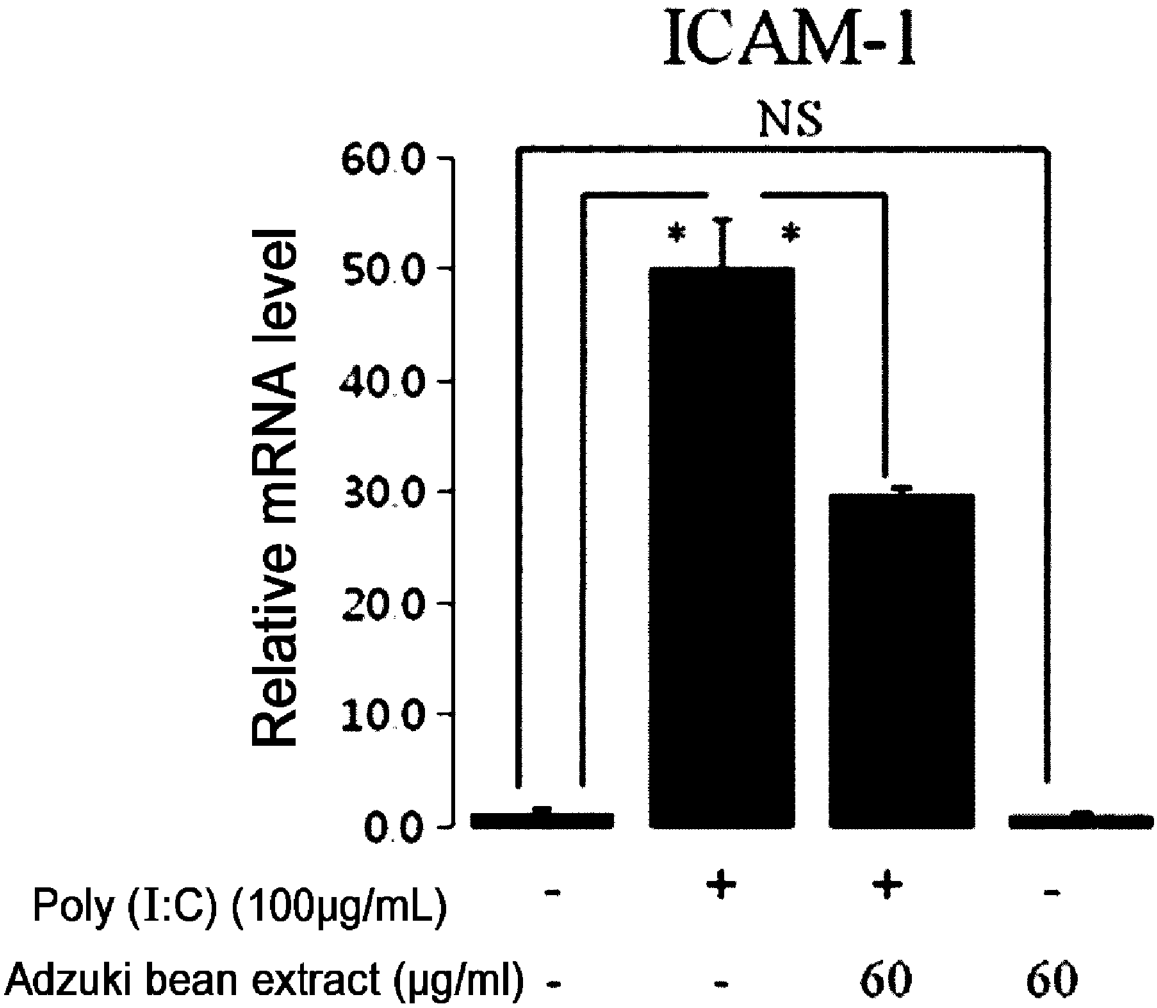
ICAM-1
NS
Relative mRNA level
60.0
50.0
40.0
30.0
20.0
10.0
0.0
*
*
Poly (I:C) (100μg/mL) - + + -
Adzuki bean extract (μg/ml) - - 60 60

[FIG. 7d]

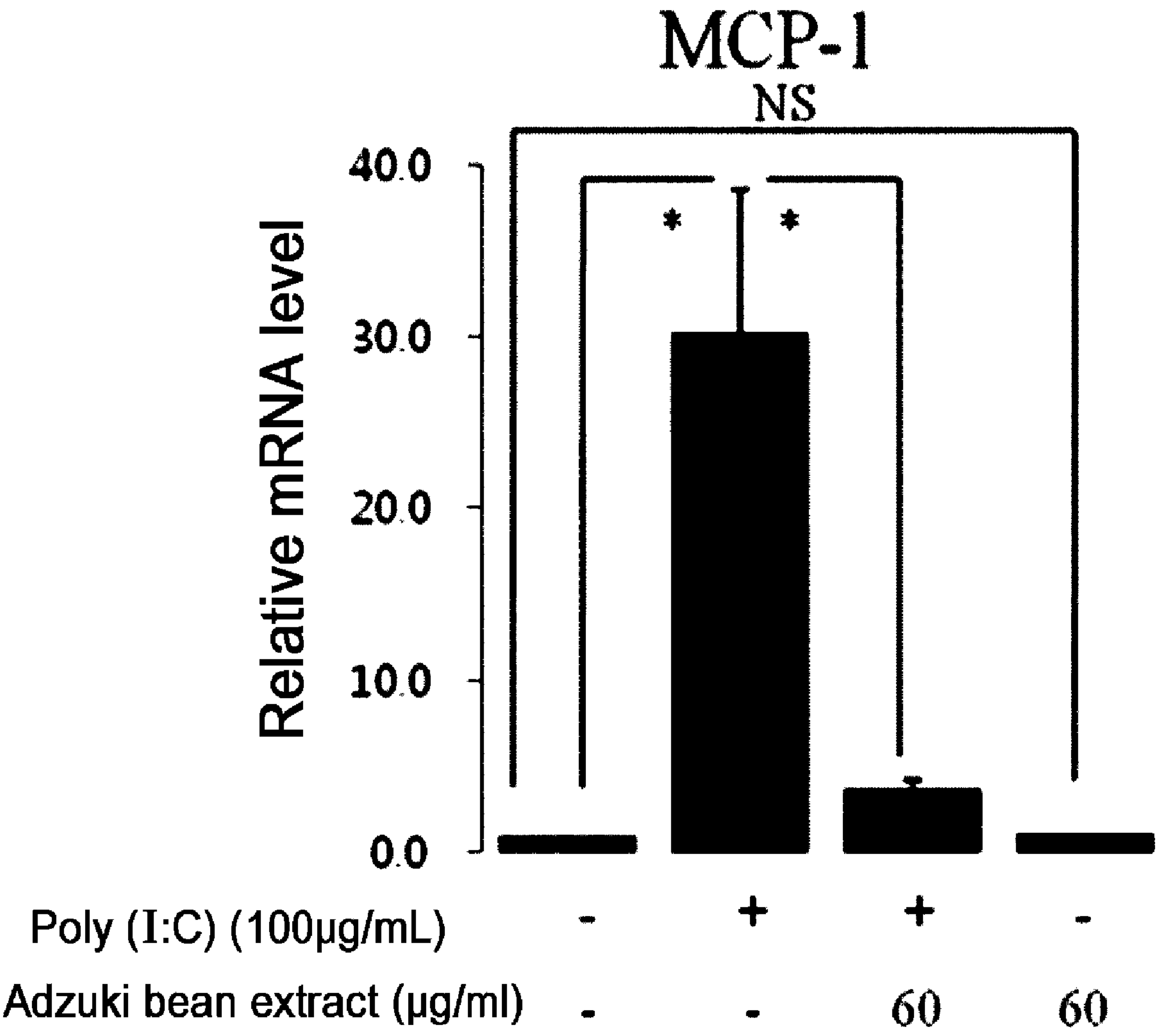
MCP-1
NS
*
*
Relative mRNA level
40.0
30.0
20.0
10.0
0.0
Poly (I:C) (100μg/mL) - + + -
Adzuki bean extract (μg/ml) - - 60 60

[FIG. 8a]

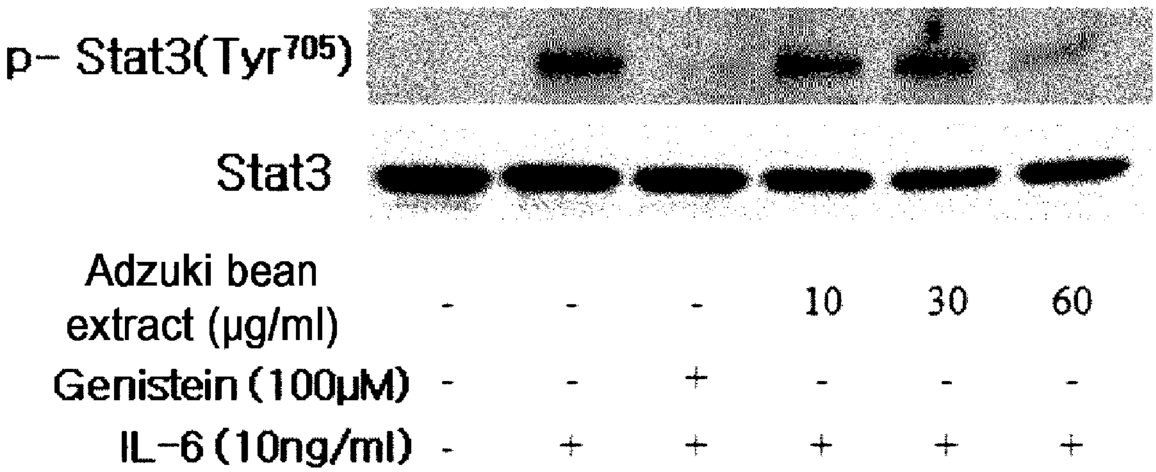
p- Stat3(Tyr705)
Stat3
Adzuki bean extract (μg/ml) - - - 10 30 60
Genistein (100μM) - - + - - -
IL-6 (10ng/ml) - + + + + +

[FIG. 8b]

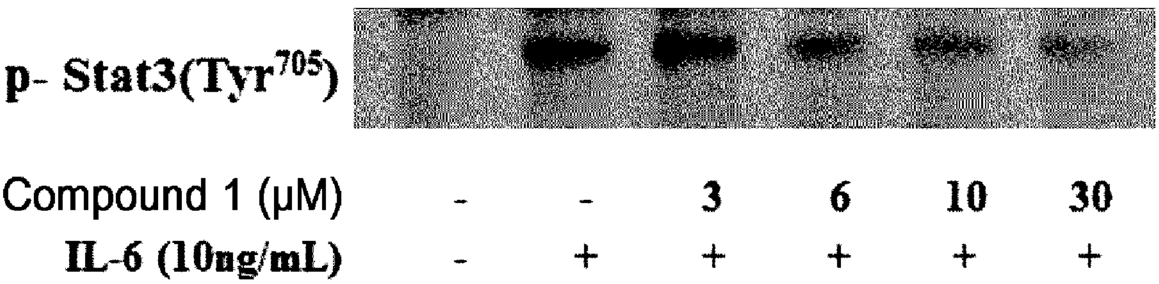
p- Stat3(Tyr705)
Compound 1 (μM) - - 3 6 10 30
IL-6 (10ng/mL) - + + + + +

[FIG. 9]

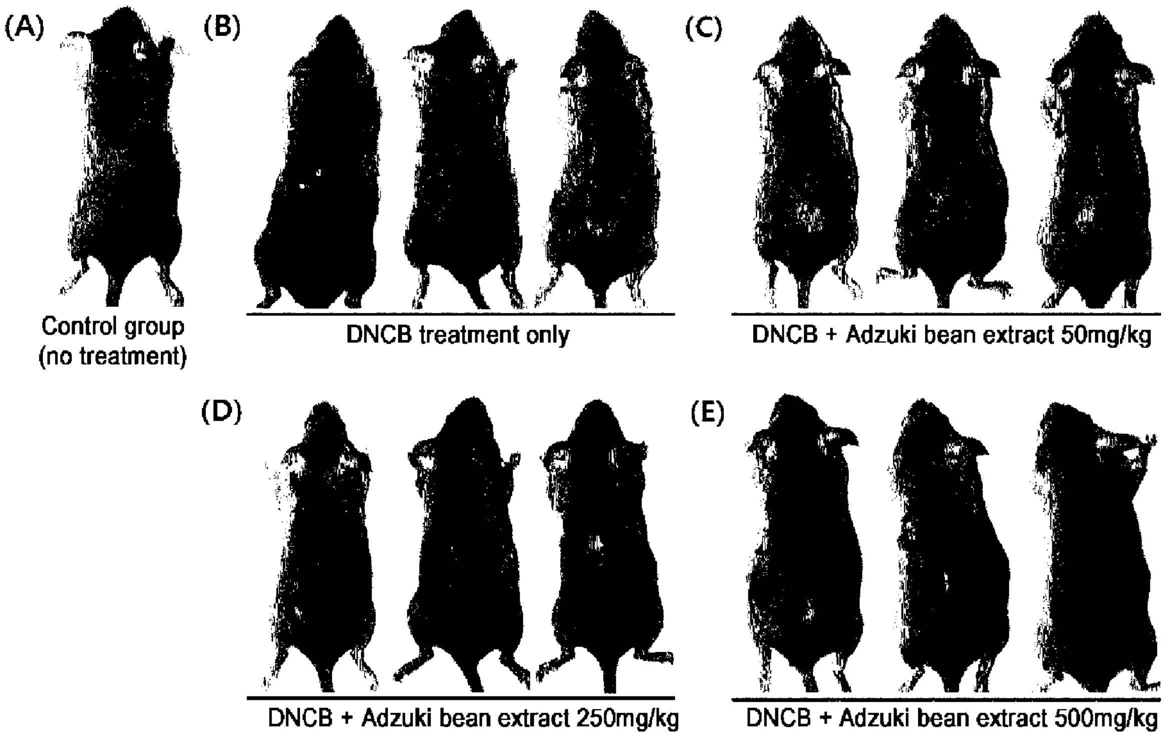
(A)
(B)
(C)
Control group
(no treatment)
DNCB treatment only
DNCB + Adzuki bean extract 50mg/kg
(D)
(E)
DNCB + Adzuki bean extract 250mg/kg
DNCB + Adzuki bean extract 500mg/kg

[FIG. 10]

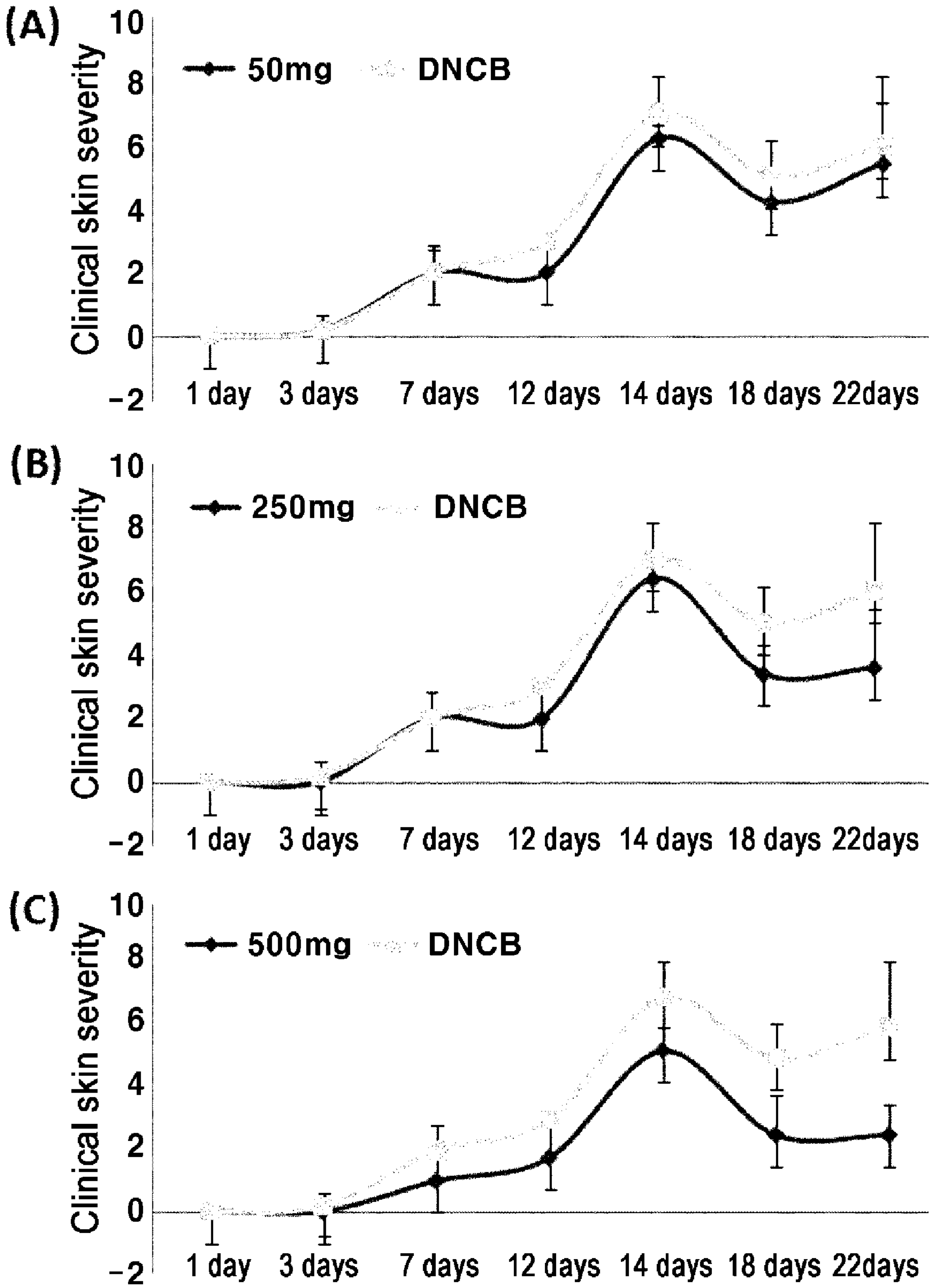
(A)
Clinical skin severity
10
8
6
4
2
0
-2
50mg
DNCB
1 day
3 days
7 days
12 days
14 days
18 days
22days
(B)
Clinical skin severity
250mg
DNCB
1 day
3 days
7 days
12 days
14 days
18 days
22days
(C)
Clinical skin severity
500mg
DNCB
1 day
3 days
7 days
12 days
14 days
18 days
22days

[FIG. 11]

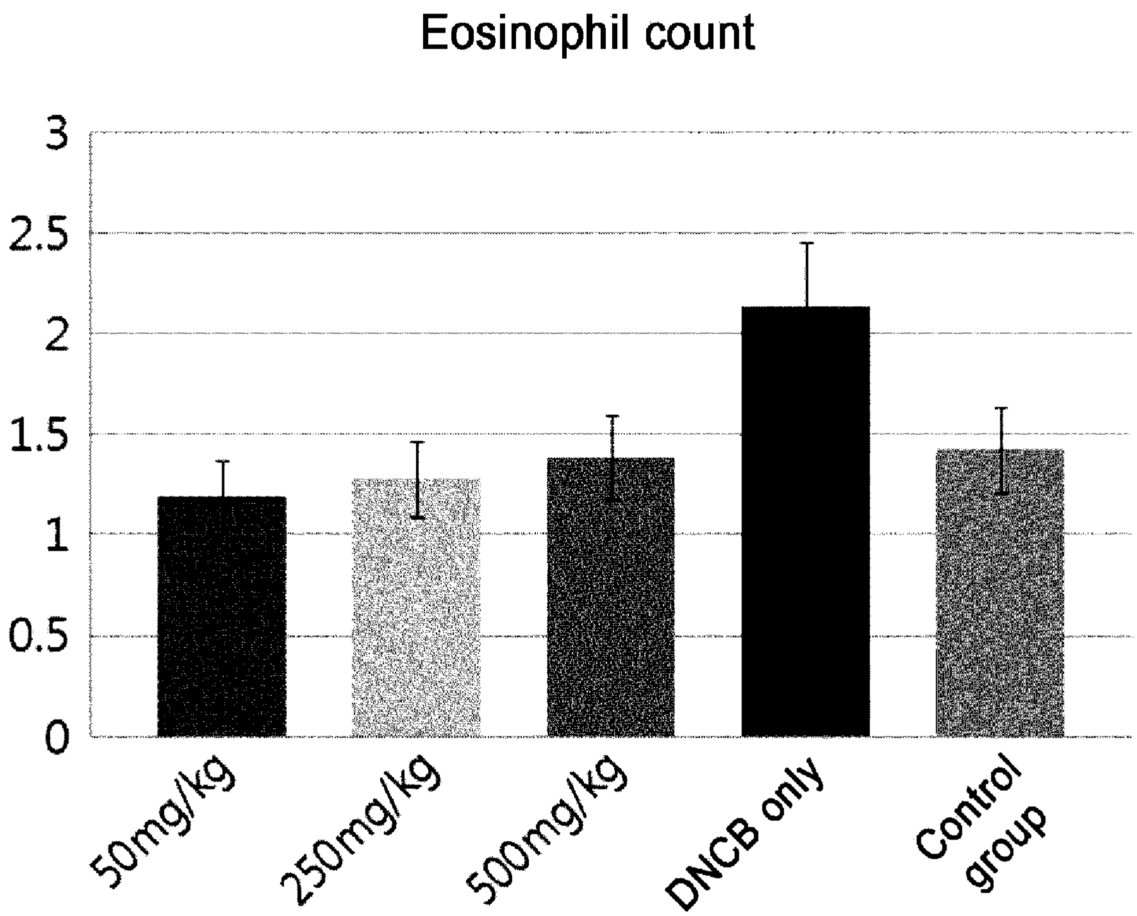
Eosinophil count
3
2.5
2
1.5
1
0.5
0
50mg/kg
250mg/kg
500mg/kg
DNCB only
Control group

[FIG. 12]

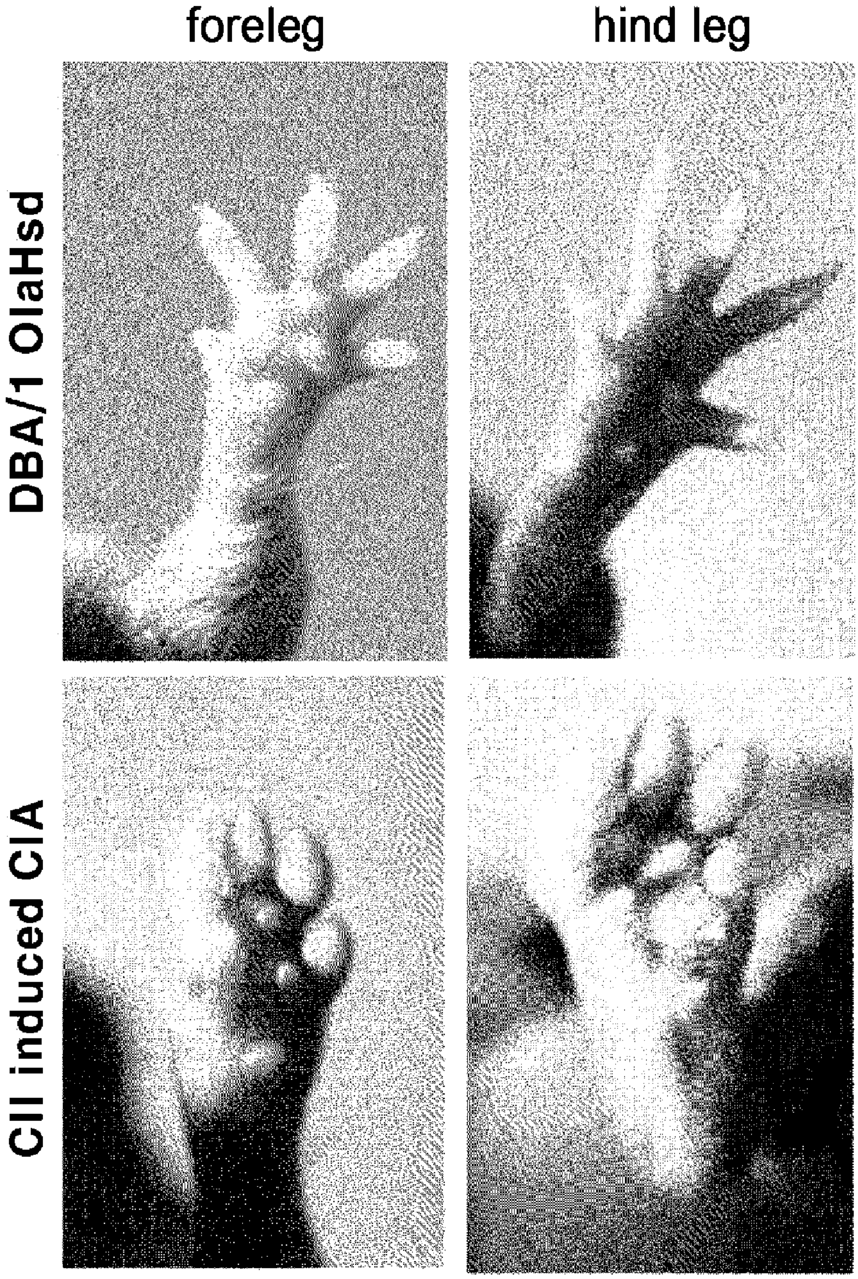

[FIG. 13]

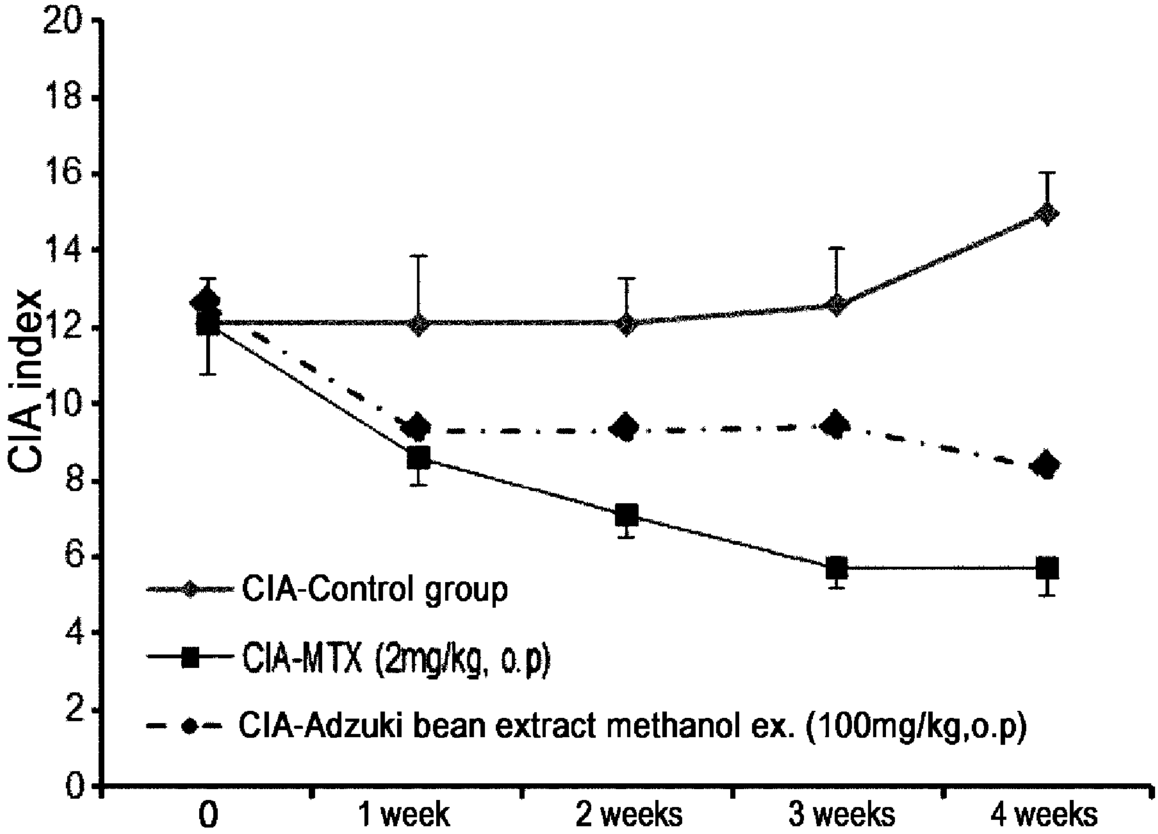
CIA index
20
18
16
14
12
10
8
6
4
2
0
0
1 week
2 weeks
3 weeks
4 weeks
CIA-Control group
CIA-MTX (2mg/kg, o.p)
CIA-Adzuki bean extract methanol ex. (100mg/kg,o.p)

[FIG. 14]

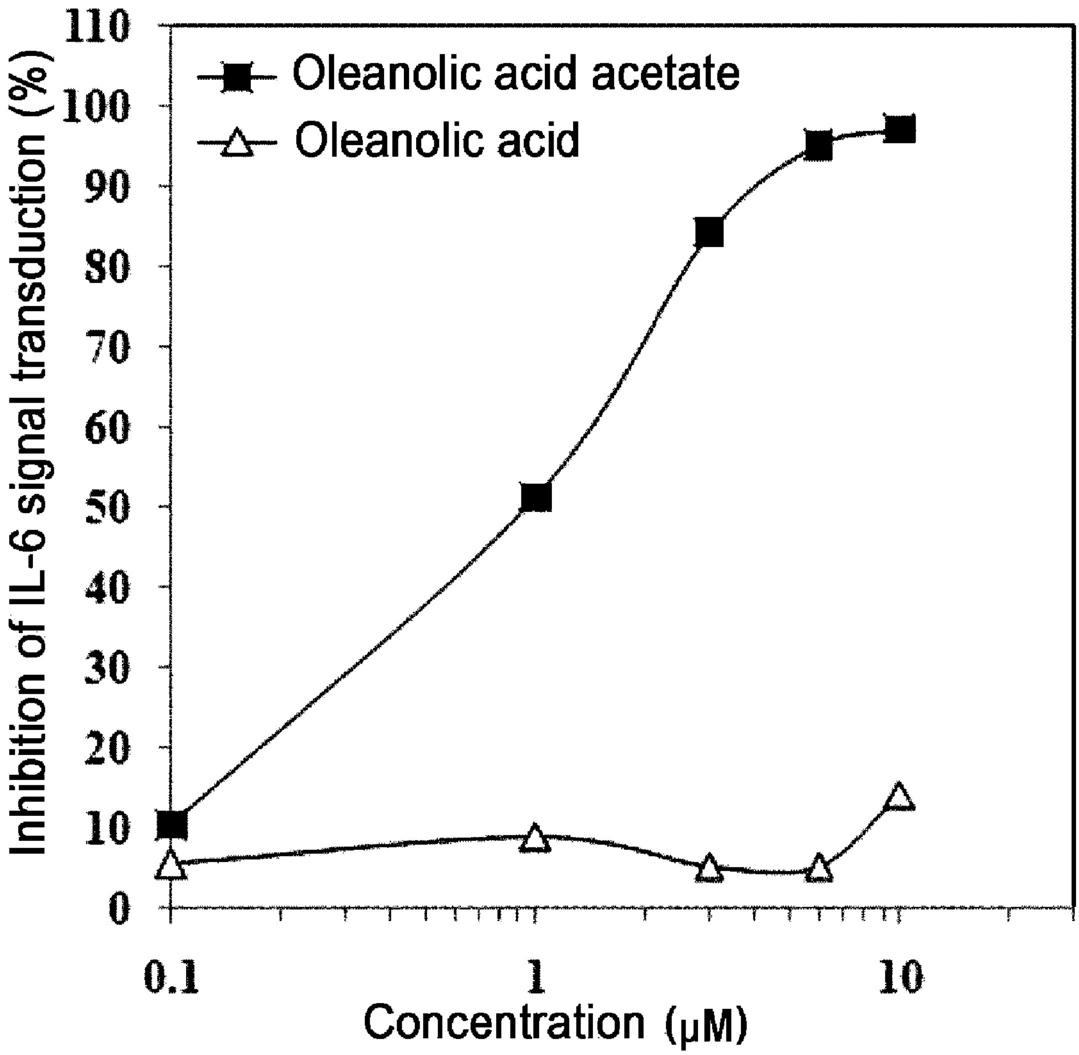
Oleanolic acid acetate
Oleanolic acid
Inhibition of IL-6 signal transduction (%)
110
100
90
80
70
60
50
40
30
20
10
0
0.1
1
10
Concentration (μM)

[FIG. 15]

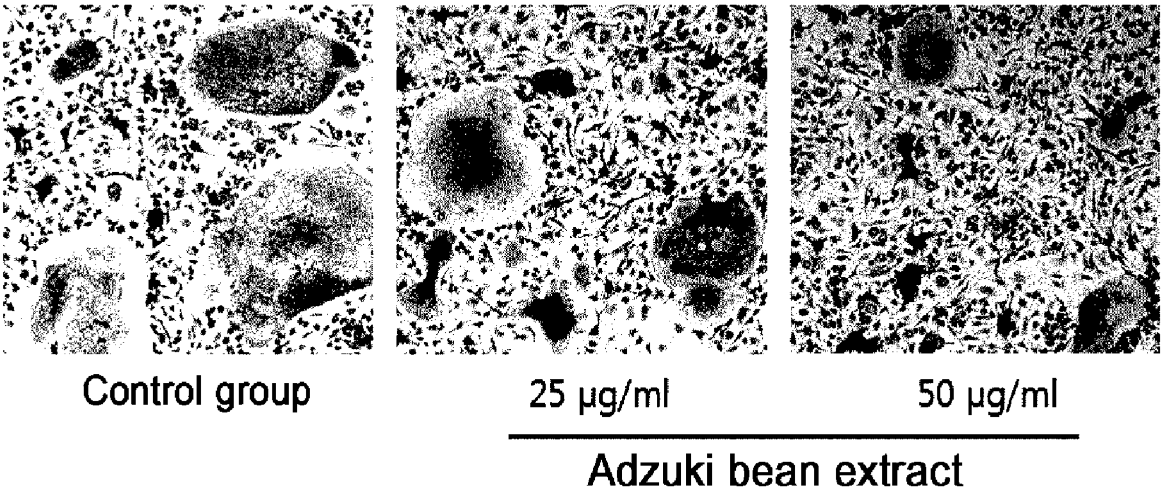
Control group
25 μg/ml
50 μg/ml
Adzuki bean extract

[FIG. 16]

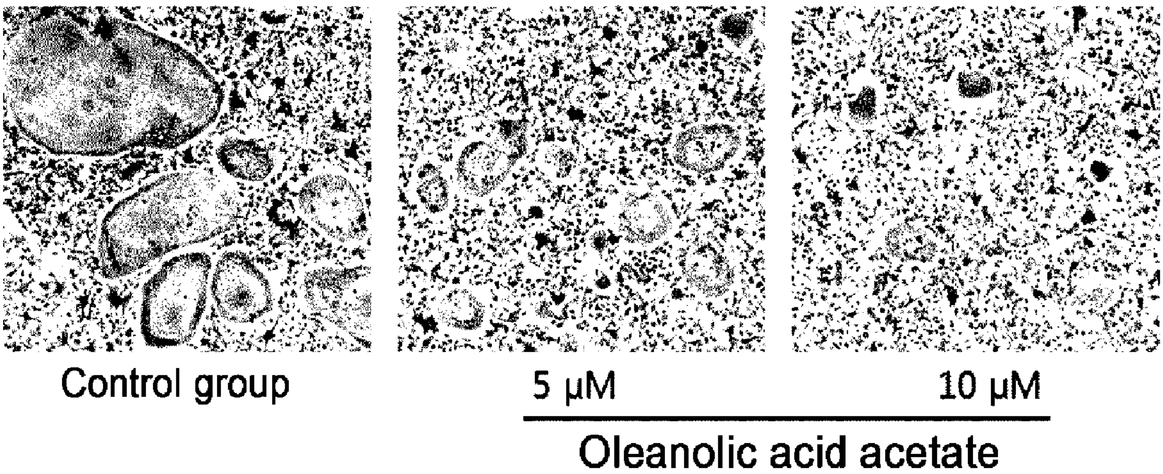
Control group
5 μM
10 μM
Oleanolic acid acetate

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007121352 A


### Non-patent literature cited in the description


- **CHEN G.Y.** *Nat. Rev. Immunol.,* 2010, vol. 10 (12), 826-837
- **BROWN J. et al.** *J. Dent. Res.,* 2010
- **PALUSINSKA-SZYSZ M. et al.** *Folia Microbiol.(Praha),* 2010, vol. 55 (5), 508-14
- **KAWAI T. et al.** *Ann. N.Y. Acad. Sci.,* 2008, vol. 1143, 1-20
- **LIEW F.Y. et al.** *Nat. Rev. Immunol.,* 2005, vol. 5 (6), 446-458
- **PALUSINSKA-SZYSZ M. et al.** *Folia Microbiol. (Praha),* 2010, vol. 55 (5), 508-14
- **ALEXOPOULOU L. et al.** *Nature,* 2001, vol. 413, 732-738
- **DARNELL JR. J.E. et al.** *Science,* 1994, vol. 264, 1415-1421
- **HIRANO T. et al.** *Nature,* 1986, vol. 324, 73-76
- **AKIRA S. et al.** *Adv. In Immunology,* 1993, vol. 54, 1-78
- **TAGA T. et al.** *J. Exp. Med.,* 1987, vol. 196, 967
- **HEINRICH P. et al.** *Biochem. J.,* 2003, vol. 374, 1-20
- **LEVY, D.E. et al.** *Nat. Rev. Mol. Cell Biol.,* 2002, vol. 3, 651-62
- **DARNELL, J.E.** *Science,* 1997, vol. 277, 1630-1635
- **AKIRA S. et al.** *Adv. Immunology,* 1993, vol. 54, 1-78
- **KALLEN K. et al.** *Exp. Opin. Invest. Drugs,* 1997, vol. 6, 237-266
- **NIU et al.** *Cancer Res.,* 1999, vol. 59, 5059-5063
- **KAREN H. COSTENBADER et al.** *American Journal of Epidemiology,* 2010, vol. 172 (2), 205-216
- **MAMESSIER, E. et al.** *European Journal of Dermatology,* 2006, vol. 16 (2), 103
- **HOFFJAN, S. et al.** *Journal of Molecular Medicine,* 2005, vol. 83 (9), 682-692
- **COOKSON, W.** *Nat. Rev. Immunol.,* 2004, vol. 4 (12), 978-988
- **NARVAEZ-MASTACHE J.M. et al.** *Journal of natural products,* 2006, vol. 69 (12), 1687-1691
- **GUIMARAES R.N. et al.** *Brazilian Journal of Pharmacognosy,* 2010, vol. 20 (1), 113-116
- **HARBORNE J.B.** Phytochemical methods: A guide to modern techniques of plant analysis. 1998, 6-7
- **VOUTQUENNE L. et al.** *Phytochemistry,* 2003, vol. 64, 781-789
- **KONGDUANG D. et al.** *Tetrahedron letters,* 2008, vol. 49, 4067-4072